# EUROPEAN PATENT APPLICATION

(11) **EP 4 495 110 A1**
(43) Date of publication of application: **22.01.2025**
(21) Application number: 23769908.7
(22) Date of filing: 17.03.2023
(51) Int. Cl.: C07D 401/14, C07D 471/04, C07D 417/14, A61K 31/506, A61P 35/00

(54) **NITROGEN-CONTAINING HETEROCYCLIC DERIVATIVE INHIBITOR, PREPARATION METHOD THEREFOR AND USE THEREOF**

(30) Priority: 17.03.2022 CN 202210266262; 23.06.2022 CN 202210724846; 09.09.2022 CN 202211104540
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: GAO, Peng, Shanghai 201203 (CN); ZENG, Mi, Shanghai 201203 (CN); SUN, Guangjun, Shanghai 201203 (CN); CHENG, Fengchang, Shanghai 201203 (CN); XIU, Wenhua, Shanghai 201203 (CN); YU, Wensheng, Shanghai 201203 (CN)
(74) Representative: EP&C
(86) International application number: PCT/CN2023/082178
(87) International publication number: WO 2023/174406

(57) **Abstract**

The present invention relates to a nitrogen-containing heterocyclic derivative EGFR inhibitor, a preparation method therefor and a use thereof. In particular, the present invention relates to a compound represented by general formula (I), a preparation method therefor, a pharmaceutical composition containing the compound, and a use thereof as an EGFR inhibitor in treating cancer.

## Description

### Technical Field

The present invention belongs to the field of biomedicine, and specifically relates to a nitrogen-containing heterocyclic derivative inhibitor, and a preparation method therefor and the use thereof.

### Background Art

EGFR (Epidermal Growth Factor Receptor) is a member of the ErbB family of transmembrane receptor tyrosine kinases, which is activated by binding to its ligand epidermal growth factor (EGF) or transforming growth factor α (TGFα). Activated EGFR forms homodimers on the cell membrane, or forms heterodimers with other receptors of the family (such as ErbB-2, ErbB-3, or ErbB-4), resulting in phosphorylation of the key tyrosine residues in EGFR cells, and activation of intracellular downstream signaling pathways, which plays an important role in cell proliferation, survival and anti-apoptosis. Activating mutations, overexpression or gene amplification of EGFR can lead to excessive activation of EGFR, accelerate the transformation of cells into tumor cells, and play an important role in the proliferation, invasion, metastasis and angiopoiesis of tumor cells. It is an important target for the development of anti-cancer drugs, especially drugs for treating lung cancer.

First-generation EGFR small molecule inhibitors including gefitinib (Iressa) and erlotinib (Tarceva) have shown good efficacy in the treatment of lung cancer and have been used as first-line drugs for the treatment of non-small cell lung cancer (NSCLC) with EGFR-activating mutations (including L858R and deIE746_A750). However, after 10 to 12 months of treatment with first-generation small molecule EGFR inhibitors, almost all NSCLC patients develop resistance to the first-generation small molecule inhibitors, and according to the resistance mechanism, more than half of the cases are due to a secondary mutation in the EGFR gatekeeper gene residue, T790M.

Osimertinib (AZD9291) is a third-generation EGFR TKI inhibitor that has a high response rate and good therapeutic effect against drug resistance caused by EGFR T790M mutation. It received accelerated approval from the US FDA in November 2015 and is clinically effective in treating advanced non-small cell lung cancer patients with EGFR T790M drug-resistant mutation. Although osimertinib has achieved great success in clinical treatment of non-small cell lung cancer with EGFR T790M mutation, patients still inevitably develop drug resistance after 9 to 14 months of treatment. Studies have shown that up to 20%-40% of patients are drug-resistant due to the EGFR C797S mutation. The EGFR C797S mutation causes the change of cysteine at position 797 to serine, resulting in the inability of osimertinib to form a covalent bond with the EGFR protein, thus causing drug resistance. Currently, there are no effective clinical inhibitors targeting the EGFR C797S drug-resistant mutation. Therefore, there is an urgent need to develop new highly active EGFR inhibitors to solve the problem of drug resistance caused by EGFR C797S mutation.

Novartis reported compound EAI0450, an EGFR allosteric inhibitor, targeting EGFR C797S drug-resistant mutation. When combined with EGFR monoclonal antibodies such as cetuximab, it showed good anti-tumor effects in a mouse in vivo pharmacodynamic model with L858R/T790M/C797S mutations. However, as a single drug, this compound is ineffective and cannot inhibit C797S drug-resistant mutation (including deIE746_A750), and has not been included in clinical research. In 2017, Ken Uchibori et al. reported that the combination of Brigatinib (AP26113) and EGFR monoclonal antibodies (such as cetuximab) can overcome the problem of drug resistance to third-generation EGFR inhibitors caused by the C797S mutation. In the PC9 (EGFR-C797S/T790M/de119) mouse pharmacodynamic model, it showed good anti-tumor efficacy. However, Brigatinib, as a single drug, also faces the problem of low in vitro activity and no significant anti-tumor activity in vivo, and further clinical research has not been conducted.

Lung cancer is a major disease that threatens human health, and its mortality rate ranks first among all malignant tumors. In China, the incidence of lung cancer is increasing year by year, with about 700,000 new cases every year. The cases of lung cancer with EGFR activating mutations in China account for about 35% of all NSCLC cases. The use of first- or third-generation EGFR inhibitors can achieve good therapeutic effects, but new drug-resistant mutations will occur in the later stages. Therefore, the development of new generation of anti-drug-resistant EGFR inhibitors has huge clinical and market value.

### Summary of the Invention

The objective of the present invention is to provide a compound as represented by general formula (I), or a stereoisomer or pharmaceutically acceptable salt thereof, wherein the compound as represented by general formula (I) has a structure as follows: 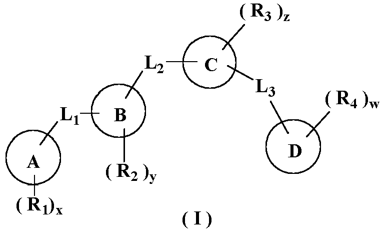 wherein:
ring A, ring B, ring C and ring D are each independently selected from cycloalkyl, heterocyclyl, aryl or heteroaryl;
L₁, L₂ and L₃ are each independently selected from a bond, substituted or unsubstituted alkenyl, substituted or unsubstituted alkynyl, -(CH₂)ₙ-, -(CH₂)ₙC(O)(CRₐₐR_{bb})ₙ₁-, -(CH₂)ₙC(O)NRₐₐ(CH₂)ₙ₁-, - (CH₂)ₙ(CRₐₐR_{bb})ₙ₂-, -(CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, - (CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, -(CH₂)ₙNRₐₐC(O)-, - (CH₂)ₙP(O)ₚRₐₐ-, -(CH₂)ₙS(O)ₘ-, -(CH₂)ₙS(O)ₘNRₐₐ- or -(CH₂)ₙNRₐₐS(O)ₘ-;
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl and heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₃ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl,
-ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
or, wherein one R₂ and one R₄ are connected to the atoms therebetween to form cycloalkyl, heterocyclyl, aryl or heteroaryl, and the cycloalkyl, heterocyclyl, aryl and heteroaryl can be optionally further substituted;
Rₐ is selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
Rₐₐ, R_{bb} and R_{cc} are each independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
z is 0, 1, 2, 3, 4, 5 or 6;
w is 0, 1, 2, 3, 4, 5 or 6;
and p, m, n, n1, n2, n3, n4 and n5 are each independently 0, 1, 2 or 3.

In certain embodiments of the present invention, the compound of formula (I) is further represented by general formula (II-A), (II-B), (II-C), (II-D), (II-E), (II-F) or (II-G): 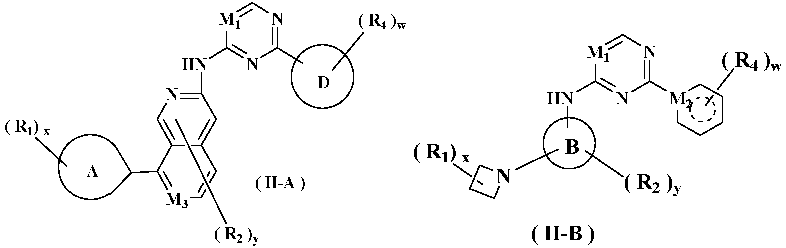 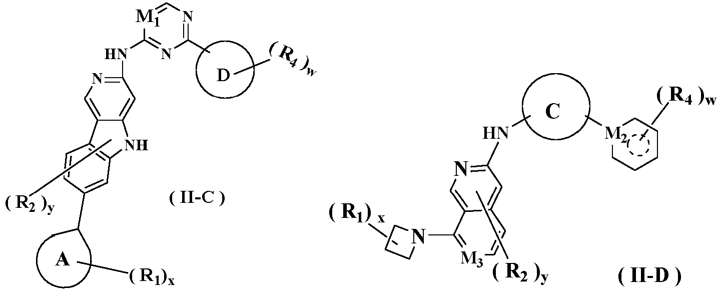 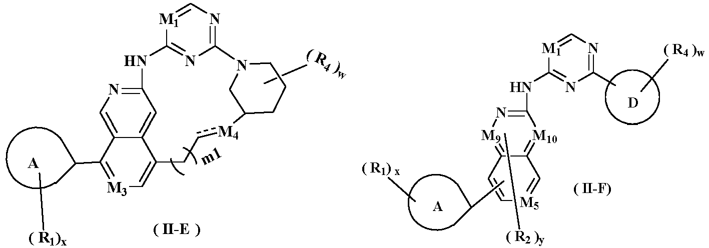 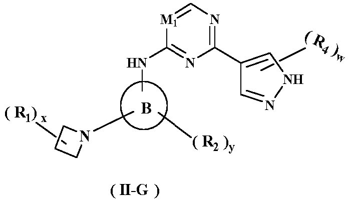 wherein: 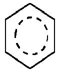 represents a saturated, unsaturated or partially saturated ring; 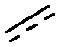
is a single bond or double bond;
M₁ is C, N or CH;
M₂ is N, NH, CH, CH₂, O or S;
M₃ is N or CH;
M₄ is N, NH, CH, CH₂, O or S;
M₅ is N or CH;
M₉ is N or CH;
M₁₀ is N or CH;
m1 is 0, 1, 2, 3 or 4.

In certain embodiments of the present invention, ring A is selected from C₃₋₁₂ cycloalkyl, 3- to 12-membered heterocyclyl, C₆₋₁₄ aryl or 5- to 14-membered heteroaryl, the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; or, ring A is absent, L₁ and R₁ are directly connected;
preferably, ring A is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring A is 4- to 10-membered heterocyclyl; more preferably, ring A is 4- to 6-membered monocyclic heterocyclyl, 7- to 9-membered spiro heterocyclyl or 8- to 10-membered fused heterocyclyl;
more preferably, ring A is 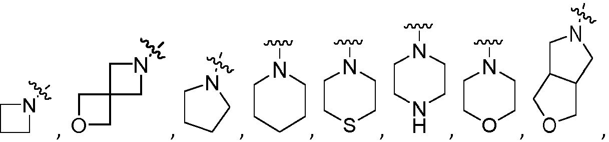 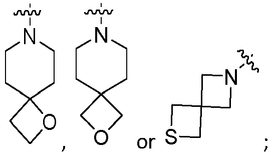 more preferably 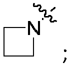
further preferably, ring A is 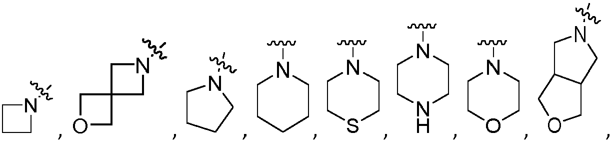 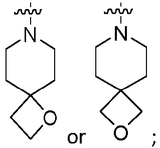
and even further preferably, ring A is 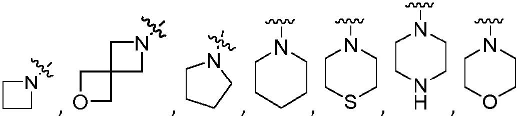 or

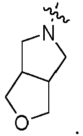

In certain embodiments of the present invention, ring B is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably 5- to 14-membered heteroaryl, and the heteroaryl is monocyclic or fused ring; preferably 8- to 14-membered heteroaryl;
preferably, ring B is 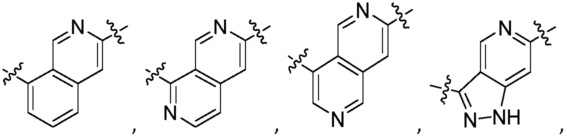 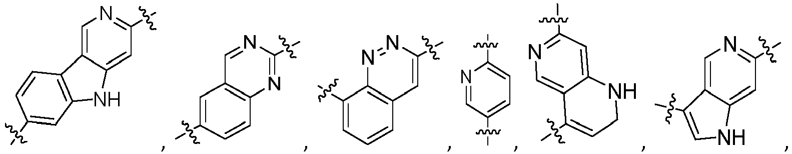 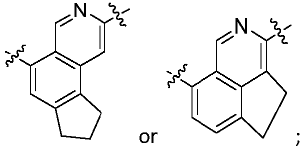 preferably, ring B is 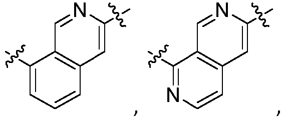 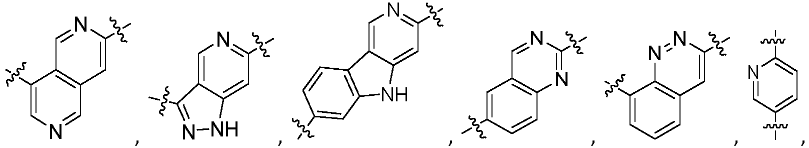 or 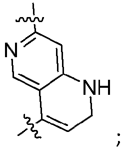 further preferably, ring B is 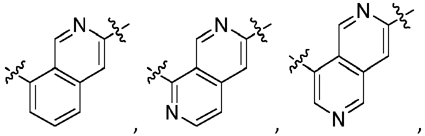 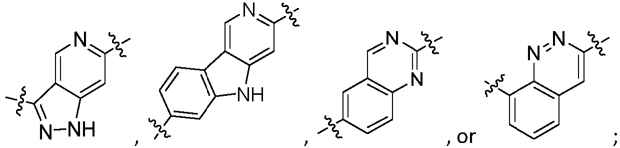 more preferably, ring B is

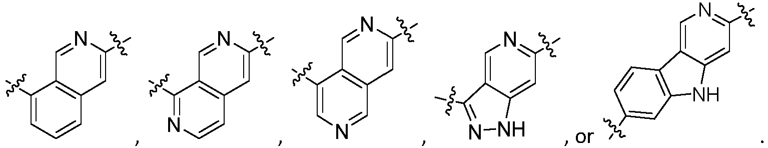

In certain embodiments of the present invention, ring C is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring C is 5- to 6-membered heteroaryl;
more preferably, ring C is 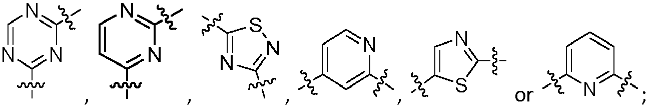
more preferably, ring C is 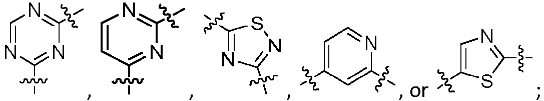 even
further preferably, ring C is

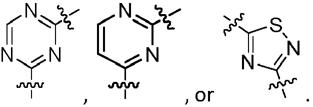

In certain embodiments of the present invention, ring D is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring D is C₃₋₆ cycloalkyl, 5- to 8-membered heterocyclyl, 5- to 8-membered heteroaryl;
more preferably, ring D is C₃₋₆ cycloalkyl, 5- to 8-membered heterocyclyl, 5- to 6-membered heteroaryl; the 5- to 8-membered heterocyclyl is selected from 5- to 6-membered monocyclic heterocyclyl, 7- to 8-membered spiro heterocyclyl or 7- to 8-membered fused heterocyclyl; more preferably, ring D is 5-membered heteroaryl, 6-membered heteroaryl, 5- to 6-membered monocyclic heterocyclyl, 7- to 8-membered spiro heterocyclyl or 7- to 8-membered fused heterocyclyl;
further preferably, ring D is 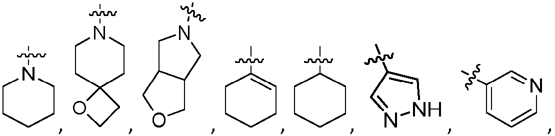 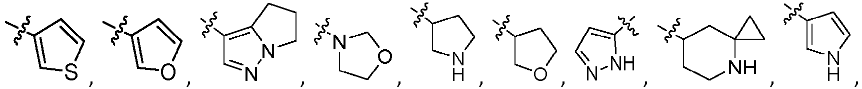 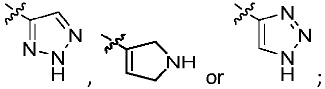 preferably 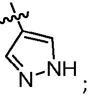 preferably, ring D is 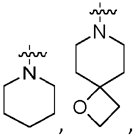 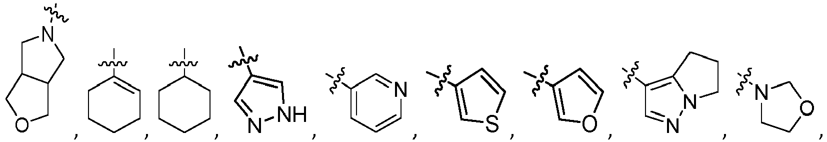 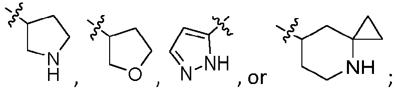 more preferably, ring D is

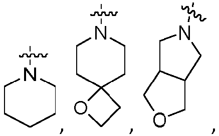

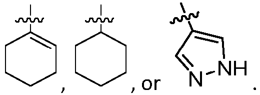

In certain embodiments of the present invention, the compound of formula (I) is further represented by general formula (II), (III), (IV), (V), (VI), (VII), (VIII), (IX), (X), (XI), (XII), (XIII) or (XIV): 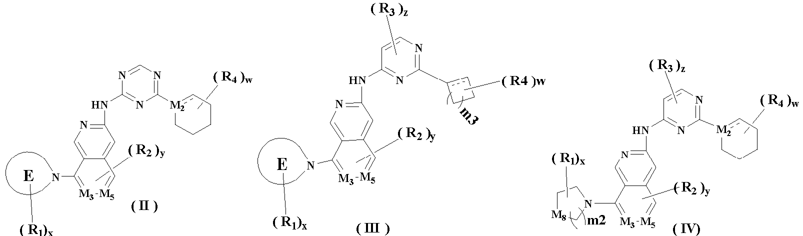 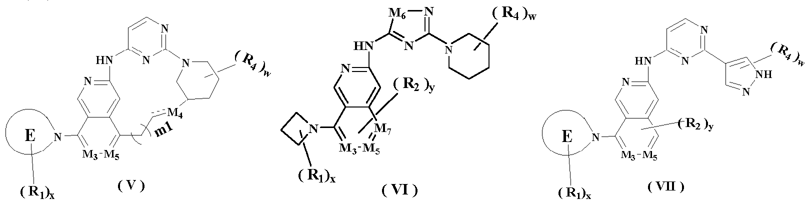 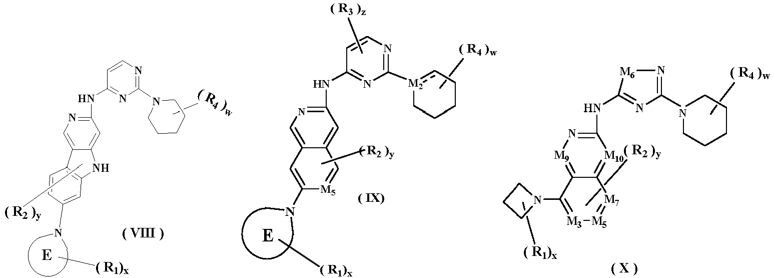 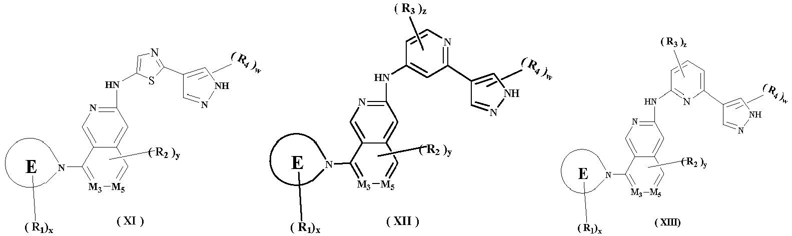
wherein, 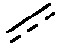 is single bond or double bond;
M₁ is C, N or CH;
M₂ is N, NH, CH, CH₂, O or S;
M₃ is N or CH;
M₄ is N, NH, CH, CH₂, O or S;
M₅ is N or CH;
M₆ is S or CH=CH;
M₇ is a bond, N, NH or CH;
M₈ is NH, CH₂, O or S;
M₉ is N or CH;
M₁₀ is N or CH;
ring E is 4- to 10-membered heterocyclyl; preferably, ring E is 4- to 10-membered heterocyclyl containing 1-4 heteroatoms selected from N, O, S or P; m1 is 0, 1, 2, 3 or 4;
m2 is 1, 2 or 3;
and m3 is 1, 2, 3, 4, 5 or 6.

In certain embodiments of the present invention, for general formula (VI), when M₆ is CH=CH and M₇ is CH, R₁ is not substituted or unsubstituted -CH₂S(O)₂Rₐ.

In certain embodiments of the present invention, for general formula (VI), when M₆ is CH=CH and M₇ is CH, R₁ is not -CH₂S(O)₂Rₐ.

In certain embodiments of the present invention, for general formula (VI), when M₆ is CH=CH and M₇ is CH, R₁ is not -CH₂S(O)₂CH₃, -CHCH₃S(O)₂CH₃, or -CH₂S(O)₂CH₂CH₃.

In certain embodiments of the present invention, ring E is selected from 4- to 6-membered monocyclic heterocyclyl, 7- to 9-membered spiro heterocyclyl or 8- to 10-membered fused heterocyclyl; preferably, ring E is 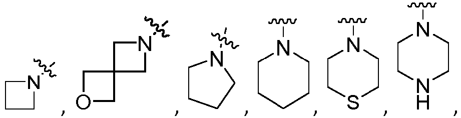 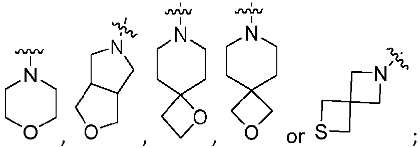 more preferably 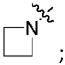 preferably, ring E is 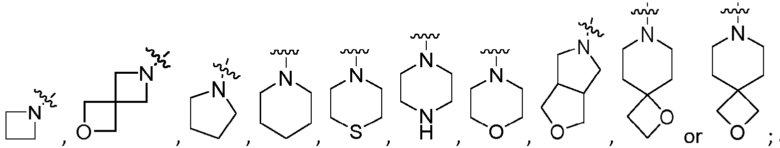 and further preferably, ring E is the group as follows:

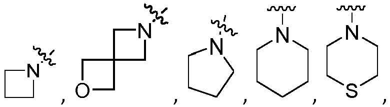

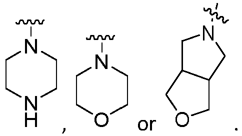

In certain embodiments of the present invention, the compound is further represented by general formula (VII-1) or (VIII-1): 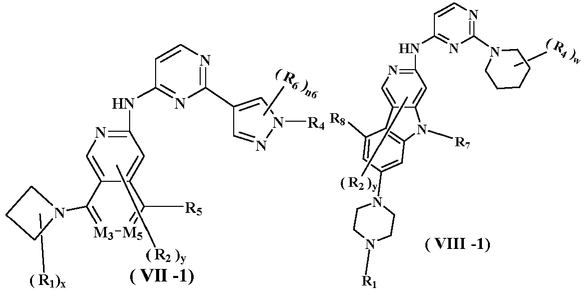
wherein R₅, R₆, R₇ and R₈ are independently selected from hydrogen, deuterium, oxo, thio, halogen, amino, hydroxyl, cyano, nitro, C₁₋₆ alkyl, C₁₋₆ deuteroalkyl, C₁₋₆ haloalkyl, C₂₋₆ alkenyl, C₂₋₆ alkynyl, C₁₋₆ alkoxy, C₁₋₆ haloalkoxy, C₁₋₆ deuteroalkoxy or C₁₋₆ hydroxyalkyl; preferably, R₅, R₆, R₇ and R₈ are independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ haloalkoxy or C₁₋₆ hydroxyalkyl; more preferably, R₅, R₆, R₇ and R₈ are independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
and n6 is 0, 1 or 2.

In certain embodiments of the present invention, R₆ is methyl, deuterated methyl, fluorine or chlorine.

In certain embodiments of the present invention, R₅ and R₇ are independently methyl, ethyl, n-propyl or isopropyl, preferably isopropyl.

In certain embodiments of the present invention, R₈ is -OCH₂CF₃, -OCH₂CHF₂, -OCH₂CH₂F, -OCHFCF₃, or -OCF₂CF₃, preferably -OCH₂CF₃.

In certain embodiments of the present invention, n6 is 0.

**In** certain embodiments of the present invention, L₁ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁-, - (CH₂)ₙO(CRₐₐR_{bb})ₙ₁-, -(CRₐₐR_{bb})ₙ₃S(CH₂)ₙ₄-, -(CH₂)ₙS(CRₐₐR_{bb})ₙ₃-, -(CRₐₐR_{bb})ₙ₃(CH₂)ₙNR_{cc}-, - (CH₂)ₙNRₐₐ(CR_{bb}R_{cc})ₙ-, -(CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNRₐₐS(O)ₘ-; preferably a bond.

In certain embodiments of the present invention, R₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -(CH₂)ₙORₐ, -(CH₂)ₙP(O)ₚ(Rₐ)ₙ₅, -(CH₂)ₙS(O)RₐₐN(Rₐₐ), -(CH₂)ₙN=S(O)(Rₐₐ)₂, - (CH₂)ₙS(O)ₘRₐ or -(CH₂)ₙC(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -S(O)RₐₐN(Rₐₐ), -N=S(O)(Rₐₐ)₂, - P(O)ₚ(Rₐₐ)ₙ₅, -S(O)ₘRₐₐ, -Se(O)ₘRₐₐ or -C(O)Rₐₐ; or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5-to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy or C₁₋₆ hydroxyalkyl.

In certain embodiments of the present invention, R₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)_{b}(Rₐₐ)ₙ₅, -S(O)ₘRₐₐ, -Se(O)ₘRₐₐ or -C(O)Rₐₐ; or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy or C₁₋₆ hydroxyalkyl;
preferably, R₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or - C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)_{b}(Rₐₐ)ₙ₅, -S(O)ₘRₐₐ or -C(O)Rₐₐ;
preferably, R₁ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, C₆₋₁₀ aryl, 5- to 12-membered heteroaryl, -ORₐ, -P(O)ₚ(Rₐ)ₙ₅, -S(O)ₘRₐ or - C(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl, 3- to 10-membered heterocyclyl, C₆₋₁₀ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, 5- to 10-membered heteroaryl, -ORₐₐ, -P(O)_{b}(Rₐₐ)ₙ₅, -S(O)ₘRₐₐ or -C(O)Rₐₐ;
or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy or C₁₋₆ hydroxyalkyl;
preferably, two R₁ are connected to the atoms therebetween to form C₃₋₁₀ cycloalkyl, 3- to 10-membered heterocyclyl, C₆₋₁₂ aryl and 5- to 12-membered heteroaryl, and the C₃₋₁₀ cycloalkyl, 3-to 10-membered heterocyclyl, C₆₋₁₀ aryl and 5- to 10-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy and C₁₋₃ hydroxyalkyl.

In certain embodiments of the present invention, R₁ is -CH₂S(O)₂N(CH₃)₂, -CH₂Se(O)₂CH₃, -CH₂S(O)₂CH₃, -CH₂S(O)₂F, -NCH₃S(O)₂CH₃, -NHS(O)₂CH₃, methyl, hydrogen, 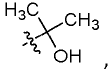 methoxy, cyano, -CH₂OCH₃, -CH₂CN, -CH(CN)₂, -S(O)₂CH₃, oxo, hydroxyl, - CH₂COCH₃, -COCH₃, - CH₂P(O)(CH₃)₂, -CH₂NO₂, 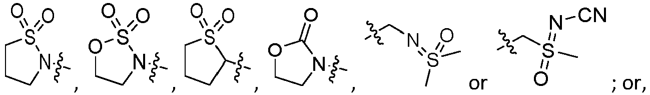 two R₁ are connected to the atoms therebetween to form 3- to 12-membered heterocyclyl, preferably

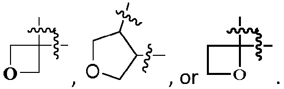

In certain embodiments of the present invention, R₁ is -CH₂S(O)₂N(CH₃)₂, -CH₂Se(O)₂CH₃, -CH₂S(O)₂CH₃, -CH₂S(O)₂F, -NHS(O)₂CH₃, methyl, hydrogen, 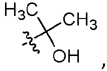 methoxy, cyano, - CH₂OCH₃, -CH₂CN, -CH(CN)₂, -S(O)₂CH₃, oxo, hydroxyl, - CH₂COCH₃, -COCH₃, -CH₂P(O)(CH₃)₂ or - CH₂NO₂; or, two R₁ are connected to the atoms therebetween to form 3- to 12-membered heterocyclyl, preferably

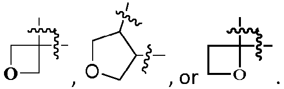

In certain embodiments of the present invention, R₁ is -CH₂S(O)₂N(CH₃)₂, -CH₂Se(O)₂CH₃, -CH₂S(O)₂CH₃, methyl, hydrogen, 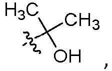 methoxy, cyano, -CH₂OCH₃, -CH₂CN, -CH(CN)₂, - S(O)₂CH₃, oxo, hydroxyl, - CH₂COCH₃, -COCH₃ or -CH₂P(O)(CH₃)₂; or, two R₁ are connected to the atoms therebetween to form 3- to 12-membered heterocyclyl, preferably 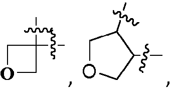 or

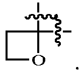

In certain embodiments of the present invention, R₁ is -CH₂S(O)₂CH₃, methyl, hydrogen, 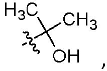 methoxy, cyano, -CH₂OCH₃, -CH₂CN, -S(O)₂CH₃, oxo, hydroxyl, - CH₂COCH₃, -COCH₃ or -CH₂P(O)(CH₃)₂; or, two R₁ are connected to the atoms therebetween to form 3- to 12-membered heterocyclyl, preferably

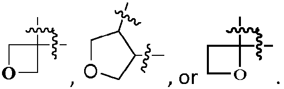

In certain embodiments of the present invention, R₁ is -CH₂S(O)₂CH₃, methyl, hydrogen, 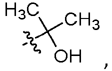 methoxy, cyano, -CH₂OCH₃, -CH₂CN, -S(O)₂CH₃, oxo, hydroxyl, -COCH₃ or - CH₂P(O)(CH₃)₂; or, two R₁ are connected to the atoms therebetween to form 3- to 12-membered heterocyclyl, preferably

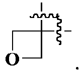

In certain embodiments of the present invention, L₂ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁-, - (CH₂)ₙO(CRₐₐR_{bb})ₙ₁-, -(CRₐₐR_{bb})ₙ₃S(CH₂)ₙ₄-, -(CH₂)ₙS(CRₐₐR_{bb})ₙ₃-, -(CRₐₐR_{bb})ₙ₃(CH₂)ₙNR_{cc}-, - (CH₂)ₙNRₐₐ(CR_{bb}R_{cc})ₙ-, -(CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNR₂₂S(O)ₘ-; preferably -NH-.

In certain embodiments of the present invention, R₂ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)_{b}(Rₐₐ)ₙ₅, -S(O)ₘRₐₐ or -C(O)Rₐₐ; preferably, R₂ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or - C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₂ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)_{b}(Rₐₐ)ₙ₅, -S(O)ₘRₐₐ or -C(O)Rₐₐ; more preferably, R₂ is isopropyl, cyano, trifluoromethyl or -OCH₂CF₃; further preferably, R₂ is isopropyl or -OCH₂CF₃.

In certain embodiments of the present invention, R₂ is oxo, fluorine, isopropyl, cyano, trifluoromethyl, -NHCH(CH)₃ or -OCH₂CF₃.

In certain embodiments of the present invention, L₃ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁-, - (CH₂)ₙO(CRₐₐR_{bb})ₙ₁-, -(CRₐₐR_{bb})ₙ₃S(CH₂)ₙ₄-, -(CH₂)ₙS(CRₐₐR_{bb})ₙ₃-, -(CRₐₐR_{bb})ₙ₃(CH₂)ₙNR_{cc}-, - (CH₂)ₙNRₐₐ(CR_{bb}R_{cc})ₙ-, -(CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNRₐₐS(O)ₘ-; preferably a bond.

In certain embodiments of the present invention, R₃ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; preferably, R₃ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, - P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)_{b}(Rₐₐ)ₙ₅, -S(O)ₘRₐₐ or -C(O)Rₐₐ; or, R₃ and R₄ are connected to form 5- to 10-membered heterocyclyl; more preferably, R₃ is hydrogen.

In certain embodiments of the present invention, -R₃-R₄- is -OCH₂-.

In certain embodiments of the present invention, R₃ is selected from hydrogen, methoxy, methyl, fluorine, chlorine, bromine, trifluoromethyl, cyano, cyclopropyl or -COCH₃.

In certain embodiments of the present invention, R₄ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more R₄₋₁; R₄₋₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)_{b}(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, and C₁₋₆ hydroxyalkyl are optionally substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy or C₁₋₆ hydroxyalkyl.

In certain embodiments of the present invention, R₄ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 10-membered heteroaryl can be optionally further substituted with one or more R₄₋₁; R₄₋₁ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐₐ, -P(O)_{b}(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy or C₁₋₃ hydroxyalkyl.

In certain embodiments of the present invention, R₄ is fluorine, chlorine, methyl, hydroxyl, methoxy, nitro, oxo, -CD₃, -OCD₃, -CH₂F, -CHF₂, CH₂CF₃, -CH₂CH₂F, hydrogen, -CH₂OCH₃, -CF₃, -CH₂CH₂OH, -CH₂CH₂OCH₃, -CH₂CH₂CN, -CH₂CH₂N(CH₃)₂, -CH₂CH₂NH₂, -CH₂CH₂NHCH₃, amino, oxo, -COCH₃, cyclopropyl, 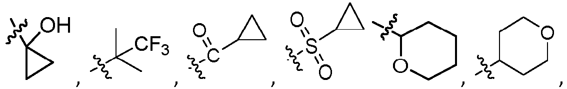 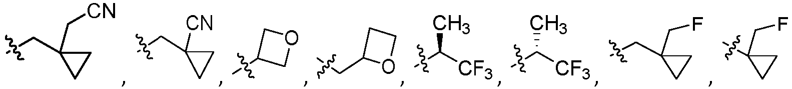 or

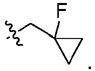

In certain embodiments of the present invention, R₄ is fluorine, chlorine, methyl, hydroxyl, methoxy, nitro, oxo, -OCD₃, -CH₂F, -CHF₂, CH₂CF₃, -CH₂CH₂F, hydrogen, -CH₂OCH₃, -CF₃, -CH₂CH₂OH, amino, oxo, -COCH₃, cyclopropyl,

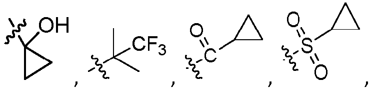

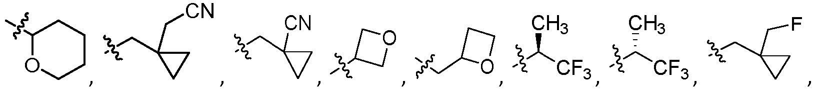

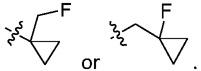

In certain embodiments of the present invention, R₄ is fluorine, methyl, hydroxyl, methoxy, -OCD₃, -CH₂F, -CHF₂, CH₂CF₃, -CH₂CH₂F, hydrogen, -CH₂OCH₃, -CF₃, amino, oxo, -COCH₃,

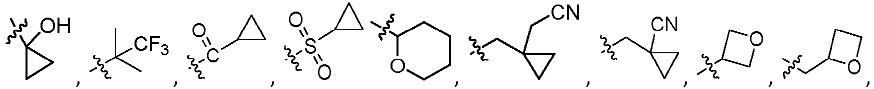

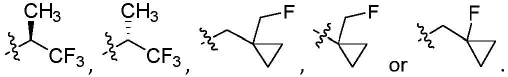

In certain embodiments of the present invention, R₄ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)_{b}(Rₐₐ)ₙ₅, -S(O)ₘRₐₐ or -C(O)Rₐₐ; preferably, R₄ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or - C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)_{b}(Rₐₐ)ₙ₅, -S(O)ₘRₐₐ or -C(O)Rₐₐ; more preferably, R₄ is fluorine, methyl, hydroxyl, methoxy, -OCD₃, hydrogen, 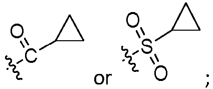 further preferably, R₄ is fluorine, methyl, hydroxyl, methoxy, -OCD₃, hydrogen or

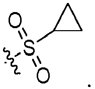

In certain embodiments of the present invention, one R₂ and one R₄ are connected to the atoms therebetween to form C₃₋₁₂ cycloalkyl, 3- to 12-membered heterocyclyl, C₆₋₁₄ aryl or 5-to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl or 5- to 12-membered heteroaryl; preferably, wherein -R₂-R₄- is 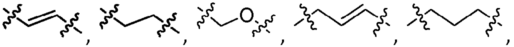 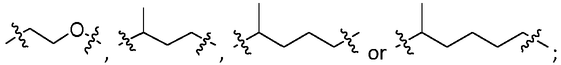 more preferably, wherein -R₂-R₄- is

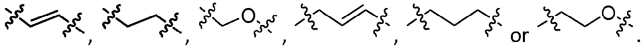

In certain embodiments of the present invention, Rₐ is selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl or 5- to 12-membered heteroaryl. Preferably, Rₐ is selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl or 5- to 10-membered heteroaryl.

In certain embodiments of the present invention, Rₐₐ, R_{bb} and R_{cc} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl. preferably, Rₐₐ, R_{bb} and R_{cc} are independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl.

In certain embodiments of the present invention, the compound is further as represented by general formula (VII-2), (VII-3) or (VII-4): 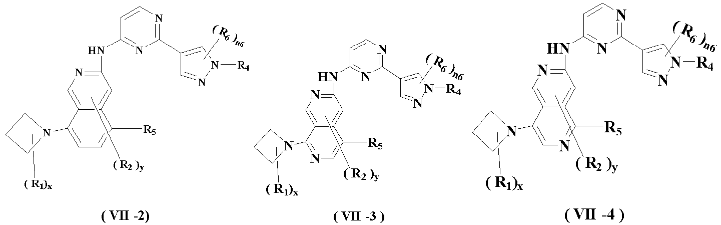
R₁ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, -(CH₂)ₙORₐ, -(CH₂)ₙP(O)ₚ(Rₐ)ₙ₅, -(CH₂)ₙS(O)RₐₐN(Rₐₐ), - (CH₂)ₙN=S(O)(Rₐₐ)₂, -(CH₂)ₙS(O)ₘRₐ or -(CH₂)ₙC(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl and 3- to 10-membered heterocyclyl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃alkoxy, C₁₋₃ hydroxyalkyl, -ORₐₐ, -S(O)RₐₐN(Rₐₐ), -N=S(O)(Rₐₐ)₂, -P(O)ₚ(Rₐₐ)ₙ₅, - S(O)ₘRₐₐ or -C(O)Rₐₐ;
R₂ is independently selected from hydrogen, deuterium, oxo, thio, halogen, amino, hydroxyl, cyano, nitro, C₁₋₃ alkyl, halo C₁₋₃alkyl, C₂₋₃ alkenyl, C₂₋₃ alkynyl, C₁₋₃ alkoxy or C₁₋₃ hydroxyalkyl;
R₄ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, -S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl and 3- to 10-membered heterocyclyl can be optionally further substituted with one or more R₄₋₁; R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄₋₁ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy or C₁₋₃ hydroxyalkyl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl or 3- to 10-membered heterocyclyl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₅ is independently selected from hydrogen, deuterium, oxo, thio, halogen, amino, hydroxyl, cyano, nitro, C₁₋₃ alkyl, halo C₁₋₃alkyl, deuterated C₁₋₃ alkyl, C₂₋₃ alkenyl, C₂₋₃ alkynyl, C₁₋₃ alkoxy or C₁₋₃ hydroxyalkyl, and the amino, C₁₋₃ alkyl, halo C₁₋₃alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃alkyl; preferably, R₅ is selected from hydrogen, hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
R₆ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₐ or -C(O)Rₐ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl; preferably, R₆ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
Rₐ is independently selected from hydrogen, deuterium, halogen, amino, hydroxyl, cyano, nitro, C₁₋₃ alkyl, C₁₋₃ haloalkyl, C₁₋₃ deuteroalkyl C₂₋₃ alkenyl, C₂₋₃ alkynyl, C₁₋₃ alkoxy, C₁₋₃ hydroxyalkyl, C₃₋₁₀ cycloalkyl or 3- to 10-membered heterocyclyl; preferably, Rₐ is selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl or 3- to 10-membered heterocyclyl;
each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy or C₁₋₃ hydroxyalkyl;
x is 0, 1, 2 or 3;
y is 0, 1, 2 or 3;
p is 0, 1, 2 or 3;
n is 0, 1, 2 or 3;
m is 0, 1, 2 or 3;
n5 is 0, 1, 2 or 3;
and n6 is 0, 1 or 2.

In certain embodiments of the present invention, the compound is further as represented by general formula (VII-2-1), (VII-3-1) or (VII-4-1): 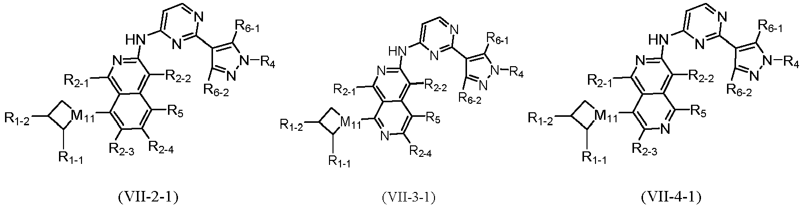 wherein
M₁₁ is independently CH or N;
R₁₋₁ is independently selected from hydrogen, deuterium, fluorine, chlorine, bromine, methyl, ethyl or halomethyl;
R₁₋₂ is independently selected from amino, hydroxyl, cyano, C₁₋₃ alkoxy, C₁₋₃ alkyl, C₃₋₆ cycloalkyl, or 3- to 8-membered heterocyclyl, and the amino, C₁₋₃ alkoxy, C₁₋₃ alkyl, C₃₋₆ cycloalkyl, and 3- to 8-membered heterocyclyl can be optionally further substituted with one or more of hydrogen, deuterium, oxo, thio, halogen, amino, hydroxyl, cyano, nitro, C₁₋₃ alkyl, C₁₋₃ deuteroalkyl, C₁₋₃ haloalkyl, C₁₋₃ alkoxy, halo C₁₋₃ alkoxy, C₁₋₃ hydroxyalkyl, -C(O)Rₑₑ, -ORₑₑ, -P(O)ₚ(Rₑₑ)ₙ₅ and - S(O)ₘRₑₑ; preferably, R₁₋₂ is selected from -NHS(O)₂CH₃, -NCH₃S(O)₂CH₃, -CH₂S(O)₂CH₃, -CH₂SOCH₃, -CH₂NO₂, methyl, hydrogen, 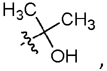 methoxy, cyano, -CH₂OCH₃, -CH₂CN, -CH(CN)₂, hydroxyl, - CH₂COCH₃, 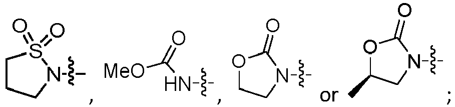
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃ alkyl;
R₄ is independently selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, - S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 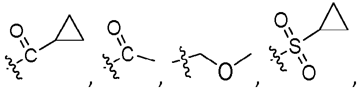 cyclopropyl, 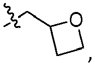 difluoromethyl, 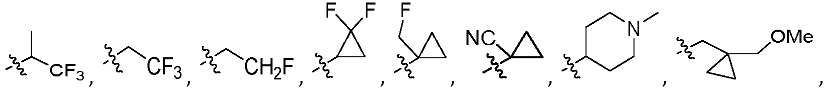 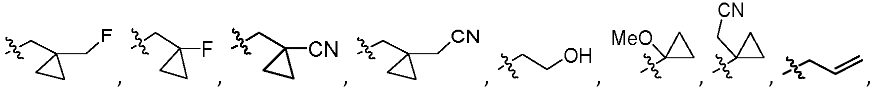 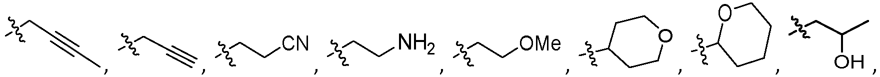 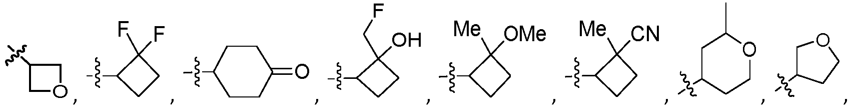 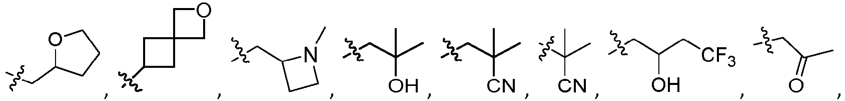 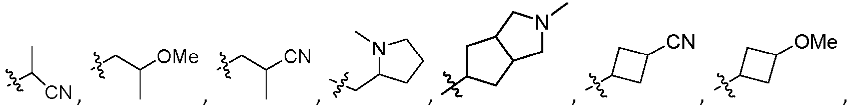 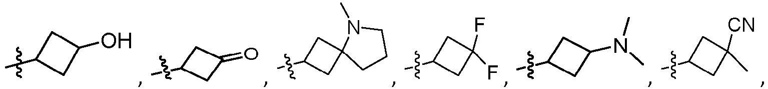 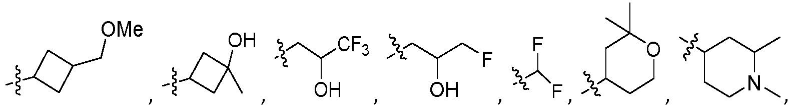 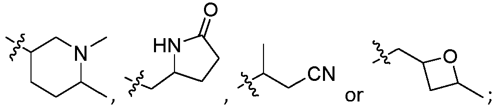 more preferably, R₄ is selected from fluorine, chlorine, cyano, trifluoromethyl, methyl, ethyl, nitro, hydroxyl, methoxy, -OCD₃, hydrogen, 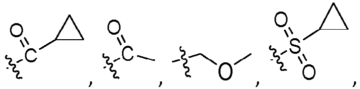 cyclopropyl, 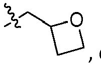 difluoromethyl, 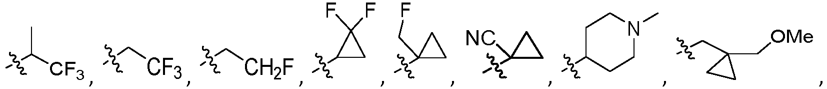 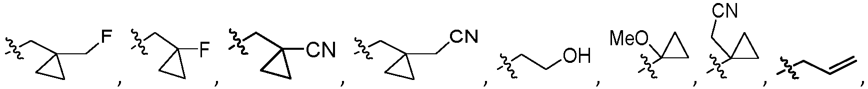 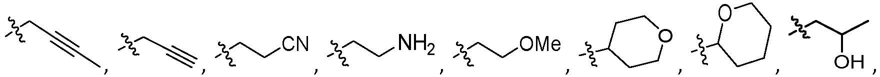 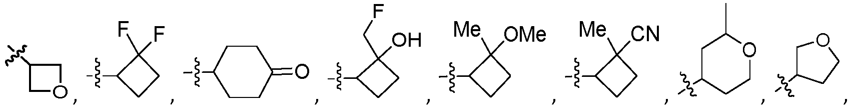 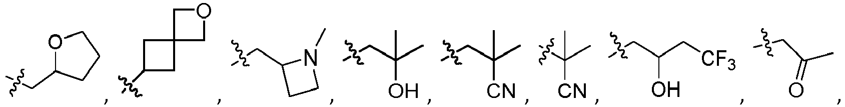 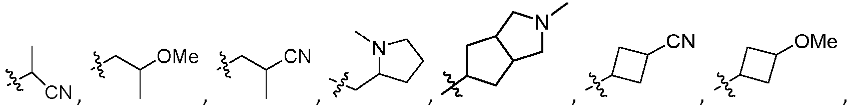 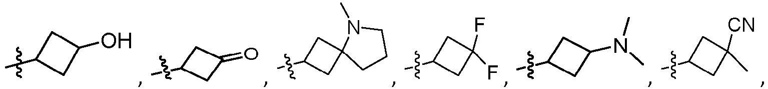 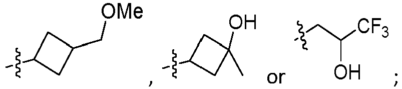
R₆₋₁ is independently selected from hydrogen, deuterium, oxo, thio, halogen, amino, hydroxyl, cyano, nitro, C₁₋₃ alkyl, C₁₋₃ haloalkyl, C₁₋₃ deuteroalkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is independently 0, 1 or 2;
p is independently 0, 1 or 2;
n5 is independently 0, 1 or 2.

In certain embodiments of the present invention, the compound is further as represented by general formula (VII-2-1-1), (VII-3-1-1) or (VII-4-1-1): 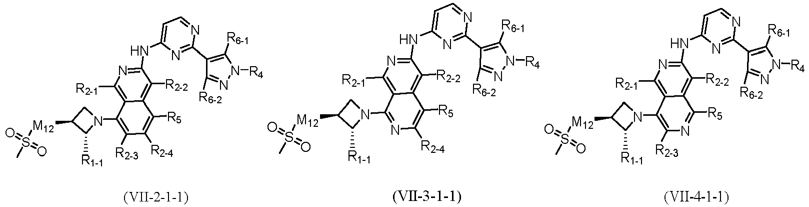 wherein
M₁₂ is independently selected from a bond, NR₉ or CR₁₀R₁₁;
R₉ is independently selected from hydrogen, deuterium, methyl, ethyl, monofluoromethyl, difluoromethyl or trifluoromethyl;
R₁₀ is independently selected from hydrogen, deuterium, fluorine, chlorine, bromine, methyl, ethyl, monofluoromethyl, difluoromethyl or trifluoromethyl;
R₁₁ is independently selected from hydrogen, deuterium, fluorine, chlorine, bromine, methyl, ethyl, monofluoromethyl, difluoromethyl or trifluoromethyl;
R₁₋₁ is independently selected from hydrogen, deuterium, fluorine, chlorine, bromine, methyl, ethyl, monofluoromethyl, difluoromethyl or trifluoromethyl;
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₅ is independently selected from isopropyl, -CH(Me)OMe or -N(Me)₂;
R₄ is independently selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, - S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 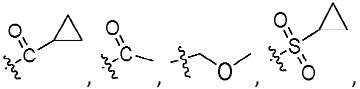 cyclopropyl, 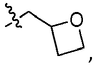 difluoromethyl, 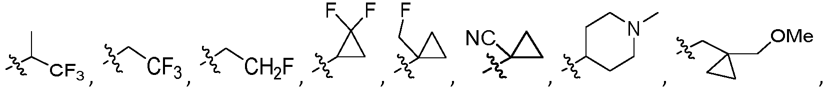 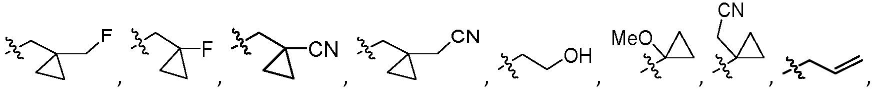 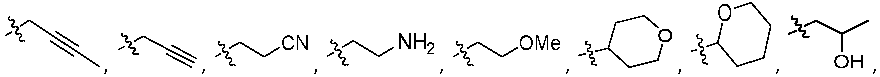 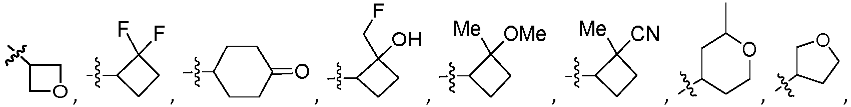 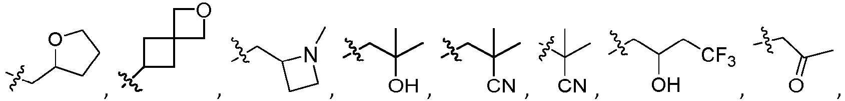 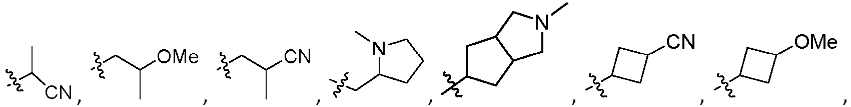 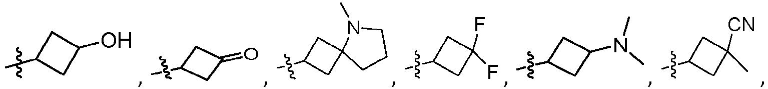 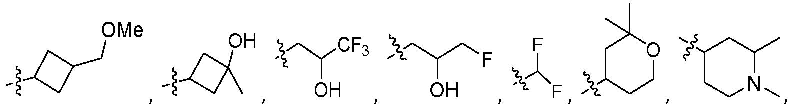 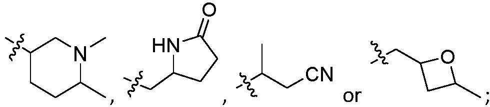 more preferably, R₄ is selected from fluorine, chlorine, cyano, trifluoromethyl, methyl, ethyl, nitro, hydroxyl, methoxy, -OCD₃, hydrogen, 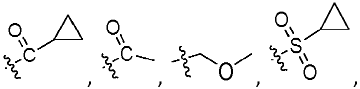 cyclopropyl, 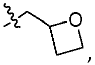 difluoromethyl, 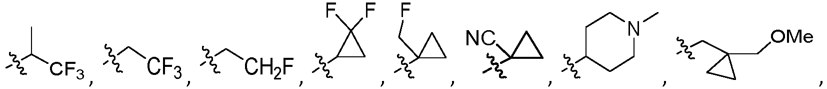 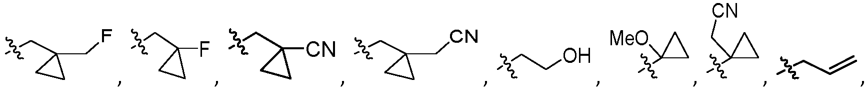 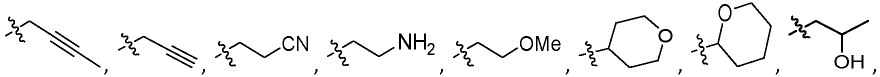 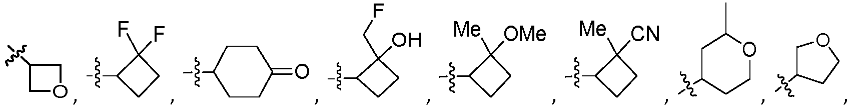 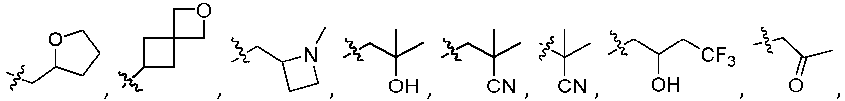 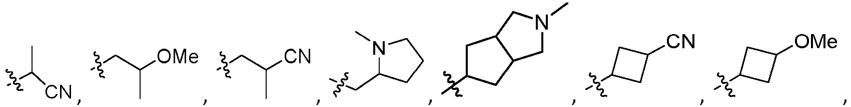 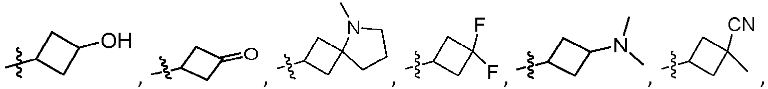 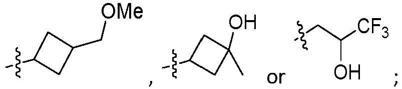
R₆₋₁ is independently selected from hydrogen, deuterium, oxo, halogen, amino, hydroxyl, cyano, nitro, C₁₋₃ alkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro and C₁₋₃ alkyl;
R₄ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is independently 0, 1 or 2.

In certain embodiments of the present invention, the compound is further as represented by general formula (VII-2-1-2), (VII-3-1-2) or (VII-4-1-2): 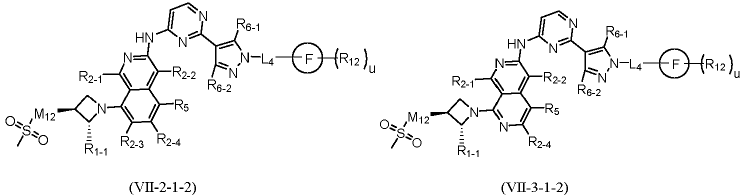 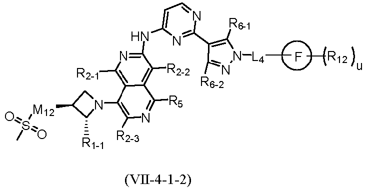 wherein
M₁₂ is independently selected from a bond, NR₉ or CR₁₀R₁₁;
R₉ is independently selected from hydrogen, deuterium, methyl, ethyl, monofluoromethyl, difluoromethyl or trifluoromethyl;
R₁₀ is independently selected from hydrogen, deuterium, fluorine, chlorine, bromine, methyl, ethyl, monofluoromethyl, difluoromethyl or trifluoromethyl;
R₁₁ is independently selected from hydrogen, deuterium, fluorine, chlorine, bromine, methyl, ethyl, monofluoromethyl, difluoromethyl or trifluoromethyl;
R₁₋₁ is independently selected from hydrogen, deuterium, fluorine, chlorine, bromine, methyl, ethyl, monofluoromethyl, difluoromethyl or trifluoromethyl;
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₅ is independently selected from isopropyl, -CH(Me)OMe or -N(Me)₂;
L₄ is independently selected from a bond, C₁₋₃ alkylene, C₂₋₄ alkenylene, C₂₋₄ alkynylene, - (CH₂)ₙ₇NH(CH₂)ₙ₈- or -(CH₂)ₙ₉O(CH₂)ₙ₁₀-, and the C₁₋₃ alkylene, C₂₋₄ alkenylene, C₂₋₄ alkynylene, - (CH₂)ₙ₇NH(CH₂)ₙ₈- and -(CH₂)ₙ₉O(CH₂)ₙ₁₀- are optionally substituted with one or more of halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl;
ring F is independently selected from C₃₋₆ cycloalkyl or 3- to 8-membered heterocyclyl;
preferably, ring F is independently selected from 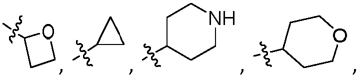 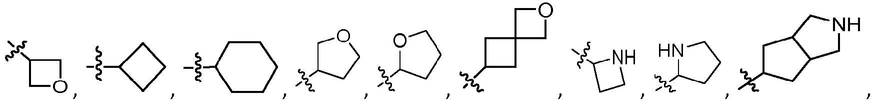 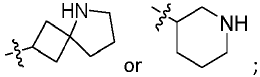 more preferably, ring F is independently selected from 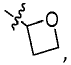 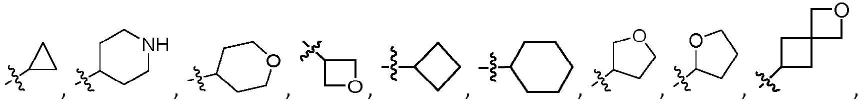 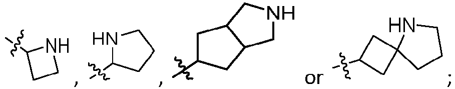
R₁₂ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy or C₁₋₃ hydroxyalkyl; the C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃hydroxyalkyl are optionally substituted with one or more of halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl;
R₆₋₁ is independently selected from hydrogen, deuterium, oxo, halogen, amino, hydroxyl, cyano, nitro, C₁₋₃ alkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro and C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is independently 0, 1 or 2;
n7 is independently 0, 1 or 2;
n8 is independently 0, 1 or 2;
n9 is independently 0, 1 or 2;
n10 is independently 0, 1 or 2;
u is independently 0, 1 or 2.

The present invention further provides a method for preparing the compound represented by general formula (II-G), or the stereoisomer or pharmaceutically acceptable salt thereof, wherein the method comprises the following steps: reacting the compound represented by formula (XV) with the compound represented by formula (XVI) to obtain a compound represented by formula (II-G), 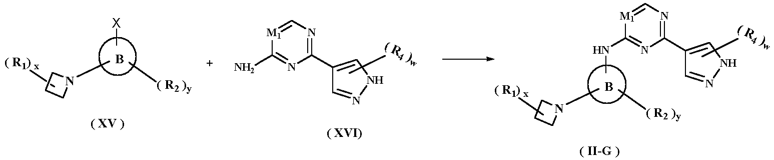
wherein X is halogen, preferably chlorine;
R₁, x, ring B, R₂, y, M₁, R₄ and w are the same as described above.

The present invention further provides a method for preparing the compound represented by general formula (VII-1), or the stereoisomer or pharmaceutically acceptable salt thereof, wherein the method comprises the following steps: reacting the compound represented by formula (XV-1) with the compound represented by formula (XVI-1) to obtain a compound represented by formula (VII-1), 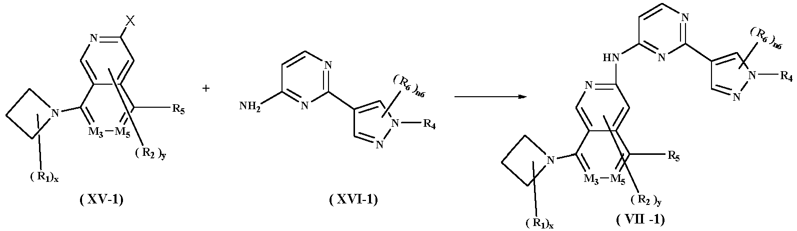
wherein X is halogen, preferably chlorine;
R₁, x, M₃, M₅, R₅, R₂, y, R₆, R₄ and n6 are the same as described above.

The present invention further provides a method for preparing the compound represented by general formula (VII-2), or the stereoisomer or pharmaceutically acceptable salt thereof, wherein the method comprises the following steps: reacting the compound represented by formula (XV-2) with the compound represented by formula (XVI-2) to obtain a compound represented by formula (VII-2), 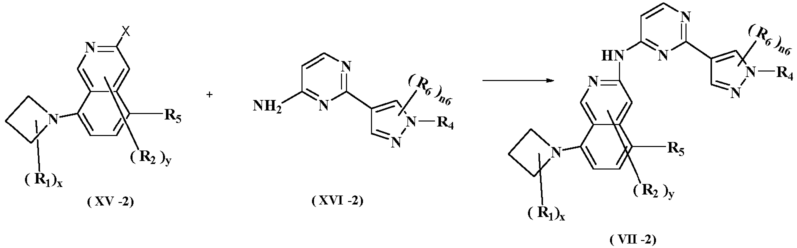
wherein X is halogen, preferably chlorine;
R₁, x, R₅, R₂, y, R₆, R₄ and n6 are the same as described above.

The present invention further provides a method for preparing the compound represented by general formula (VII-2-1), or the stereoisomer or pharmaceutically acceptable salt thereof, wherein the method comprises the following steps: reacting the compound represented by formula (XV-3) with the compound represented by formula (XVI-3) to obtain a compound represented by formula (VII-2-1), 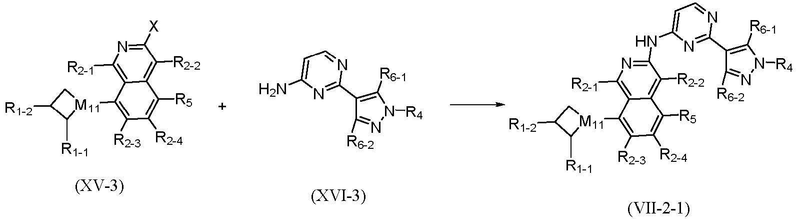
wherein X is halogen, preferably chlorine;
R₁₋₁, R₁₋₂, M₁₁, R₂₋₁, R₂₋₂, R₂₋₃, R₂₋₄, R₅, R₆₋₁, R₆₋₂ and R₄ are the same as described above.

The present invention further provides a method for preparing the compound represented by general formula (VII-2-1-1), or the stereoisomer or pharmaceutically acceptable salt thereof, wherein the method comprises the following steps: reacting the compound represented by formula (XV-4) with the compound represented by formula (XVI-4) to obtain a compound represented by formula (VII-2-1-1), 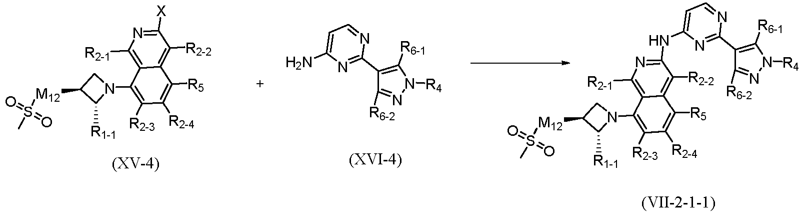
wherein X is halogen, preferably chlorine;
R₁₋₁, M₁₂, R₂₋₁, R₂₋₂, R₂₋₃, R₂₋₄, R₅, R₆₋₁, R₆₋₂ and R₄ are the same as described above.

The present invention further relates to a pharmaceutical composition, which comprises a therapeutically effective dose of the compounds of various general formulas as mentioned above, the stereoisomers or pharmaceutically acceptable salts thereof and one or more pharmaceutically acceptable carriers, diluents or excipients.

In certain embodiments of the present invention, the weight percentage of the compound or the stereoisomer or pharmaceutically acceptable salt thereof in the composition is 0.1-95%, preferably 0.5-85%, more preferably 1-60%, further preferably 10-50%, further more preferably 15-40%, further more preferably 20-30%, and further more preferably 20-25% (based on the total weight of the pharmaceutical composition).

In certain embodiments of the present invention, the compound or the stereoisomer or pharmaceutically acceptable salt thereof is administered at a dose of 1 mg-1000 mg, such as 1 mg, 5 mg, 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg or 1000 mg (or any integer therebetween).

The present invention further relates to the use of the compounds of various general formulas as mentioned above, the stereoisomers or pharmaceutically acceptable salts thereof, or the pharmaceutical composition as mentioned above in the preparation of EGFR inhibitor.

The present invention further relates to the use of the compounds of various general formulas as mentioned above, the stereoisomers or pharmaceutically acceptable salts thereof, or the pharmaceutical composition as mentioned above in the preparation of a drug for the treatment of a cancer; preferably, the cancer is selected from ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular carcinoma, gastric cancer, gastrointestinal stromal tumor (GIST), thyroid cancer, bile duct cancer, endometrial cancer, renal cancer, anaplastic large cell lymphoma, acute myeloid leukemia (AML), multiple myeloma, melanoma or mesothelioma; more preferably, the cancer is non-small cell lung cancer.

The present invention further relates to a method for preventing and/or treating EGFR-related diseases, which comprises administering to the patient a therapeutically effective dose of the compounds of various general formulas, the stereoisomers or pharmaceutically acceptable salts thereof, or the pharmaceutical composition thereof. The EGFR-related disease is ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular carcinoma, gastric cancer, gastrointestinal stromal tumor (GIST), thyroid cancer, bile duct cancer, endometrial cancer, renal cancer, anaplastic large cell lymphoma, acute myeloid leukemia (AML), multiple myeloma, melanoma or mesothelioma, preferably non-small cell lung cancer.

In certain embodiments of the present invention, the EGFR is a mutated EGFR, preferably with one or more mutations of Del19, L858R, T790M or C797S, and more preferably with Del19, L858R, L858R/T790M, Del19/T790M, Del19/C797S, L858R/C797S, Del19/T790M/C797S or L858R/T790M/C797S mutation.

In certain embodiments of the present invention, the cancer is a cancer with EGFR Del19, L858R, L858R/T790M, Del19/T790M, Del19/C797S, L858R/C797S, Del19/T790M/C797S or L858R/T790M/C797S mutation.

In certain embodiments of the present invention, the cancer is a non-small cell lung cancer with EGFR L858R/T790M, Del19/T790M, Del19/C797S, L858R/C797S, Del19/T790M/C797S or L858R/T790M/C797S mutation.

The present invention has obtained a series of compounds through research. These compounds have strong inhibitory effects on EGFR. They not only have good inhibitory activity against EGFR with three mutations, but also have strong inhibitory effects against EGFR with two mutations. In addition, they have excellent pharmacokinetic properties and significant pharmacological effects, with good clinical development prospects.

### Detailed Description of the Invention

Unless stated to the contrary, the terms used in the description and claims have the following meanings.

The term "alkyl" refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, the substituent can be substituted at any available connection point, and the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group. In the present invention, alkyl is preferably methyl, ethyl, isopropyl, tert-butyl, haloalkyl, deuteroalkyl, alkoxy-substituted alkyl and hydroxyl-substituted alkyl.

The term "alkylene" refers to one hydrogen atom of alkyl being further substituted, for example: "methylene" refers to -CH₂-, "ethylene" refers to -(CH₂)₂-, "propylene" refers to - (CH₂)₃-, and "butylene" refers to -(CH₂)₄-, etc. The term "alkenyl" refers to an alkyl group as defined above consisting of at least two carbon atoms and at least one carbon-carbon double bond, such as ethenyl, 1-propenyl, 2-propenyl, 1-, 2- or 3 -butenyl, etc. Alkenyl can be substituted or unsubstituted. When the alkenyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent, and a cycloalkyl ring comprises 3 to 20 carbon atoms, preferably 3 to 12 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a polycyclic group with 5 to 20 membered monocyclic rings sharing one carbon atom (called a spiro atom). It may contain one or more double bonds, but no ring has a completely conjugated π electron system. Preferably, the spirocycloalkyl is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, it is a 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 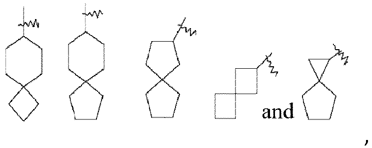 etc.;

Also included are spirocycloalkyl groups in which monospirocycloalkyl and heterocycloalkyl share a spiro atom. Non-limiting examples include: 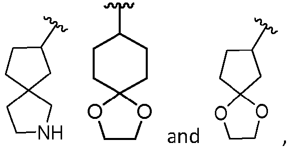 etc.

The term "fused cycloalkyl" refers to a 5- to 20-membered all-carbon polycyclic group in which each ring in the system shares an adjacent pair of carbon atoms with other rings in the system, one or more ring of which may contain one or more double bonds, but no ring has a fully conjugated π electron system. Preferably, the fused cycloalkyl is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 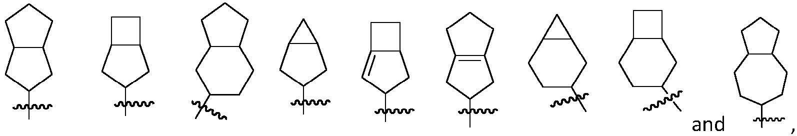 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, the bridged cycloalkyl is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

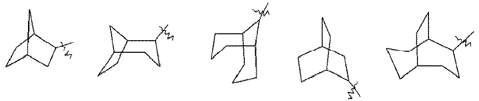

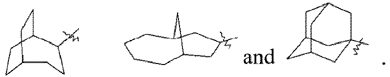

The cycloalkyl ring can be fused to an aryl, heteroaryl or heterocycloalkyl ring, wherein the ring connected to the parent structure is cycloalkyl, and the non-limiting examples include indanyl, tetrahydronaphthyl, benzocycloheptanyl, etc. Cycloalkyl can be optionally substituted or unsubstituted. When the cycloalkyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen, P(O)ₚₚ or S(O)ₘₘ (wherein pp and mm are integers of 0 to 2), but excluding ring moieties of -O-O-, -O-S- or -S-S-, and the remaining ring atoms are carbon. Preferably, the heterocyclyl comprises 3 to 12 ring atoms, of which 1-4 are heteroatoms; more preferably, the heterocyclyl comprises 3 to 8 ring atoms or comprises 4 to 10 ring atoms; most preferably, the heterocyclyl comprises 3 to 8 ring atoms; further preferably, the heterocyclyl is a 3- to 8-membered heterocyclyl group comprising 1-3 nitrogen atoms, which is optionally substituted with 1-2 oxygen atoms, sulfur atoms or an oxo group, including nitrogen-containing monocyclic heterocyclyl, nitrogen-containing spiro heterocyclyl or nitrogen-containing fused heterocyclyl.

Non-limiting examples of monocyclic heterocyclyl include azetidinyl, pyrrolidyl, imidazolidinyl, tetrahydrofuryl, tetrahydrothiophenyl, dihydroimidazolyl, dihydrofuryl, dihydropyrazolyl, dihydropyrrolyl, piperidyl, piperazinyl, morpholinyl, thiomorpholinyl, homopiperazinyl, azepinyl, 1,4-diazacycloheptyl, pyranyl, etc., preferably pyrrolidyl, morpholinyl, piperidyl, azepinyl, 1,4-diazacycloheptyl 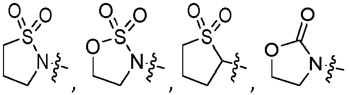 and piperazinyl. Polycyclic heterocyclyl includes spiro, fused and bridged heterocyclyl, wherein the spiro, fused and bridged heterocyclyl groups are optionally connected to other groups through a single bond, or further fused to other cycloalkyl, heterocyclyl, aryl and heteroaryl through any two or more atoms on the ring.

The term "spiroheterocyclyl" refers to a 5- to 20-membered polycyclic heterocyclic group with monocyclic rings sharing one atom (called a spiro atom), wherein one or more ring atoms are heteroatoms selected from nitrogen, oxygen, P(O)ₚₚ or S(O)ₘₘ (wherein pp and mm are integers of 0 to 2), and the remaining ring atoms are carbon. Spiroheterocyclyl may contain one or more double bonds, but no ring has a fully conjugated π electron system. Preferably, the spiro heterocyclyl is 6- to 14-membered, and more preferably 7- to 10-membered. According to the number of shared spiro atoms between the rings, the spiro heterocyclyl is divided into monospiroheterocyclyl, bispiroheterocyclyl or polyspiroheterocyclyl, preferably monospiroheterocyclyl and bispiroheterocyclyl. More preferably, it is a 3-membered/5-membered, 3-membered/6-membered, 4-membered/4-membered, 4-membered/5-membered, 4-membered/6-membered, 5-membered/5-membered or 5-membered/6-membered monospiro heterocyclyl. Non-limiting examples of spiro heterocyclyl include: 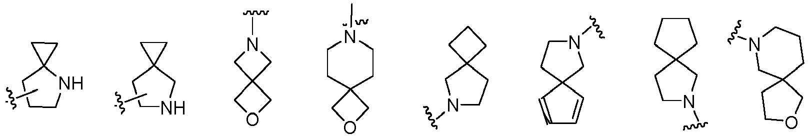 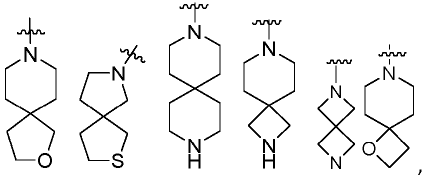 etc.

The term "fused heterocyclyl" refers to a 5- to 20-membered polycyclic heterocyclic group with each ring in the system sharing an adjacent pair of atoms with other rings in the system, and one or more rings may contain one or more double bonds, but no ring has a fully conjugated π electron system, wherein one or more ring atoms are heteroatoms selected from nitrogen, oxygen, P(O)ₚₚ or S(O)ₘₘ (wherein pp and mm are integers of 0 to 2), and the remaining ring atoms are carbon. Preferably, the fused heterocyclyl is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include: 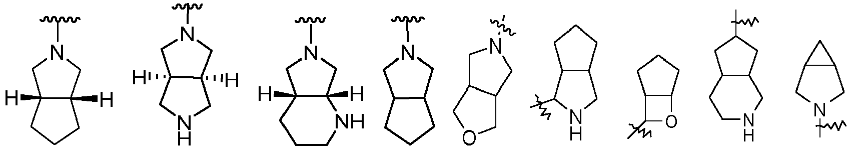 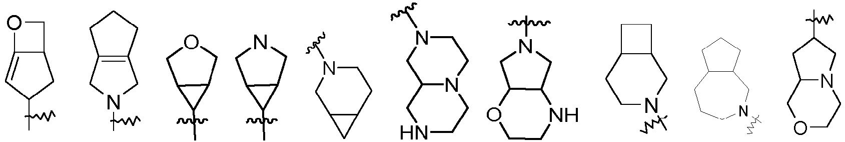 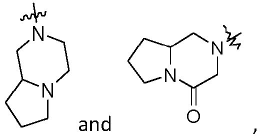 etc.

The term "bridged heterocyclyl" refers to a 5- to 14-membered polycyclic heterocyclic group with any two rings sharing two atoms that are not directly connected. It may contain one or more double bonds, but no ring has a fully conjugated π electron system, wherein one or more ring atoms are heteroatoms selected from nitrogen, oxygen, P(O)ₚₚ or S(O)ₘₘ (wherein pp and mm are integers of 0 to 2), and the remaining ring atoms are carbon. Preferably, the bridged heterocyclyl is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 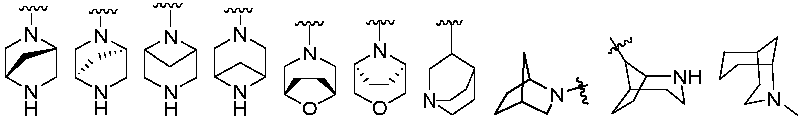 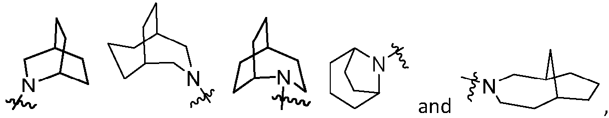 etc.

The heterocyclyl ring can be fused to an aryl, heteroaryl or cycloalkyl ring, wherein the ring connected to the parent structure is heterocyclyl, and the non-limiting examples thereof include: 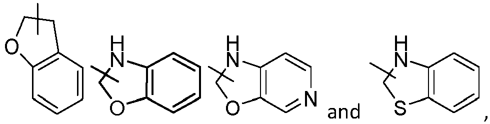 etc.

Heterocyclyl can be optionally substituted or unsubstituted. When the heterocyclyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl. The aryl ring can be fused to heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl and benzo 3- to 6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is aryl ring, and the non-limiting examples thereof include: 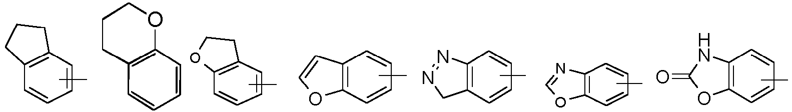 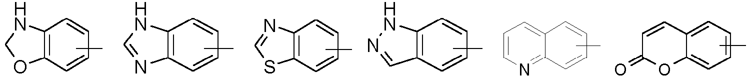 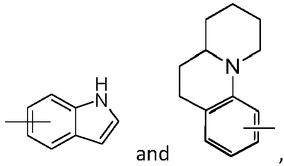 etc.

Aryl can be substituted or unsubstituted. When the aryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "heteroaryl" refers to a heteroaromatic system containing 1 to 4 heteroatoms and 5 to 14 ring atoms, wherein the heteroatoms are selected from oxygen, sulfur and nitrogen. The heteroaryl is preferably 5- to 12-membered, more preferably 8- to 11-membered, 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl or thiazolyl; more preferably, pyrazolyl, pyrrolyl and oxazolyl. The heteroaryl ring can be fused to an aryl, heterocyclyl or cycloalkyl ring, wherein the ring connected to the parent structure is heteroaryl ring, and the non-limiting examples thereof include: 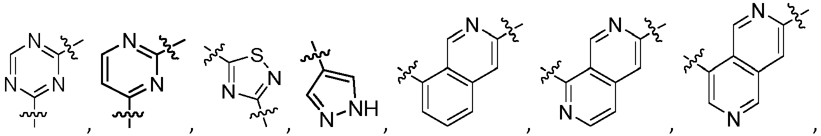 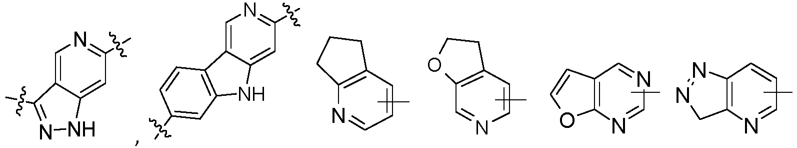 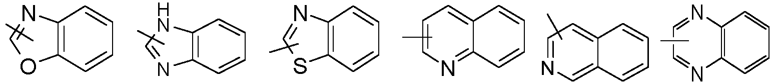 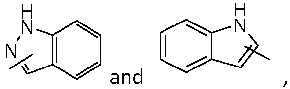 etc.

Heteroaryl can be optionally substituted or unsubstituted. When the heteroaryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkoxy" refers to -O- (alkyl) and -O- (unsubstituted cycloalkyl), where alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

"Haloalkyl" refers to alkyl substituted with one or more halogen, wherein alkyl is defined as above.

"Haloalkoxy" refers to alkoxy substituted with one or more halogen, wherein alkoxy is defined as above.

"Hydroxyalkyl" refers to alkyl substituted with hydroxyl, wherein alkyl is defined as above.

"Alkenyl" refers to a chain alkenyl group, also known as an alkene group, preferably alkyl containing 2 to 8 carbon atoms, more preferably alkyl containing 2 to 6 carbon atoms, and most preferably alkyl containing 2 to 3 carbon atoms, wherein the alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

"Alkynyl" refers to CH≡C-, preferably alkyl containing 2 to 8 carbon atoms, more preferably alkyl containing 2 to 6 carbon atoms, and most preferably alkyl containing 2 to 3 carbon atoms. wherein the alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkenylcarbonyl" refers to -C(O)-(alkenyl), wherein alkenyl is defined as above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

"Hydroxyl" refers to an -OH group.

"Halogen" refers to fluorine, chlorine, bromine or iodine.

"Amino" refers to -NH₂.

"Cyano" refers to -CN.

"Nitro" refers to -NO₂.

"Carbonyl" refers to -C(O)-.

"Carboxyl" refers to -C(O)OH.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"Et₂O" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"Pd₂(dba)₃" refers to tris(dibenzylideneacetone)dipalladium.

"Dppf" refers to 1,1'-bisdiphenylphosphine ferrocene.

"HATU" refers to 2-(7-azabenzotriazol-1-yl)-N,N,N',N'-tetramethyluronium hexafluorophosphate.

"KHMDS" refers to potassium hexamethyldisilamide.

"LiHMDS" lithium bistrimethylsilylamide.

"MeLi" refers to lithium methyl.

"n-BuLi" refers to n-butyl lithium.

"NaBH(OAc)₃" refers to sodium triacetoxyborohydride.

Different terms such as "X is selected from A, B, or C", "X is selected from A, B and C", "X is A, B or C", "X is A, Band C" all express the same meaning, i.e., X can be any one or more of A, B, and C.

The hydrogen atoms described in the present invention can be replaced with its isotope deuterium, and any hydrogen atom in the example compounds involved in the present invention can also be replaced with a deuterium atom.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, and the description includes the case where the heterocyclic group is substituted with alkyl and the case where the heterocyclic group is not substituted with alkyl.

**"Substituted"** refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, the amino group having a free hydrogen or a hydroxyl group may be unstable when combined the carbon atoms having an unsaturated (e.g., olefinic) bond.

The "more" in "substituted with one or more of..." can mean 2, 3, 4, 5, 6 or more than 6.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

"Pharmaceutical composition" denotes a mixture containing one or more of the compounds as described herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

"Pharmaceutically acceptable salts" refer to salts of the compounds of the present invention, which are safe and effective when used in mammals, and have appropriate biological activity.

### Detailed Description of Embodiments

The present invention will be further described below in conjunction with examples, but these examples are not meant to limit the scope of the present invention.

### Example

The structure of the compound of the present invention is determined by nuclear magnetic resonance (NMR) or/and liquid chromatography-mass spectrometry (LC-MS). NMR chemical shift (δ) is given in the unit of 10⁻⁶ (ppm). NMR is determined with Bruker AVANCE-400 nuclear magnetic resonance instrument; the solvents for determination are deuterated dimethyl sulfoxide (DMSO-*d₆*), deuterated methanol (CD₃OD) and deuterated chloroform (CDCl₃); and the internal standard is tetramethylsilane (TMS).

Agilent 1200 Infinity Series mass spectrometer is used for LC-MS. HPLC uses Agilent 1200DAD high-pressure liquid chromatograph (Sunfire C18 150 × 4.6 mm column) and Waters 2695-2996 high-pressure liquid chromatograph (Gimini C₁₈ 150 × 4.6 mm column).

Yantai Huanghai HSGF254 or Qingdao GF254 silica gel plate is used as a thin layer chromatography silica plate, and TLC is of the specification of 0.15 mm-0.20 mm, and the specification when separating and purifying a product by thin layer chromatography is 0.4 mm - 0.5 mm. For the column chromatography, Yantai Huanghai silica gel of 200-300 mesh silica gel is generally used as a carrier.

The starting materials in the examples of the present invention are known and can be purchased on the market, or can be synthesized using or according to methods known in the art.

Unless otherwise specified, all reactions of the present invention are carried out under continuous magnetic stirring in a dry nitrogen or argon atmosphere, the solvent is a dry solvent, and the reaction temperature unit is degrees Celsius.

### Intermediate 1

### 3-((methanesulfonyl)methyl)azetidine

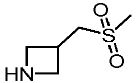

### Step 1 Synthesis of tert-butyl 3-((methylthio)methyl)azetidine-1-carboxylate

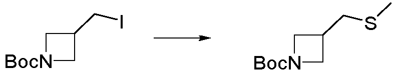

Tert-butyl 3-(iodomethyl)azetidine-1-carboxylate (2 g, 6.73 mmol) and sodium methyl mercaptide (970 mg, 13.50 mmol) were dissolved in ACN (15 mL) and H₂O (5 mL), and the mixture was warmed to 60°C and reacted for 12 hours. The reaction solution was cooled to room temperature, concentrated under reduced pressure, and then separated by column chromatography to obtain the target compound tert-butyl 3-((methylthio)methyl)azetidine-1-carboxylate (1.30 g, 88.9%).

MS m/z (ESI) :162.0 [M+H-56]⁺.

### Step 2 Synthesis of tert-butyl 3-((methanesulfonyl)methyl)azetidine-1-carboxylate

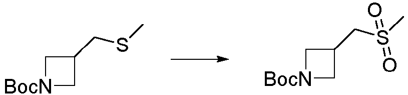

Tert-butyl 3-((methylthio)methyl)azetidine-1-carboxylate (1.30 g, 5.99 mmol) was dissolved in dichloromethane (15 mL), m-chloroperoxybenzoic acid (2 g, 12 mmol) was added and the mixture was stirred overnight. The reaction solution was washed with saturated sodium thiosulfate solution and saturated sodium chloride solution. The organic phase was dried over anhydrous sodium sulfate, concentrated under reduced pressure and separated by column chromatography to obtain the target compound tert-butyl 3-((methanesulfonyl)methyl)azetidine-1-carboxylate (1.30 g, 87.1%).

MS m/z (ESI) :194.0 [M+H-56]⁺.

### Step 3 Synthesis of 3-((methanesulfonyl)methyl)azetidine trifluoroacetate

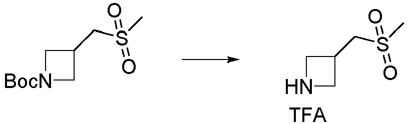

Tert-butyl 3-((methanesulfonyl)methyl)azetidine-1-carboxylate (1.30 g, 5.22 mmol) was dissolved in dichloromethane (15 mL), TFA (3 mL) was added, and the mixture was stirred for 4 hours. The reaction solution was concentrated under reduced pressure to obtain the crude target compound 3-((methanesulfonyl)methyl)azetidine trifluoroacetate (740 mg).

MS m/z (ESI) :150.0 [M+H]⁺.

### Intermediate 2

### 1,6-dichloro-4-isopropyl-2,7-diazanaphthalene

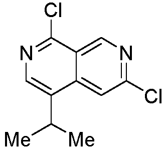

### Step 1 Synthesis of 6-chloro-4-iodo-2,7-diazanaphthalen-1(2H)-one

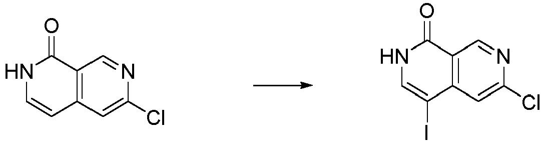

6-Chloro-2,7-diazanaphthalen-1(2H)-one (10 g, 55.37 mmol) was dissolved in DMF (60 mL), the reaction solution was cooled to 0°C, NIS (14.80 g, 66 mmol) was added in batches, and the mixture was warmed to room temperature and stirred 12 hours. The reaction solution was filtered, and the filtrate was concentrated under reduced pressure to obtain the crude target compound 6-chloro-4-iodo-2,7-diazanaphthalen-1(2H)-one (12 g).

MS m/z (ESI) :307.1 [M+H]⁺.

### Step 2 Synthesis of 1,6-dichloro -4-iodo-2,7-diazanaphthalene

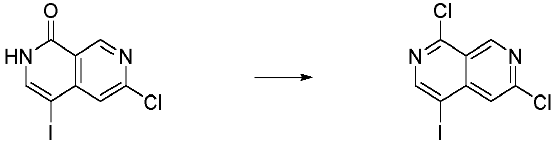

6-Chloro-4-iodo-2,7-diazanaphthalen-1(2H)-one (12 g, 39.15 mmol) was dissolved in phosphorus oxychloride (80 mL), and the mixture was warmed to 100°C and reacted for 2 hours. The reaction solution was cooled to room temperature, and concentrated under reduced pressure. The residue was subjected to liquid separation with dichloromethane and water. The organic phase was dried over anhydrous sodium sulfate, concentrated under reduced pressure and separated by column chromatography to obtain the target compound 1,6-dichloro -4-iodo-2,7-diazanaphthalene (10.60 g, 83.3%).

MS m/z (ESI) :325.1 [M+H]⁺.

### Step 3 Synthesis of 1,6-dichloro-4-(prop-1-en-2-yl)-2,7-diazanaphthalene

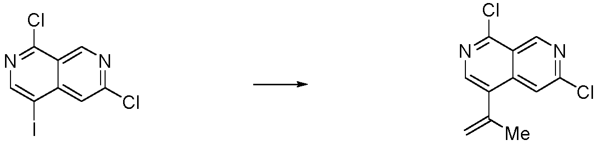

1,6-Dichloro -4-iodo-2,7-diazanaphthalene (10.60 g, 32.62 mmol) and 4,4,5,5-tetramethyl-2-(prop-1-en-2-yl)-1,3,2-dioxaborolane (5.50 g, 32.70 mmol) were mixed in dioxane (60 mL) and water (10 mL), potassium carbonate (13.60 g, 98.10 mmol) and bis(di-tert-butyl(4-dimethylaminophenyl)phosphine)dichloropalladium (II) (2.34 g, 3.30 mmol) were added, and the mixture was warmed to 50°C and reacted for 1 hour. The reaction solution was cooled to room temperature, and concentrated under reduced pressure. The residue was subjected to liquid separation with dichloromethane and water. The organic phase was dried over anhydrous sodium sulfate, concentrated under reduced pressure and separated by column chromatography to obtain the target compound 1,6-dichloro-4-(prop-1-en-2-yl)-2,7-diazanaphthalene (5 g, 64.1%).

MS m/z (ESI) :239.0 [M+H]⁺.

### Step 4 Synthesis of 1,6-dichloro-4-isopropyl-2,7-diazanaphthalene

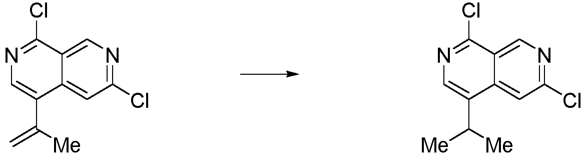

1,6-Dichloro -4-(prop-1-en-2-yl)-2,7-diazanaphthalene (5 g, 20.91 mmol) was dissolved in ethyl acetate (80 mL), platinum dioxide (5.72 mg, 25.20 mmol) was added, the reaction system was evacuated, and filled with hydrogen, the operations were repeated 3 times, and the mixture was reacted under hydrogen atmosphere for 12 hours. The reaction solution was filtered, and the filtrate was concentrated under reduced pressure and separated by column chromatography to obtain the target compound 1,6-dichloro-4-isopropyl-2,7-diazanaphthalene (4 g, 79.3%).

MS m/z (ESI) :241.0 [M+H]⁺.

### Intermediate 3

### (2R,3S)-2-methyl-3-((methanesulfonyl)methyl)azetidine

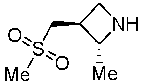

### Step 1 Synthesis of (2R,3S)-1-diphenylmethyl-2-methylazetidin-3-yl methanesulfonate

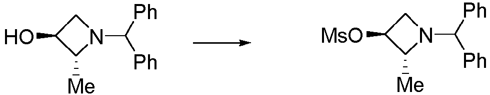

(2R,3S)-1-Diphenylmethyl-2-methylazetidin-3-ol (10 g, 39.50 mmol) was dissolved in dichloromethane (100 mL), triethylamine (4.80 g, 47.30 mmol) was added, the reaction solution was cooled to 0°C, methanesulfonyl chloride (5 g, 43.40 mmol) was slowly added dropwise, and the mixture was allowed to react at room temperature overnight. The reaction was quenched by adding water, and the mixture was subjected to liquid separation with dichloromethane and water. The organic phase was dried over anhydrous sodium sulfate, and concentrated under reduced pressure to obtain the crude target compound (2R, 3S)-1-diphenylmethyl-2-methylazetidin-3-yl methanesulfonate (12.80 g).

MS m/z (ESI) :332.2 [M+H]⁺.

### Step 2 Synthesis of methyl (S)-2-((2R,3S)-1-diphenylmethyl-2-methylazetidin-3-yl)-2-(methanesulfonyl)acetate

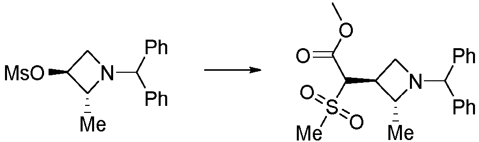

(2R,3S)-1-Diphenylmethyl-2-methylazetidin-3-yl methanesulfonate (12.80 g, 38.62 mmol) and methyl 2-(methylsulfonyl)acetate (7.70 g, 50.60 mmol) were dissolved in DMF (100 mL), sodium hydride (2.20 g, 60% in mineral oil, 55.50 mmol) was added in batches, and the mixture was reacted at room temperature for 15 minutes, and then warmed to 80°C and reacted overnight. The reaction solution was cooled to room temperature and quenched with a saturated ammonium chloride solution, and the mixture was subjected to liquid separation with ethyl acetate and water. The organic phase was dried over anhydrous sodium sulfate, concentrated and separated by column chromatography to obtain the target compound methyl (S)-2-((2R,3S)-1-diphenylmethyl-2-methylazetidin-3-yl)-2-(methanesulfonyl)acetate (11.60 g, 77.5%).

MS m/z (ESI) :388.2 [M+H]⁺.

### Step 3 Synthesis of (2R,3S)-1-diphenylmethyl-2-methyl-3-((methanesulfonyl) methyl)azetidine

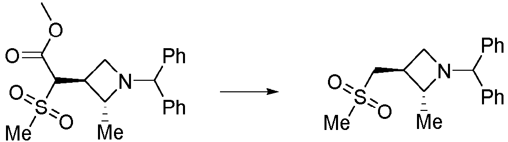

Methyl (S)-2-((2R,3S)-1-diphenylmethyl-2-methylazetidin-3-yl)-2-(methanesulfonyl)acetate (11.60 g, 29.94 mmol) was dissolved in DMA (120 mL), lithium chloride (10.50 g, 247.50 mmol) was added, and the mixture was warmed to 150°C and reacted for 2 hours. The reaction solution was cooled to room temperature, and subjected to liquid separation with ethyl acetate and water. The organic phase was dried over anhydrous sodium sulfate, concentrated under reduced pressure and separated by column chromatography to obtain the target compound (2R,3S)-1-diphenylmethyl-2-methyl-3-((methanesulfonyl)methyl)azetidine (9.20 g, 93.3%).

MS m/z (ESI) :330.0 [M+H]⁺.

### Step 4 Synthesis of (2R,3S)-2-methyl-3-((methanesulfonyl)methyl)azetidine

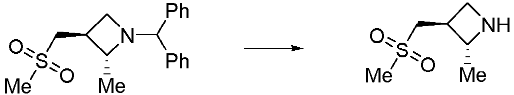

(2R,3S)-1-Diphenylmethyl-2-methyl-3-((methanesulfonyl)methyl)azetidine (9.20 g, 27.92 mmol) was dissolved in methanol (120 mL), TFA (5 mL) and palladium hydroxide (2.80 g) were added, and the reaction system was evacuated and filled with hydrogen. The operations were repeated three times, and the reaction solution was reacted overnight under hydrogen atmosphere. The reaction solution was filtered, and the filtrate was concentrated under reduced pressure to obtain the crude target compound (2R,3S)-2-methyl-3-((methanesulfonyl)methyl)azetidine (4 g).

MS m/z (ESI) :164.0 [M+H]⁺.

### Intermediate 4

### 8-bromo-3-chloro-6-fluoro-5-isopropylisoquinoline

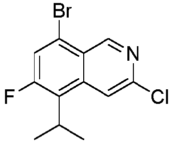

### Step 1 Synthesis of 3-chloro-6-fluoroisoquinoline

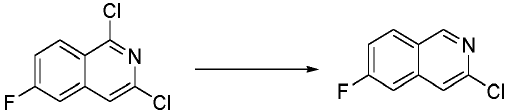

1,3-Dichloro-6-fluoroisoquinoline (7.50 g, 34.72 mmol) was dissolved in glacial acetic acid (40 mL) and hydriodic acid (20 mL, 45% aqueous), red phosphorus (2.69 g, 86.8 mmol) was added, and the temperature was warmed to 100°C and the mixture was reacted for 4 hours. The reaction solution was cooled to room temperature, concentrated, diluted with dichloromethane (100 mL), and washed with an aqueous saturated sodium carbonate solution. The organic phase was separated, dried over anhydrous sodium sulfate, and filtered. The organic solvent was concentrated under reduced pressure. The resulting mixture was separated by column chromatography to obtain the target compound 3-chloro-6-fluoroisoquinoline (4.90 g, 77.8%).

MS m/z (ESI) :182.0 [M+H]⁺.

### Step 2 Synthesis of 5-bromo-3-chloro-6-fluoroisoquinoline

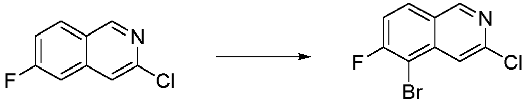

3-Chloro-6-fluoroisoquinoline (3.20 g, 17.62 mmol) was dissolved in concentrated sulfuric acid (20 mL), NBS (3.45 g, 19.38 mmol) was added, and the mixture was stirred at room temperature overnight. The reaction solution was slowly added to ice water, and the mixture was subjected to liquid separation with ethyl acetate and water. The organic phase was separated and dried over anhydrous sodium sulfate, filtered, and the organic solvent was concentrated under reduced pressure. The resulting mixture was separated by column chromatography to obtain the target compound 5-bromo-3-chloro-6-fluoroisoquinoline (4.0 g, 87.1%).

MS m/z (ESI) :260.0 [M+H]⁺.

### Step 3 Synthesis of 3-chloro-6-fluoro-5-(prop-1-en-2-yl)isoquinoline

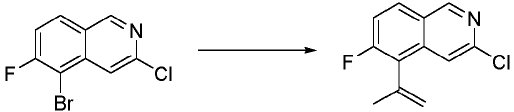

5-Bromo-3-chloro-6-fluoroisoquinoline (4.0 g, 15.36 mmol) and 4,4,5,5-tetramethyl-2-(prop-1-en-2-yl)-1,3,2-dioxaborolane (2.71 g, 16.72 mmol) were dissolved in 1,4-dioxane (20 mL) and water (3 mL), and [1,1'-bis(diphenylphosphine)ferrocene]palladium dichloride (562 mg, 0.77 mmol) and cesium carbonate (10.01 g, 30.71 mmol) were added, and the mixture was warmed to 80°C and reacted for 2 hours. The reaction solution was cooled to room temperature, concentrated under reduced pressure, and then separated by column chromatography to obtain the target compound 3-chloro-6-fluoro-5-(prop-1-en-2-yl)isoquinoline (3.0 g, 88.1%).

MS m/z (ESI) :222.0 [M+H]⁺.

### Step 4 Synthesis of 3-chloro-6-fluoro-5-isopropylisoquinoline

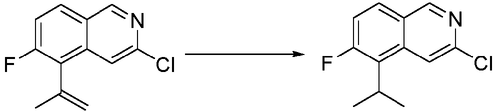

3-Chloro-6-fluoro-5-(prop-1-en-2-yl)isoquinoline (3.0 g, 13.53 mmol) was dissolved in ethyl acetate (30 mL), platinum dioxide (615 mg, 2.71 mmol) was added, and the mixture was stirred at room temperature for 4 hours under a hydrogen atmosphere. The reaction solution was filtered, and the filtrate was concentrated under reduced pressure and separated by column chromatography to obtain the target compound 3-chloro-6-fluoro-5-isopropylisoquinoline (2.10 g, 69.4%).

MS m/z (ESI) :224.1 [M+H]⁺.

### Step 5 Synthesis of 8-bromo-3-chloro-6-fluoro-5-isopropylisoquinoline

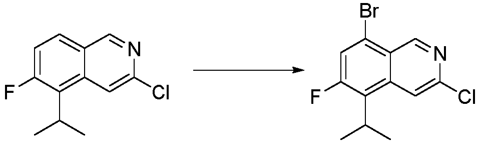

In an ice bath, to a solution of 3-chloro-6-fluoro-5-isopropylisoquinoline (800 mg, 3.58 mmol) in concentrated sulfuric acid (5 mL) was slowly added dibromohydantoin (1.12 g, 3.93 mmol) in batches, and the mixture was stirred in the ice bath for additional 0.5 hours. The reaction solution was carefully added to ice water and the mixture was subjected to liquid separation with dichloromethane and water. The organic phase was dried over anhydrous sodium sulfate, filtered, and the organic solvent was concentrated under reduced pressure. The resulting mixture was separated by column chromatography to obtain the target compound 8-bromo-3-chloro-6-fluoro-5-isopropylisoquinoline (320 mg, 29.6%).

MS m/z (ESI) :302.0 [M+H]⁺.

### Intermediate 5

### N-methyl-N-((2R,3S)-2-methylazetidin-3-yl)methanesulfonamide trifluoroacetate

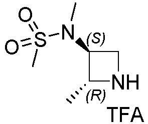

### Step 1 Synthesis of (2R,3S)-1-diphenylmethyl-2-methylazetidin-3-yl methanesulfonate

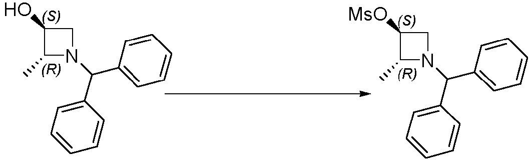

(2R,3S)-1-Diphenylmethyl-2-methylazetidin-3-ol (100 g, 397 mmol), triethylamine (110 mL, 793 mmol) and anhydrous dichloromethane (1000 mL) were added to a 2L reaction bottle. The mixture was cooled to 5°C under nitrogen atmosphere, and methanesulfonic anhydride (138 g, 795 mmol) was added in batches, and the mixture was warmed to room temperature and stirred for 2 h. The reaction solution was subjected to liquid separation with water and dichloromethane, and the organic phase was separated and washed sequentially with 5% sodium carbonate solution and a saturated sodium chloride solution. The organic phase was separated and dried over anhydrous sodium sulfate, and filtered, and the filtrate was concentrated under reduced pressure to obtain the crude target product (2R,3S)-1-diphenylmethyl-2-methylazetidin-3-yl methanesulfonate (138 g).

MS m/z (ESI) :332.1[M+H]⁺.

### Step 2 Synthesis of N-((2R,3S)-1-diphenylmethyl-2-methylazetidin-3-yl)-N-methyl methanesulfonamide

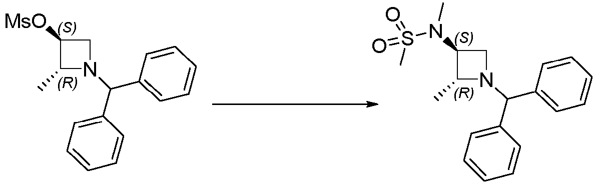

At room temperature, (2R,3S)-1-diphenylmethyl-2-methylazetidin-3-yl methanesulfonate (138 g, 0.42 mol) was dissolved in MeCN (950 mL), Cs₂CO₃ (273 g, 0.84 mol) and N-methyl methanesulfonamide (82 g, 0.75 mol) were added, and the mixture was stirred overnight at 80°C under nitrogen atmosphere. The reaction solution was cooled to room temperature, water (0.5 L) was added to the reaction solution, and the mixture was extracted with ethyl acetate (1L × 2). The organic phases were combined and washed with an aqueous saturated sodium chloride solution, and separated to obtain the organic phases, which were dried over anhydrous sodium sulfate and filtered, and the filtrate was concentrated under reduced pressure to remove the organic solvent and separated by column chromatography to obtain the target product N-((2R,3S)-1-diphenylmethyl-2-methylazetidin-3-yl)-N-methyl methanesulfonamide (110 g, 76.7 %).

MS m/z (ESI) :345.1 [M+H]⁺.

### Step 3 Synthesis of N-methyl-N-((2R,3S)-2-methylazetidin-3-yl)methanesulfonamide trifluoroacetate

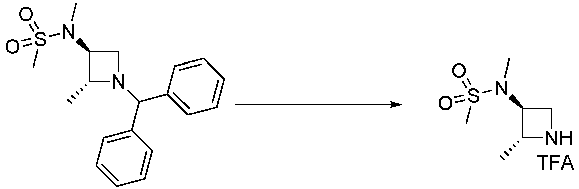

N-((2R,3S)-1-diphenylmethyl-2-methylazetidin-3-yl)-N-methyl methanesulfonamide (85 g, 0.247 mol) was dissolved in a mixed solution of MeOH (850 mL) and TFA (52 mL), 10% Pd(OH)₂/C (28 g) was added, and the mixture was stirred overnight at room temperature under H2 atmosphere. The reaction solution was filtered over celite, and the filtrate was concentrated under reduced pressure to obtain the crude target product N-methyl-N-((2R,3S)-2-methylazetidin-3-yl)methanesulfonamide trifluoroacetate (66 g).
¹H NMR(400 MHz, DMSO-*d₆*) *δ* 8.88 (s, 1H),4.62-4.56 (m, 1H), 4.31-4.25 (m, 1H), 4.10-4.06 (m, 1H), 3.95-3.91 (m, 1H), 2.95 (s, 3H), 2.83 (s, 3H), 1.40 (d, J = 6.0 Hz, 3H);
MS m/z (ESI) :179.1 [M+H]⁺.

### Intermediate 6

### 3-chloro-5-isopropyl-8-(3-((methanesulfonyl)methyl)azetidin-1-yl)isoquinoline

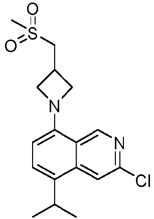

### Step 1 Synthesis of 8-bromo-3-chloroisoquinolin-5-yl trifluoromethanesulfonic acid

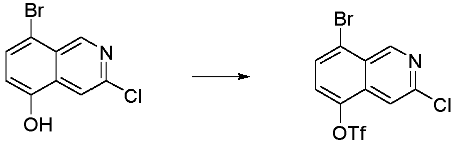

8-Bromo-3-chloroisoquinolin-5-ol (5 g, 19.50 mmol) was dissolved in dichloromethane (50 mL), TEA (7.90 g, 78 mmol) was added, and the reaction solution was cooled to -60°C, (TfO)₂O (16.50 g, 58.50 mmol) was slowly added dropwise, and the mixture was warmed to room temperature and reacted for 4 hours. The reaction solution was concentrated under reduced pressure and separated by column chromatography to obtain the target compound 8-bromo-3-chloroisoquinolin-5-yl trifluoromethanesulfonic acid (6.50 g, 85.3%).

MS m/z (ESI) :390.0 [M+H]⁺.

### Step 2 Synthesis of 8-bromo-3-chloro-5-(prop-1-en-2-yl)isoquinoline

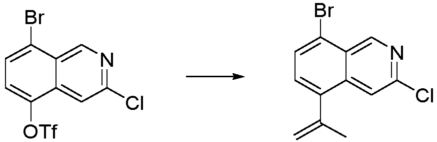

8-Bromo-3-chloroisoquinolin-5-yl trifluoromethanesulfonic acid (6.50 g, 16.64 mmol) was dissolved in dioxane (60 mL) and H₂O (6 mL), isopropenylboronic acid pinacol ester (4.20 g, 25 mmol), potassium carbonate (4.60 g, 33.40 mmol), and PdCl₂(dppf) (610 mg, 0.84 mmol) were added, and the mixture was warmed to 100°C and reacted for 4 hours. The reaction solution was cooled to room temperature, and concentrated. The residue was subjected to liquid separation with dichloromethane and water. The organic phase was dried over anhydrous sodium sulfate, concentrated under reduced pressure and separated by column chromatography to obtain the target compound 8-bromo-3-chloro-5-(prop-1-en-2-yl)isoquinoline (2.90 g, 61.7%).

MS m/z (ESI) :282.0 [M+H]⁺.

### Step 3 Synthesis of 8-bromo-3-chloro-5-isopropylisoquinoline

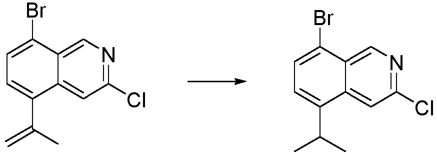

8-Bromo-3-chloro-5-(prop-1-en-2-yl)isoquinoline (2.90 g, 10.26 mmol) was dissolved in ethyl acetate (100 mL), PtO₂ (700 mg, 3.10 mmol) was added, the reaction system was evacuated, and filled with hydrogen, the operations were repeated 3 times, and the mixture was stirred under hydrogen atmosphere for 12 hours. The reaction solution was filtered, and the filtrate was concentrated under reduced pressure and separated by column chromatography to obtain the target compound 8-bromo-3-chloro-5-isopropylisoquinoline (2.70 g, 92.5%).

MS m/z (ESI) :284.0 [M+H]⁺.

### Step 4 Synthesis of 3-chloro-5-isopropyl-8-(3-((methanesulfonyl)methyl)azetidin-1-yl)isoquinoline

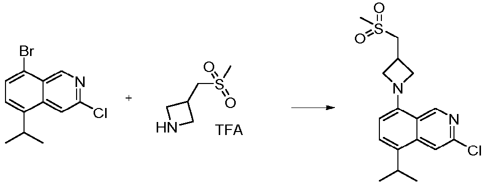

8-Bromo-3-chloro-5-isopropylisoquinoline (500 mg, 1.76 mmol) and 3-((methanesulfonyl)methyl)azetidine trifluoroacetate (435 mg, 1.77 mmol) were dissolved in dioxane (10 mL), cesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate, concentrated under reduced pressure and separated by column chromatography to obtain the target compound 3-chloro-5-isopropyl-8-(3-((methanesulfonyl)methyl)azetidin-1-yl)isoquinoline (286 mg, 46.0%).

MS m/z (ESI) :353.0 [M+H]⁺.

### Reference example 1

### N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

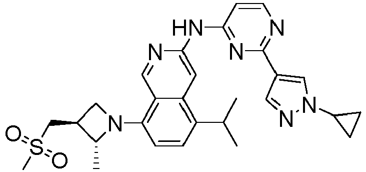

### Step 1 Synthesis of 3-chloro-5-isopropyl-8-((2R,3S)-2-methyl-3-((methanesulfonyl)methyl)azetidin-1-yl)isoquinoline

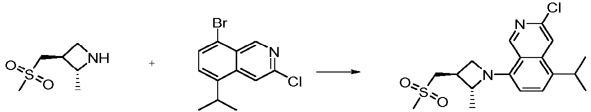

8-Bromo-3-chloro-5-isopropylisoquinoline (500 mg, 1.76 mmol) and (2R,3S)-2-methyl-3-((methanesulfonyl)methyl)azetidine (462 mg, 2.83 mmol) were dissolved in dioxane (10 mL), cesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound 3-chloro-5-isopropyl-8-((2R,3S)-2-methyl-3-((methanesulfonyl)methyl)azetidin-1-yl)isoquinoline (318 mg, 49.2%).

MS m/z (ESI) :367.1 [M+H]⁺.

### Step 2 Synthesis of 2-(1-cyclopropyl-1H-pyrazol-4-yl)pyrimidin-4-amine

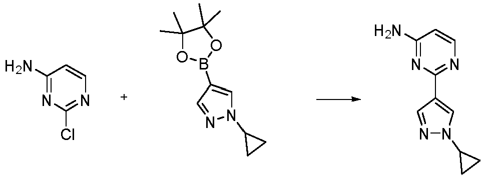

2-Chloropyrimidin-4-amine (1.29 g, 10 mmol), 1-cyclopropyl-4-(4,4,5,5-tetramethyl-1,3,2-dioxaborolan-2-yl)-1H-pyrazole (2.58 g, 11 mmol) and potassium carbonate (3.46 g, 25 mmol) were mixed in dioxane (20 mL) and water (2 mL), bis(triphenylphosphine)palladium dichloride (702 mg, 1 mmol) was added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound 2-(1-cyclopropyl-1H-pyrazol-4-yl)pyrimidin-4-amine (1.05 g, 52.2%).

MS m/z (ESI) :202.1 [M+H]⁺.

### Step 3 Synthesis of N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

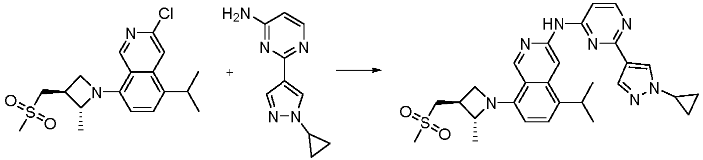

3-Chloro-5-isopropyl-8-((2R,3S)-2-methyl-3-((methanesulfonyl)methyl)azetidin-1-yl)isoquinoline (100 mg, 0.27 mmol) and 2-(1-cyclopropyl-1H-pyrazol-4-yl)pyrimidin-4-amine (54 mg, 0.27 mmol) were dissolved in dioxane (5 mL), cesium carbonate (274 mg, 0.84 mmol) and

BrettPhos Pd G3 (24 mg, 27 µmol) were added, and the mixture was warmed to 100°C and stirred for 12 hours. The reaction solution was cooled to room temperature, concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (31 mg, 21.6%).
¹H NMR (400 MHz, DMSO-*d*₆) *δ* 10.22 (s, 1H), 9.09 (s, 1H), 8.82 (s, 1H), 8.36-8.32 (m, 2H), 8.05 (s, 1H), 7.46 (d, J = 7.9 Hz, 1H), 7.15-7.05 (m, 1H), 6.59 (d, J = 8.1 Hz, 1H), 4.68 (t, J = 7.5 Hz, 1H), 4.25-4.16 (m, 1H), 3.87-3.80 (m, 1H), 3.68-3.51 (m, 4H), 3.00 (s, 3H), 2.95-2.85 (m, 1H), 1.43 (d, *J* = 6.0 Hz, 3H), 1.38 (dd, *J* = 6.8, 3.9 Hz, 6H), 1.17-1.11 (m, 2H), 1.06-1.00 (m, 2H);
MS m/z (ESI) :532.2 [M+H]⁺.

### Reference example 2

### Preparation of 2-(3-chloro-4-(4-((5-isopropyl-8-((2R,3S)-2-methyl-3-((methanesulfonyl)methyl)azabut-1-yl)isoquinolin-3-yl)amino)pyrimidin-2-yl)-1H-pyrazol-1-yl)ethanol

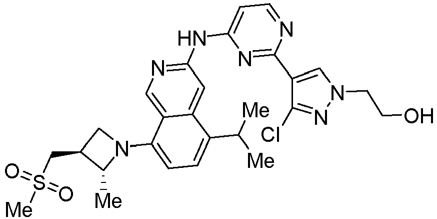

### Step 1 Synthesis of 4-bromo-3-chloro-1-((2-(trimethylsilyl)ethoxy)methyl)-1H-pyrazole

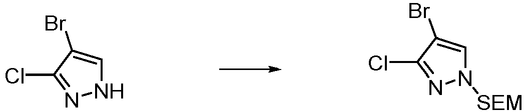

4-Bromo-3-chloro-1H-pyrazole (1.09 g, 6 mmol) and potassium carbonate (2.49 g, 18 mmol) were mixed in acetonitrile (20 mL), 2-(trimethylsilyl)ethoxymethyl chloride (1.50 g, 9 mmol) was added dropwise, and the mixture was reacted at room temperature for 3 hours. The reaction solution was diluted with dichloromethane and washed with saturated sodium chloride solution. The organic phase was dried over anhydrous sodium sulfate, filtered, and concentrated under reduced pressure to remove the solvent, and the residue was separated by silica gel column chromatography to obtain the title compound 4-bromo-3-chloro-1-((2-(trimethylsilyl)ethoxy)methyl)-1H-pyrazole (1.59 g, 85.0%).

MS m/z (ESI) :311.0 [M+H]⁺.

### Step 2 Synthesis of 3-chloro-4-(4,4,5,5-tetramethyl-1,3,2-dioxaborolan-2-yl)-1-((2-(trimethylsilyl)ethoxy)methyl)-1H-pyrazole

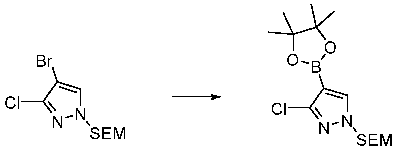

4-Bromo-3-chloro-1-((2-(trimethylsilyl)ethoxy)methyl)-1H-pyrazole (1.59 g, 5.1 mmol) and 4,4,4',4',5,5,5',5'-octamethyl-2,2'-di(1,3,2-dioxaborolane) (2.59 g, 10.2 mmol) were dissolved in dioxane (30 mL), potassium phosphate (3.18 g, 15 mmol) and [1,1'-bis(diphenylphosphino)ferrocene]palladium dichloride (412.4 mg, 0.5 mmol) were added, and the mixture was warmed to 95°C and stirred for 12 hours. The mixture was cooled to room temperature, and concentrated under reduced pressure to remove the solvent. The residue was subjected to liquid separation with dichloromethane and water. The organic phase was dried over anhydrous sodium sulfate, filtered, and concentrated under reduced pressure, and the residue was separated by silica gel column chromatography to obtain the title compound 3-chloro-4-(4,4,5,5-tetramethyl-1,3,2-dioxaborolan-2-yl)-1-((2-(trimethylsilyl)ethoxy)methyl)-1H-pyrazole (1.22 g, 66.7%).

MS m/z (ESI) :359.2 [M+H]⁺.

### Step 3 Synthesis of 2-(3-chloro-1-((2-(trimethylsilyl)ethoxy)methyl)-1H-pyrazol-4-yl)pyrimidin-4-amine

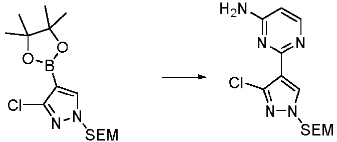

2-Chloropyrimidin-4-amine (388.6 mg, 3 mmol), 3-chloro-4-(4,4,5,5-tetramethyl-1,3,2-dioxaborolan-2-yl)-1-((2-(trimethylsilyl)ethoxy)methyl)-1H-pyrazole (1.22 g, 3.4 mmol) and potassium carbonate (1.38 g, 10 mmol) were added to a mixed solvent of dioxane (10 mL) and water (2 mL), [1,1'-bis(diphenylphosphino)ferrocene]palladium dichloride (247.4 mg, 0.3 mmol) was added and the reaction solution was warmed to 100°C and stirred for 12 hours. The reaction solution was cooled to room temperature, and concentrated under reduced pressure to remove the solvent, and the residue was separated by silica gel column chromatography to obtain the title compound 2-(3-chloro-1-((2-(trimethylsilyl)ethoxy)methyl)-1H-pyrazol-4-yl)pyrimidin-4-amine (490 mg, 50.1%).

MS m/z (ESI) :326.1 [M+H]⁺.

### Step 4 Synthesis of N-(2-(3-chloro-1-((2-(trimethylsilyl)ethoxy)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

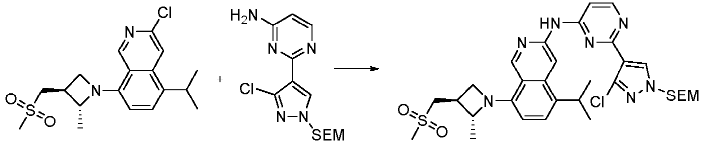

3-Chloro-5-isopropyl-8-((2R,3S)-2-methyl-3-((methanesulfonyl)methyl)azetidin-1-yl)isoquinoline (100 mg, 0.27 mmol) and 2-(3-chloro-1-((2-(trimethylsilyl)ethoxy)methyl)-1H-pyrazol-4-yl)pyrimidin-4-amine (88 mg, 0.27 mmol) were dissolved in dioxane (3 mL), cesium carbonate (274 mg, 0.84 mmol) and BrettPhos Pd G3 (24 mg, 27 µmol) were added, and the mixture was warmed to 105°C and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound N-(2-(3-chloro-1-((2-(trimethylsilyl)ethoxy)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine (81 mg, 45.7%).

MS m/z (ESI) :656.3 [M+H]⁺.

### Step 5 Synthesis of N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

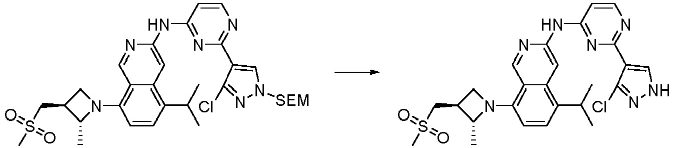

N-(2-(3-chloro-1-((2-(trimethylsilyl)ethoxy)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine (81 mg, 0.12 mmol) was dissolved in THF (2 mL), 1 M TBAF solution (2 mL, 2 mmol) was added, and the mixture was warmed to reflux and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (31 mg, 49.1%).
¹H NMR (400 MHz, DMSO-*d*₆) *δ* 13.50 (s, 1H), 10.22 (s, 1H), 9.09 (s, 1H), 8.68 (s, 1H), 8.41 (d, J = 6.0 Hz, 1H), 8.35 (s, 1H), 7.44 (d, J = 8.0 Hz, 1H), 7.25-7.18 (m, 1H), 6.58 (d, J = 8.2 Hz, 1H), 4.68 (t, *J* = 7.6 Hz, 1H), 4.25-4.15 (m, 1H), 3.65 (t, J = 7.1 Hz, 1H), 3.61-3.49 (m, 3H), 3.00 (s, 3H), 2.95-2.84 (m, 1H), 1.43 (d, J = 6.0 Hz, 3H), 1.36-1.26 (m, 6H);
MS m/z (ESI): 526.2 [M+H]⁺.

### Step 6 Synthesis of 2-(3-chloro-4-(4-((5-isopropyl-8-((2R,3S)-2-methyl-3-((methanesulfonyl)methyl)azabut-1-yl)isoquinolin-3-yl)amino)pyrimidin-2-yl)-1H-pyrazol-1-yl)ethanol

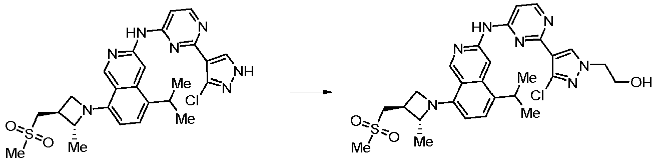

N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (57.9 mg, 0.11 mmol) was dissolved in DMF (2 mL), cesium carbonate (107.5 mg, 0.33 mmol) was added, and the mixture was warmed to 40°C and reacted for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound 2-(3-chloro-4-(4-((5-isopropyl-8-((2R,3S)-2-methyl-3-((methanesulfonyl)methyl)azabut-1-yl)isoquinolin-3-yl)amino)pyrimidin-2-yl)-1H-pyrazol-1-yl)ethanol (21.1 mg, 33.6%).
¹H NMR (400 MHz, DMSO-*d*₆) *δ* 10.19 (s, 1H), 9.10 (s, 1H), 8.53 (s, 1H), 8.41 (d, J = 6.0 Hz, 1H), 8.32 (s, 1H), 7.43 (d, *J* = 7.8 Hz, 1H), 7.32-7.25 (m, 1H), 6.58 (d, J = 8.0 Hz, 1H), 5.03 (t, J = 5.2 Hz, 1H), 4.72-4.64 (m, 1H), 4.24-4.15 (m, 3H), 3.82-3.75 (m, 2H), 3.68-3.63 (m, 1H), 3.60-3.51 (m, 3H), 3.00 (s, 3H), 2.96-2.86 (m, 1H), 1.43 (d, *J* = 6.0 Hz, 3H), 1.35-1.27 (m, 6H);
MS m/z (ESI): 570.2 [M+H]⁺.

### 2-(3-Chloro-4-(4-((5-isopropyl-8-((2R,3S)-2-methyl-3-((methanesulfonyl)methyl)azabut-1-yl)isoquinolin-3-yl)amino)pyrimidin-2-yl)-1H-pyrazol-1-yl)ethanol was also prepared with reference to reference example 1.

### Example 1

### Preparation of N-(2-(1,3-dimethyl-1H-pyrazolyl-4-yl)pyrimidin-4-yl)-5-isopropyl-8-((2R,3S)-2-methyl-3-((methanesulfonyl)methyl)azabut-1-yl)isoquinolin-3-amine

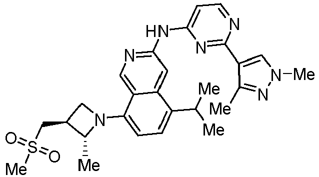

N-(2-(1,3-dimethyl-1H-pyrazolyl-4-yl)pyrimidin-4-yl)-5-isopropyl-8-((2R,3S)-2-methyl-3-((methanesulfonyl)methyl)azabut-1-yl)isoquinolin-3-amine was prepared with reference to reference example 1.
¹H NMR (400 MHz, DMSO-*d*₆) *δ* 10.08 (s, 1H), 9.09 (s, 1H), 8.56 (s, 1H), 8.36 (d, J = 5.8 Hz, 1H), 8.18 (s, 1H), 7.43 (d, J = 7.6 Hz, 1H), 7.21-7.14 (m, 1H), 6.56 (d, J = 8.0 Hz, 1H), 4.67-4.65 (m, 1H), 4.29-4.10 (m, 1H), 3.84 (s, 3H), 3.67-3.62 (m, 1H), 3.60-3.51(m, 3H), 2.99 (s, 3H), 2.91-2.83 (m, 1H), 2.49 (s, 3H),1.42 (d, J = 5.6 Hz, 3H), 1.35-1.30 (m, 6H);
MS m/z (ESI): 520.2 [M+H]⁺.

### Example 2

### Preparation of 5-isopropyl-N-(2-(3-methyl-1-(methyl-d3)-1H-pyrazol-4-yl)pyrimidin-4-yl)-8-((2R,3S)-2-methyl-3-((methylsulfonyl)methyl)azetidin-1-yl)isoquinolin-3-amine

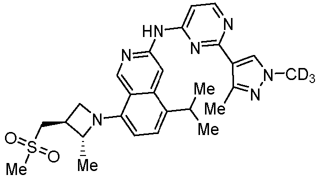

5-Isopropyl-N-(2-(3-methyl-1-(methyl-d3)-1H-pyrazol-4-yl)pyrimidin-4-yl)-8-((2R,3S)-2-methyl-3-((methylsulfonyl)methyl)azetidin-1-yl)isoquinolin-3-amine was prepared with reference to reference example 1.
¹H NMR (400 MHz, DMSO-*d*₆) *δ* 10.09 (s, 1H), 9.10 (s, 1H), 8.57 (s, 1H), 8.37 (d, J = 5.8 Hz, 1H), 8.19 (s, 1H), 7.45 (d, J = 8.0 Hz, 1H), 7.19 (d, J = 5.4 Hz, 1H), 6.58 (d, J = 8.1 Hz, 1H), 4.72-4.65 (m, 1H), 4.25-4.17 (m, 1H), 3.68-3.51 (m, 4H), 3.00 (s, 3H), 2.94-2.86 (m, 1H), 2.50 (s, 3H), 1.44 (d, J = 6.1 Hz, 3H), 1.37-1.31 (m, 6H);
MS m/z (ESI): 523.2 [M+H]⁺.

### Example 3

### Preparation of 5-isopropyl-8-((2R,3S)-2-methyl-3-((methanesulfonyl)methyl)azetidin-1-yl)-N-(2-(1-methyl-3-(methyl-d3)-1H-pyrazol-4-yl)pyrimidin-4-yl)isoquinolin-3-amine

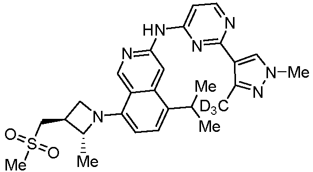

5-Isopropyl-8-((2R,3S)-2-methyl-3-((methanesulfonyl)methyl)azetidin-1-yl)-N-(2-(1-methyl-3-(methyl-d3)-1H-pyrazol-4-yl)pyrimidin-4-yl)isoquinolin-3-amine was prepared with reference to reference example 1.

MS m/z (ESI): 523.2 [M+H]⁺.

### Example 4

### Preparation of N-(2-(1,3-bis(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine

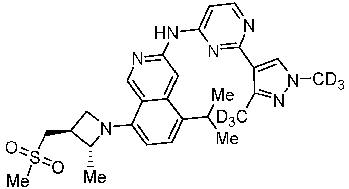

N-(2-(1,3-bis(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine was prepared with reference to reference example 1.

MS m/z (ESI): 526.2 [M+H]⁺.

### Example 5

### Preparation of N-(2-(3-chloro-1-methyl-1H-pyrazol-4-yl)pyrimidin-4-yl)-5-isopropyl-8-((2R,3S)-2-methyl-3-((methanesulfonyl)methyl)azabut-1-yl)isoquinolin-3-amine

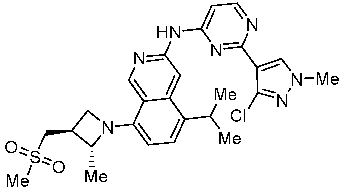

N-(2-(3-chloro-1-methyl-1H-pyrazol-4-yl)pyrimidin-4-yl)-5-isopropyl-8-((2R,3S)-2-methyl-3-((methanesulfonyl)methyl)azabut-1-yl)isoquinolin-3-amine was prepared with reference to reference example 1
or synthesized according to the following steps:

### Step 1 Synthesis of 4-bromo-3-chloro-1-methyl-1H-pyrazole

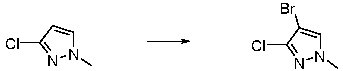

3-Chloro-1-methyl-1H-pyrazole (500 mg, 4.29 mmol) was dissolved in dichloromethane (10 mL) and cooled to 0°C, NBS (764 mg, 4.29 mmol) was added in batches, and the mixture was warmed to room temperature and stirred for 2 hours. The reaction solution was concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound 4-bromo-3-chloro-1-methyl-1H-pyrazole (790 mg, 94.2%).

### Step 2 Synthesis of 3-chloro-1-methyl-4-(4,4,5,5-tetramethyl-1,3,2-dioxaborolan-2-yl)-1H-pyrazole

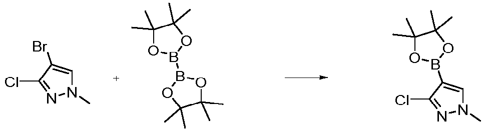

4-Bromo-3-chloro-1-methyl-1H-pyrazole (790 mg, 4.04 mmol) and 4,4,4',4',5,5,5',5'-octamethyl-2,2'-di(1,3,2-dioxaborolane) (1.23 g, 4.85 mmol) were dissolved in dioxane (15 mL), potassium phosphate (793 mg, 8.08 mmol) and [1,1'-bis(diphenylphosphino)ferrocene]palladium dichloride (300 mg, 0.41 mmol) were added, and the mixture was warmed to 95°C and stirred for 12 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound 3-chloro-1-methyl-4-(4,4,5,5-tetramethyl-1,3,2-dioxaborolan-2-yl)-1H-pyrazole (590 mg, 60.2%).

MS m/z (ESI) :243.1 [M+H]⁺.

### Step 3 Synthesis of 2-(3-chloro-1-methyl-1H-pyrazol-4-yl)pyrimidin-4-amine

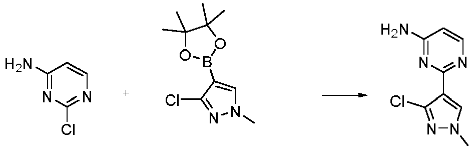

2-Chloropyrimidin-4-amine (259 mg, 2 mmol), 3-chloro-1-methyl-4-(4,4,5,5-tetramethyl-1,3,2-dioxaborolan-2-yl)-1H-pyrazole (534 mg, 2.20 mmol) and potassium carbonate (829 mg, 6 mmol) were mixed with dioxane (5 mL) and water (1 mL), [1,1'-bis(diphenylphosphino)ferrocene]palladium dichloride (146 mg, 0.20 mmol) was added, and the mixture was warmed to 95°C and stirred for 12 hours. The reaction solution was cooled to room temperature, and concentrated under reduced pressure. The residue was subjected to liquid separation with dichloromethane and water, the organic phase was dried over anhydrous sodium sulfate and filtered, and the organic solvent was concentrated under reduced pressure. The resulting mixture was separated by column chromatography to obtain the target compound 2-(3-chloro-1-methyl-1H-pyrazol-4-yl)pyrimidin-4-amine (143 mg, 34.2%).

MS m/z (ESI) :210.0 [M+H]⁺.

### Step 4 Synthesis of N-(2-(3-chloro-1-methyl-1H-pyrazol-4-yl)pyrimidin-4-yl)-5-isopropyl-8-((2R,3S)-2-methyl-3-((methanesulfonyl)methyl)azabut-1-yl)isoquinolin-3-amine

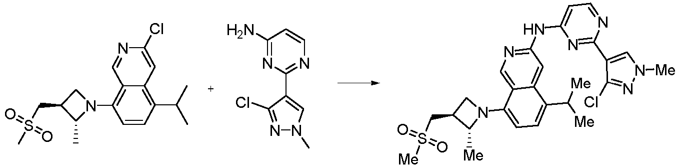

3-Chloro-5-isopropyl-8-((2R,3S)-2-methyl-3-((methanesulfonyl)methyl) azetidin-1-yl)isoquinoline (100 mg, 0.27 mmol) and 2-(3-chloro-1-methyl-1H-pyrazol-4-yl)pyrimidin-4-amine (57 mg, 0.27 mmol) were dissolved in dioxane (5 mL), cesium carbonate (274 mg, 0.84 mmol) and BrettPhos Pd G3 (24 mg, 27 µmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound N-(2-(3-chloro-1-methyl-1H-pyrazol-4-yl)pyrimidin-4-yl)-5-isopropyl-8-((2R,3S)-2-methyl-3-((methanesulfonyl)methyl)azabut-1-yl)isoquinolin-3-amine (44 mg, 30.5%).
¹H NMR (400 MHz, DMSO-*d*₆) *δ* 10.18 (s, 1H), 9.10 (s, 1H), 8.59 (s, 1H), 8.40 (d, J = 5.9 Hz, 1H), 8.33 (s, 1H), 7.43 (d, J = 8.0 Hz, 1H), 7.25 (d, J = 5.8 Hz, 1H), 6.58 (d, J = 8.1 Hz, 1H), 4.68 (t, J = 7.5 Hz, 1H), 4.21 (t, *J* = 6.2 Hz, 1H), 3.90 (s, 3H), 3.68-3.51 (m, 4H), 3.00 (s, 3H), 2.94-2.85 (m, 1H), 1.43 (d, J = 6.0 Hz, 3H), 1.34-1.26 (m, 6H);
MS m/z (ESI): 540.2 [M+H]⁺.

### Example 6

### Preparation of N-(2-(3-chloro-1-(methyl-d3)-1H-pyrazol-4-yl)pyrimidin-4-yl)-5-isopropyl-8-((2R,3S)-2-methyl-3-((methylsulfonyl)methyl)azetidin-1-yl)isoquinolin-3-amine

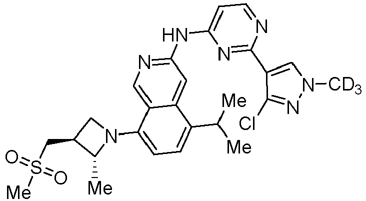

N-(2-(3-chloro-1-(methyl-d3)-1H-pyrazol-4-yl)pyrimidin-4-yl)-5-isopropyl-8-((2R,3S)-2-methyl-3-((methylsulfonyl)methyl)azetidin-1-yl)isoquinolin-3-amine was prepared with reference to reference example 1.
¹H NMR (400 MHz, DMSO-*d*₆) *δ* 10.19 (s, 1H), 9.10 (s, 1H), 8.59 (s, 1H), 8.40 (d, J = 5.9 Hz, 1H), 8.33 (s, 1H), 7.43 (d, *J* = 8.0 Hz, 1H), 7.25 (d, J = 5.6 Hz, 1H), 6.58 (d, J = 8.1 Hz, 1H), 4.71-4.64 (m, 1H), 4.24-4.16 (m, 1H), 3.67-3.48 (m, 4H), 3.00 (s, 3H), 2.93-2.85 (m, 1H), 1.43 (d, J = 6.1 Hz, 3H), 1.33-1.27 (m, 6H);
MS m/z (ESI): 543.2 [M+H]⁺.

### Example 7

### Preparation of N-(2-(1-cyclopropyl-3-methyl-1H-pyrazol-4-yl)pyrimidin-4-yl)-5-isopropyl-8-((2R,3S)-2-methyl-3-((methylsulfonyl)methyl)azetidin-1-yl)isoquinolin-3-amine

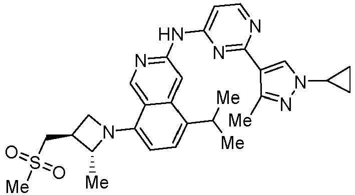

N-(2-(1-cyclopropyl-3-methyl-1H-pyrazol-4-yl)pyrimidin-4-yl)-5-isopropyl-8-((2R,3S)-2-methyl-3-((methylsulfonyl)methyl)azetidin-1-yl)isoquinolin-3-amine was prepared with reference to reference example 1.

MS m/z (ESI): 546.2 [M+H]⁺.

### Example 8

### Preparation of N-(2-(1-cyclopropyl-3-(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine

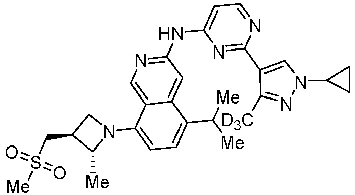

N-(2-(1-cyclopropyl-3-(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine was prepared with reference to reference example 1.

MS m/z (ESI): 549.2 [M+H]⁺.

### Example 9

### Preparation of N-(2-(1-cyclopropyl-3-fluoro-1H-pyrazol-4-yl)pyrimidin-4-yl)-5-isopropyl-8-((2R,3S)-2-methyl-3-((methylsulfonyl)methyl)azetidin-1-yl)isoquinolin-3-amine

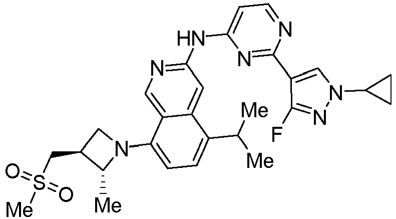

N-(2-(1-cyclopropyl-3-fluoro-1H-pyrazol-4-yl)pyrimidin-4-yl)-5-isopropyl-8-((2R,3S)-2-methyl-3-((methylsulfonyl)methyl)azetidin-1-yl)isoquinolin-3-amine was prepared with reference to reference example 1.

MS m/z (ESI): 550.2 [M+H]⁺.

### Example 10

### Preparation of 2-(1-cyclopropyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine

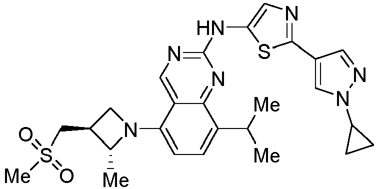

2-(1-Cyclopropyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine was prepared with reference to reference example 1.

MS m/z (ESI): 538.2 [M+H]⁺.

### Example 11

### Preparation of N-(8-isopropyl-5-((2R,3S)-2-methyl-3-((methanesulfonyl)methyl)azetidin-1-yl)quinazolin-2-yl)-2-(1-methyl-1H-pyrazol-4-yl)thiazol-5-amine

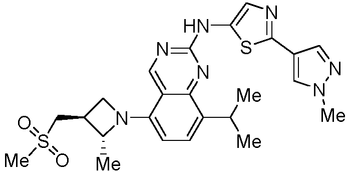

N-(8-Isopropyl-5-((2R,3S)-2-methyl-3-((methanesulfonyl)methyl)azetidin-1-yl)quinazolin-2-yl)-2-(1-methyl-1H-pyrazol-4-yl)thiazol-5-amine was prepared with reference to reference example 1.

MS m/z (ESI): 512.2 [M+H]⁺.

### Example 12

### Preparation of 2-(1,3-dimethyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine

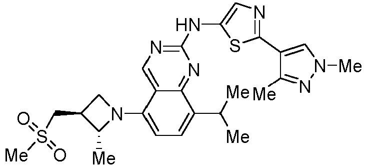

2-(1,3-Dimethyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine was prepared with reference to reference example 1.

MS m/z (ESI): 526.2 [M+H]⁺.

### Example 13

### Preparation of 2-(3-chloro-1-methyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine

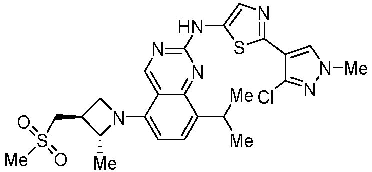

2-(3-Chloro-1-methyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine was prepared with reference to reference example 1.

MS m/z (ESI): 546.2 [M+H]⁺.

### Example 14

### Preparation of 2-(3-fluoro-1-methyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine

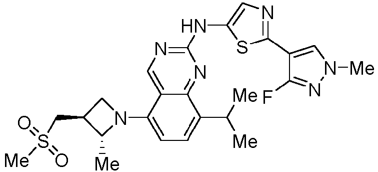

2-(3-Fluoro-1-methyl-1H-pyrazol-4-yl)-N-(8-isopropyl-5-((2R,3S)-2-methyl-3-((methanesulfonyl)methyl)azetidin-1-yl)quinazolin-2-yl)thiazol-5-amine was prepared with reference to reference example 1.

MS m/z (ESI): 530.2 [M+H]⁺.

### Example 15

### Preparation of N-(2-(1-cyclopropyl-1H-pyrazolyl-4-yl)-3-fluoropyridin-4-yl)-5-isopropyl-8-((2R,3S)-2-methyl-3-((methylsulfonyl)methyl)azabut-1-yl)isoquinolin-3-amine

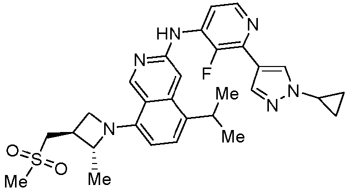

N-(2-(1-cyclopropyl-1H-pyrazolyl-4-yl)-3-fluoropyridin-4-yl)-5-isopropyl-8-((2R,3S)-2-methyl-3-((methylsulfonyl)methyl)azabut-1-yl)isoquinolin-3-amine was prepared with reference to reference example 1.

MS m/z (ESI): 549.2 [M+H]⁺.

### Example 16

### Preparation of N-(3-fluoro-2-(1-methyl-1H-pyrazol-4-yl)pyridin-4-yl)-5-isopropyl-8-((2R,3S)-2-methyl-3-((methylsulfonyl)methyl)azetidin-1-yl)isoquinolin-3-amine

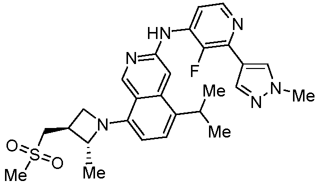

N-(3-fluoro-2-(1-methyl-1H-pyrazol-4-yl)pyridin-4-yl)-5-isopropyl-8-((2R,3S)-2-methyl-3-((methylsulfonyl)methyl)azetidin-1-yl)isoquinolin-3-amine was prepared with reference to reference example 1.

MS m/z (ESI): 523.2 [M+H]⁺.

### Example 17

### Preparation of N-(2-(1,3-dimethyl-1H-pyrazolyl-4-yl)-3-fluoropyridin-4-yl)-5-isopropyl-8-((2R,3S)-2-methyl-3-((methanesulfonyl)methyl)azabut-1-yl)isoquinolin-3-amine

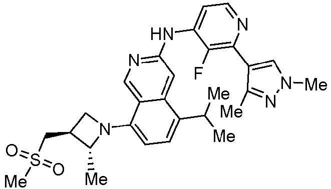

N-(2-(1,3-dimethyl-1H-pyrazolyl-4-yl)-3-fluoropyridin-4-yl)-5-isopropyl-8-((2R,3S)-2-methyl-3-((methanesulfonyl)methyl)azabut-1-yl)isoquinolin-3-amine was prepared with reference to reference example 1.

MS m/z (ESI): 537.2 [M+H]⁺.

### Example 18

### Preparation of N-(2-(3-chloro-1-methyl-1H-pyrazol-4-yl)-3-fluoropyridin-4-yl)-5-isopropyl-8-((2R,3S)-2-methyl-3-((methanesulfonyl)methyl)azabut-1-yl)isoquinolin-3-amine

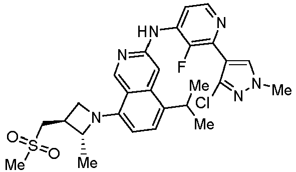

N-(2-(3-chloro-1-methyl-1H-pyrazol-4-yl)-3-fluoropyridin-4-yl)-5-isopropyl-8-((2R,3S)-2-methyl-3-((methanesulfonyl)methyl)azabut-1-yl)isoquinolin-3-amine was prepared with reference to reference example 1 or example 5.

MS m/z (ESI): 557.2 [M+H]⁺.

### Example 19

### Preparation of N-(3-fluoro-2-(3-fluoro-1-methyl-1H-pyrazol-4-yl)pyridin-4-yl)-5-isopropyl-8-((2R,3S)-2-methyl-3-((methanesulfonyl)methyl)azabut-1-yl)isoquinolin-3-amine

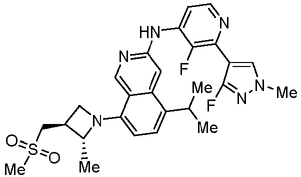

N-(3-fluoro-2-(3-fluoro-1-methyl-1H-pyrazol-4-yl)pyridin-4-yl)-5-isopropyl-8-((2R,3S)-2-methyl-3-((methanesulfonyl)methyl)azabut-1-yl)isoquinolin-3-amine was prepared with reference to reference example 1.

MS m/z (ESI): 541.2 [M+H]⁺.

### Example 20

### Preparation of 1-isopropyl-N-(2-(1-methyl-1H-pyrazol-4-yl)pyrimidin-4-yl)-3-((2R,3S)-2-methyl-3-((methylsulfonyl)methyl)azetidin-1-yl)-1H-pyrazolo[4,3-c]pyridin-6-amine

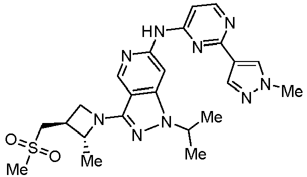

1-Isopropyl-N-(2-(1-methyl-1H-pyrazol-4-yl)pyrimidin-4-yl)-3-((2R,3S)-2-methyl-3-((methylsulfonyl)methyl)azetidin-1-yl)-1H-pyrazolo[4,3-c]pyridin-6-amine was prepared with reference to reference example 1.

MS m/z (ESI): 496.2 [M+H]⁺.

### Example 21

### Preparation of N-(2-(1-cyclopropyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine

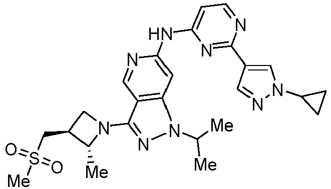

N-(2-(1-cyclopropyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine was prepared with reference to reference example 1.

MS m/z (ESI): 522.2 [M+H]⁺.

### Example 22

### Preparation of N-(2-(1,3-dimethyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine

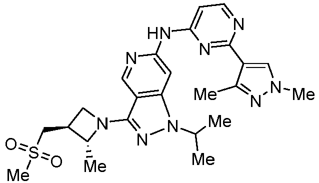

N-(2-(1,3-dimethyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine was prepared with reference to reference example 1.

¹H NMR (400 MHz, DMSO-*d*₆) *δ* 10.00 (s, 1H), 8.58 (s, 1H), 8.33 (d, *J* = 6.0 Hz, 1H), 8.18 (s, 1H), 8.04 (s, 1H), 7.04-6.99 (m, 1H), 4.68-4.60 (m, 1H), 4.37 (d, *J* = 7.4 Hz, 1H), 4.18 (d, *J* = 6.6 Hz, 1H), 3.82 (s, 3H), 3.80-3.75 (m, 1H), 3.55-3.50 (m, 2H), 2.98 (s, 3H), 2.93-2.86 (m, 1H), 2.54 (s, 3H), 1.52 (d, *J* = 6.1 Hz, 3H), 1.43 (d, *J* = 5.6 Hz, 6H);

MS m/z (ESI): 510.2 [M+H]⁺.

### Example 23

### Preparation of N-(2-(3-chloro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine

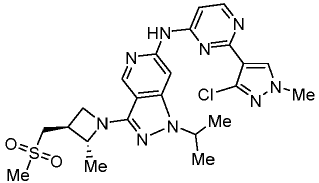

N-(2-(3-chloro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrazolo[4,3-c]pyridin-6-amine was prepared with reference to reference example 1 or example 5.

MS m/z (ESI): 530.2 [M+H]⁺.

### Example 24

### Preparation of N-(2-(3-fluoro-1-methyl-1H-pyrazolyl-4-yl)pyrimidin-4-yl)-1-isopropyl-3-((2R,3S)-2-methyl-3-((methanesulfonyl)methyl)azabut-1-yl)-1H-pyrazolo[4,3-c]pyridin-6-amine

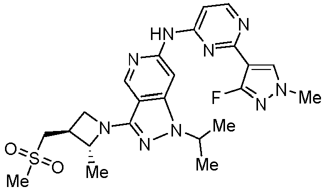

N-(2-(3-fluoro-1-methyl-1H-pyrazolyl-4-yl)pyrimidin-4-yl)-1-isopropyl-3-((2R,3S)-2-methyl-3-((methanesulfonyl)methyl)azabut-1-yl)-1H-pyrazolo[4,3-c]pyridin-6-amine was prepared with reference to reference example 1.

MS m/z (ESI): 514.2 [M+H]⁺.

### Example 25

### Preparation of 1-isopropyl-N-(2-(1-methyl-1H-pyrazol-4-yl)pyrimidin-4-yl)-3-((2R,3S)-2-methyl-3-((methylsulfonyl)methyl)azetidin-1-yl)-1H-pyrrolo[3,2-c]pyridin-6-amine

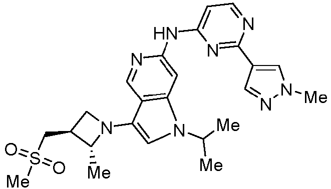

1-Isopropyl-N-(2-(1-methyl-1H-pyrazol-4-yl)pyrimidin-4-yl)-3-((2R,3S)-2-methyl-3-((methylsulfonyl)methyl)azetidin-1-yl)-1H-pyrrolo[3,2-c]pyridin-6-amine was prepared with reference to reference example 1.

MS m/z (ESI): 495.2 [M+H]⁺.

### Example 26

### Preparation of N-(2-(1-cyclopropyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine

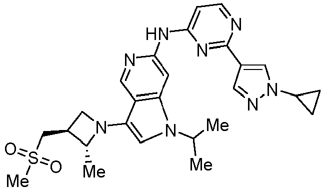

N-(2-(1-cyclopropyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine was prepared with reference to reference example 1.

MS m/z (ESI): 521.2 [M+H]⁺.

### Example 27

### Preparation of N-(2-(1,3-dimethyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine

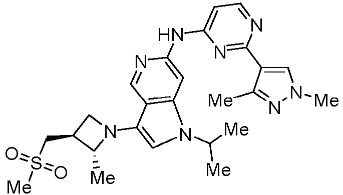

N-(2-(1,3-dimethyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine was prepared with reference to reference example 1.

MS m/z (ESI): 509.2 [M+H]⁺.

### Example 28

### Preparation of N-(2-(3-chloro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine

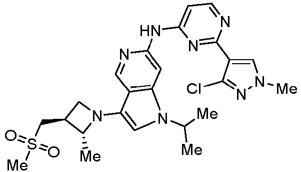

N-(2-(3-chloro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine was prepared with reference to reference example 1 or example 5.

MS m/z (ESI): 529.2 [M+H]⁺.

### Example 29

### Preparation of N-(2-(3-fluoro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine

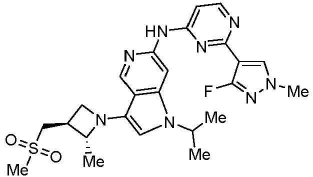

N-(2-(3-fluoro-1-methyl-1H-pyrazol-4-yl)pyrimidin-4-yl)-1-isopropyl-3-((2R,3S)-2-methyl-3-((methanesulfonyl)methyl)azetidin-1-yl)-1H-pyrrolo[3,2-c]pyridin-6-amine was prepared with reference to reference example 1.

MS m/z (ESI): 513.2 [M+H]⁺.

### Example 30

### Preparation of N-(2-(1-cyclopropyl-1H-pyrazol-4-yl)pyrimidin-4-yl)-5-isopropyl-8-((2R,3R)-2-methyl-3-(nitromethyl)azetin-1-yl)isoquinolin-3-amine

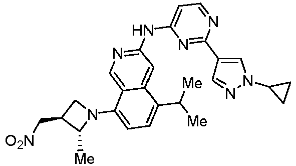

N-(2-(1-cyclopropyl-1H-pyrazol-4-yl)pyrimidin-4-yl)-5-isopropyl-8-((2R,3R)-2-methyl-3-(nitromethyl)azetin-1-yl)isoquinolin-3-amine was prepared with reference to reference example 1.

MS m/z (ESI): 499.2 [M+H]⁺.

### Example 31

### Preparation of 5-isopropyl-N-(2-(1-methyl-1H-pyrazol-4-yl)pyrimidin-4-yl)-8-((2R,3R)-2-methyl-3-(nitromethyl)azetidin-1-yl)isoquinolin-3-amine

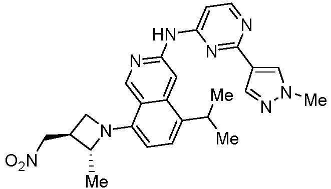

5-Isopropyl-N-(2-(1-methyl-1H-pyrazol-4-yl)pyrimidin-4-yl)-8-((2R,3R)-2-methyl-3-(nitromethyl)azetidin-1-yl)isoquinolin-3-amine was prepared with reference to reference example 1.

MS m/z (ESI): 473.2 [M+H]⁺.

### Example 32

### Preparation of N-(2-(1,3-dimethyl-1H-pyrazol-4-yl)pyrimidin-4-yl)-5-isopropyl-8-((2R,3R)-2-methyl-3-(nitromethyl)azetidin-1-yl)isoquinolin-3-amine

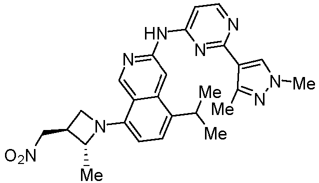

N-(2-(1,3-dimethyl-1H-pyrazol-4-yl)pyrimidin-4-yl)-5-isopropyl-8-((2R,3R)-2-methyl-3-(nitromethyl)azetidin-1-yl)isoquinolin-3-amine was prepared with reference to reference example 1.

MS m/z (ESI): 487.2 [M+H]⁺.

### Example 33

### Preparation of N-(2-(3-chloro-1-methyl-1H-pyrazol-4-yl)pyrimidin-4-yl)-5-isopropyl-8-((2R,3R)-2-methyl-3-(nitromethyl)azetidin-1-yl)isoquinolin-3-amine

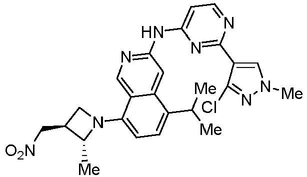

N-(2-(3-Chloro-1-methyl-1H-pyrazol-4-yl)pyrimidin-4-yl)-5-isopropyl-8-((2R,3R)-2-methyl-3-(nitromethyl)azetidin-1-yl)isoquinolin-3-amine was prepared with reference to reference example 1.

MS m/z (ESI): 507.2 [M+H]⁺.

### Example 34

### Preparation of N-(2-(3-fluoro-1-methyl-1H-pyrazol-4-yl)pyrimidin-4-yl)-5-isopropyl-8-((2R,3R)-2-methyl-3-(nitromethyl)azetidin-1-yl)isoquinolin-3-amine

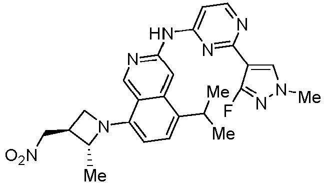

N-(2-(3-Fluoro-1-methyl-1H-pyrazol-4-yl)pyrimidin-4-yl)-5-isopropyl-8-((2R,3R)-2-methyl-3-(nitromethyl)azetidin-1-yl)isoquinolin-3-amine was prepared with reference to reference example 1.

MS m/z (ESI): 491.2 [M+H]⁺.

### Example 35

### Preparation of N-(2-(1-cyclopropyl-1H-pyrazol-4-yl)pyrimidin-4-yl)-5-isopropyl-8-(3-(nitromethyl)azetidin-1-yl)isoquinolin-3-amine

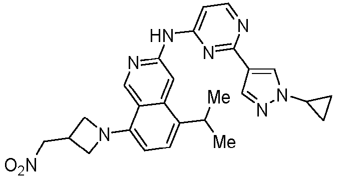

N-(2-(1-Cyclopropyl-1H-pyrazol-4-yl)pyrimidin-4-yl)-5-isopropyl-8-(3-(nitromethyl)azetidin-1-yl)isoquinolin-3-amine was prepared with reference to reference example 1.

MS m/z (ESI): 485.2 [M+H]⁺.

### Example 36

### Preparation of 5-isopropyl-N-(2-(1-methyl-1H-pyrazol-4-yl)pyrimidin-4-yl)-8-(3-(nitromethyl)azacyclodextrin-1-yl)isoquinolin-3-amine

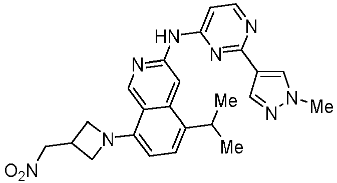

5-Isopropyl-N-(2-(1-methyl-1H-pyrazol-4-yl)pyrimidin-4-yl)-8-(3-(nitromethyl)azacyclodextrin-1-yl)isoquinolin-3-amine was prepared with reference to reference example 1.

MS m/z (ESI): 459.2 [M+H]⁺.

### Example 37

### Preparation of N-(2-(1,3-dimethyl-1H-pyrazol-4-yl)pyrimidin-4-yl)-5-isopropyl-8-(3-(nitromethyl)azetidin-1-yl)isoquinolin-3-amine

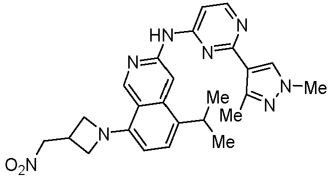

N-(2-(1,3-dimethyl-1H-pyrazol-4-yl)pyrimidin-4-yl)-5-isopropyl-8-(3-(nitromethyl)azetidin-1-yl)isoquinolin-3-amine was prepared with reference to reference example 1.

MS m/z (ESI): 473.2 [M+H]⁺.

### Example 38

### Preparation of N-(2-(3-fluoro-1-methyl-1H-pyrazol-4-yl)pyrimidin-4-yl)-5-isopropyl-8-(3-(nitromethyl)azetidin-1-yl)isoquinolin-3-amine

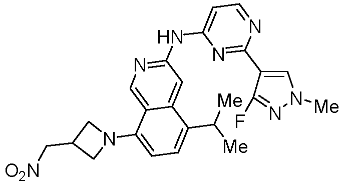

N-(2-(3-fluoro-1-methyl-1H-pyrazol-4-yl)pyrimidin-4-yl)-5-isopropyl-8-(3-(nitromethyl)azetidin-1-yl)isoquinolin-3-amine was prepared with reference to reference example 1.

MS m/z (ESI): 477.2 [M+H]⁺.

### Example 39

### Preparation of ((2R,3S)-1-(3-((2-(1-cyclopropyl-1H-pyrazol-4-yl)pyrimidin-4-yl)amino)-5-isopropylisoquinolin-8-yl)-2-methylazetidin-3-yl)methanesulfonyl fluoride

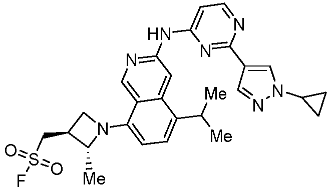

((2R,3S)-1-(3-((2-(1-Cyclopropyl-1H-pyrazol-4-yl)pyrimidin-4-yl)amino)-5-isopropylisoquinolin-8-yl)-2-methylazetidin-3-yl)methanesulfonyl fluoride was prepared with reference to reference example 1.

MS m/z (ESI): 536.2 [M+H]⁺.

### Example 40

### Preparation of ((2R,3S)-1-(5-isopropyl-3-((2-(1-methyl-1H-pyrazol-4-yl)pyrimidin-4-yl)amino)isoquinolin-8-yl)-2-methyl nitride-3-yl)methanesulfonyl fluoride

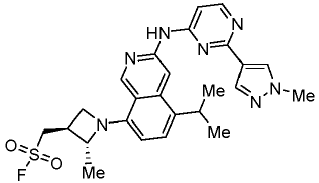

((2R,3S)-1-(5-Isopropyl-3-((2-(1-methyl-1H-pyrazol-4-yl)pyrimidin-4-yl)amino)isoquinolin-8-yl)-2-methyl nitride-3-yl)methanesulfonyl fluoride was prepared with reference to reference example 1.

MS m/z (ESI): 510.2 [M+H]⁺.

### Example 41

### Preparation of ((2R,3S)-1-(3-((2-(1,3-dimethyl-1H-pyrazol-4-yl)pyrimidin-4-yl)amino)-5-isopropylquinolin-8-yl)-2-methylazetidin-3-yl)methanesulfonyl fluoride

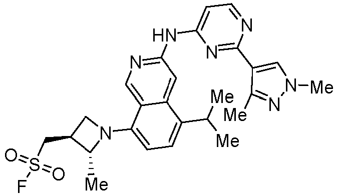

((2R,3S)-1-(3-((2-(1,3-dimethyl-1H-pyrazol-4-yl)pyrimidin-4-yl)amino)-5-isopropylquinolin-8-yl)-2-methylazetidin-3-yl)methanesulfonyl fluoride was prepared with reference to reference example 1.

MS m/z (ESI): 524.2 [M+H]⁺.

### Example 42

### Preparation of ((2R,3S)-1-(3-((2-(3-chloro-1-methyl-1H-pyrazol-4-yl)pyrimidin-4-yl)amino)-5-isopropylquinolin-8-yl)-2-methylazetidin-3-yl)methanesulfonyl fluoride

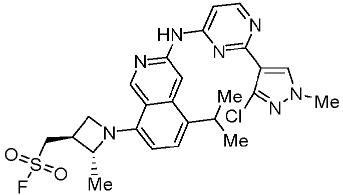

((2R,3S)-1-(3-((2-(3-chloro-1-methyl-1H-pyrazol-4-yl)pyrimidin-4-yl)amino)-5-isopropylquinolin-8-yl)-2-methylazetidin-3-yl)methanesulfonyl fluoride was prepared with reference to reference example 1.

MS m/z (ESI): 544.2 [M+H]⁺.

### Example 43

### Preparation of ((2R,3S)-1-(3-((2-(3-fluoro-1-methyl-1H-pyrazol-4-yl)pyrimidin-4-yl)amino)-5-isopropylquinolin-8-yl)-2-methylazetidin-3-yl)methanesulfonyl fluoride

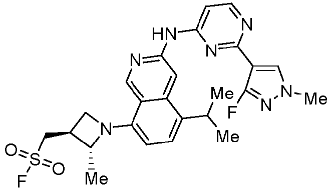

((2R,3S)-1-(3-((2-(3-fluoro-1-methyl-1H-pyrazol-4-yl)pyrimidin-4-yl)amino)-5-isopropylquinolin-8-yl)-2-methylazetidin-3-yl)methanesulfonyl fluoride was prepared with reference to reference example 1.

MS m/z (ESI): 528.2 [M+H]⁺.

### Example 44

### Preparation of N-(1-(3-((2-(3-chloro-1-methyl-1H-pyrazol-4-yl)pyrimidin-4-yl)amino)-5-isopropylisoquinolin-8-yl)azetidin-3-yl)methanesulfonamide

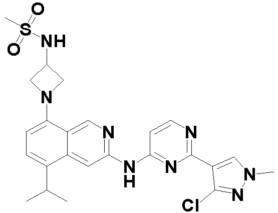

### Step 1 Synthesis of N-(1-(3-chloro-5-isopropylisoquinolin-8-yl)azetidin-3-yl)methanesulfonamide

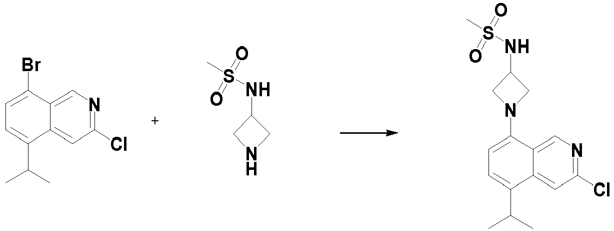

8-Bromo-3-chloro-5-isopropylisoquinoline (300 mg, 1.05 mmol) and N-(azetidin-3-yl)methanesulfonamide (158 mg, 1.05 mmol) were dissolved in dioxane (10 mL), cesium carbonate (1.15 g, 3.54 mmol) and Xantphos Pd G4 (164 mg, 0.17 mmol) were added, and the mixture was warmed to 125°C by microwave and reacted for 2 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound N-(1-(3-chloro-5-isopropylisoquinolin-8-yl)azetidin-3-yl)methanesulfonamide (170 mg, 45.6%).

MS m/z (ESI) :354.1 [M+H]⁺.

### Step 2 Synthesis of N-(1-(3-((2-(3-chloro-1-methyl-1H-pyrazol-4-yl)pyrimidin-4-yl)amino)-5-isopropylisoquinolin-8-yl)azetidin-3-yl)methanesulfonamide

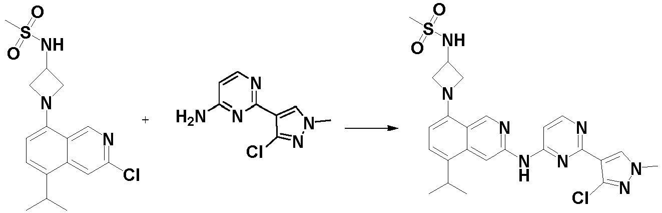

N-(1-(3-chloro-5-isopropylisoquinolin-8-yl)azetidin-3-yl)methanesulfonamide (50 mg, 0.14 mmol) and 2-(3-chloro-1-methyl-1H-pyrazol-4-yl)pyrimidin-4-amine (30 mg, 0.14 mmol) were dissolved in dioxane (5 mL), cesium carbonate (81 mg, 0.25 mmol) and Xantphos Pd G4 (26 mg, 27 µmol) were added, and the mixture was reacted under microwave at 120°C for 2 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound N-(1-(3-((2-(3-chloro-1-methyl-1H-pyrazol-4-yl)pyrimidin-4-yl)amino)-5-isopropylisoquinolin-8-yl)azetidin-3-yl)methanesulfonamide (11 mg, 14.9%).

¹H NMR (400 MHz, DMSO-*d*₆) *δ* 10.16 (s, 1H), 9.08 (s, 1H), 8.57 (s, 1H), 8.42-8.39 (m, 1H), 8.33 (s, 1H), 7.85-7.82 (m, 1H), 7.43 (d, *J* = 7.6 Hz, 1H), 7.29-7.25 (m, 1H), 6.45 (d, *J* = 8.2 Hz, 1H), 4.57-4.51 (m, 2H), 4.40-4.33 (m, 1H), 3.95-3.91 (m, 2H), 3.90 (s, 3H), 3.59-3.51 (m, 1H), 2.98 (s, 3H), 1.31-1.28 (m, 6H);

MS m/z (ESI) :527.1 [M+H]⁺.

### Example 45

### Preparation of N-((2R,3S)-1-(3-((2-(1,3-dimethyl-1H-pyrazol-4-yl)pyrimidin-4-yl)amino)-5-isopropylquinolin-8-yl)-2-methyl azetidin-3-yl)methanesulfonimide

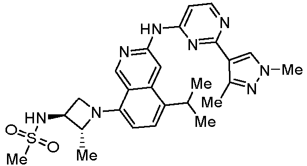

N-((2R,3S)-1-(3-((2-(1,3-Dimethyl-1H-pyrazol-4-yl)pyrimidin-4-yl)amino)-5-isopropyl quinolin-8-yl)-2-methyl azetidin-3-yl)methanesulfonimide was prepared with reference to reference example 1 or example 44.

MS m/z (ESI): 521.2 [M+H]⁺.

### Example 46

### Preparation of N-((2R,3S)-1-(3-((2-(3-chloro-1-methyl-1H-pyrazol-4-yl)pyrimidin-4-yl)amino)-5-isopropylquinolin-8-yl)-2-methylazetidin-3-yl)methanesulfonimide

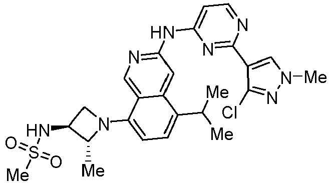

N-((2R,3S)-1-(3-((2-(3-Chloro-1-methyl-1H-pyrazol-4-yl)pyrimidin-4-yl)amino)-5-isopropylquinolin-8-yl)-2-methylazetidin-3-yl)methanesulfonimide was prepared with reference to reference example 1 or example 44.

MS m/z (ESI): 541.2 [M+H]⁺.

### Example 47

### Preparation of N-((2R,3S)-1-(3-((2-(3-fluoro-1-methyl-1H-pyrazol-4-yl)pyrimidin-4-yl)amino)-5-isopropylquinolin-8-yl)-2-methylazetidin-3-yl)methanesulfonimide

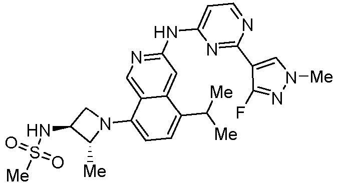

N-((2R,3S)-1-(3-((2-(3-Fluoro-1-methyl-1H-pyrazol-4-yl)pyrimidin-4-yl)amino)-5-isopropylquinolin-8-yl)-2-methylazetidin-3-yl)methanesulfonimide was prepared with reference to reference example 1 or example 44.

MS m/z (ESI): 525.2 [M+H]⁺.

### Example 48

### Preparation of 5-isopropyl-8-((2R,3S)-2-methyl-3-((methanesulfonyl)methyl)azetidin-1-yl)-N-(2-(1-methyl-5-nitro-1H-pyrrol-3-yl)pyrimidin-4-yl)isoquinolin-3-amine

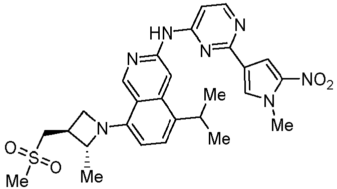

5-Isopropyl-8-((2R,3S)-2-methyl-3-((methanesulfonyl)methyl)azetidin-1-yl)-N-(2-(1-methyl-5-nitro-1H-pyrrol-3-yl)pyrimidin-4-yl)isoquinolin-3-amine was prepared with reference to reference example 1.

MS m/z (ESI): 550.2 [M+H]⁺.

### Example 49

### Preparation of N-(2-(1,2-dimethyl-5-nitro-1H-pyrrol-3-yl)pyrimidin-4-yl)-5-isopropyl-8-((2R,3S)-2-methyl-3-((methanesulfonyl)methyl)azetidin-1-yl)isoquinolin-3-amine

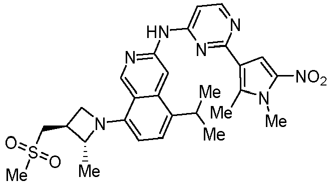

N-(2-(1,2-Dimethyl-5-nitro-1H-pyrrol-3-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1.

MS m/z (ESI): 564.2 [M+H]⁺.

### Example 50

### Preparation of N-(2-(2-chloro-1-methyl-5-nitro -1H-pyrrol-3-yl)pyrimidin-4-yl)-5-isopropyl-8-((2R,3S)-2-methyl-3-((methanesulfonyl)methyl)azetidin-1-yl)isoquinolin-3-amine

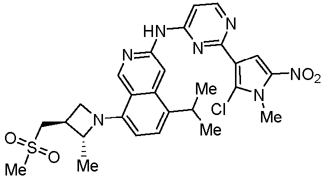

N-(2-(2-Chloro-1-methyl-5-nitro-1H-pyrrol-3-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1.

MS m/z (ESI): 584.2 [M+H]⁺.

### Example 51

### Preparation of 4-(4-((5-isopropyl-8-((2R,3S)-2-methyl-3-((methanesulfonyl)methyl)azetidin-1-yl)isoquinolin-3-yl)amino)pyrimidin-2-yl)-1-methyl-1,5-dihydro-2H-pyrrol-2-one

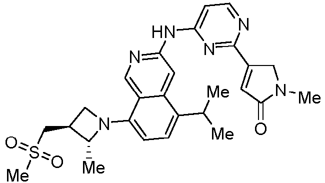

4-(4-((5-Isopropyl-8-((2R,3S)-2-methyl-3-((methanesulfonyl)methyl)azetidin-1-yl)isoquinolin-3-yl)amino)pyrimidin-2-yl)-1-methyl-1,5-dihydro-2H-pyrrol-2-one was prepared with reference to reference example 1.

MS m/z (ESI): 521.2 [M+H]⁺.

### Example 52

### Preparation of 3-(4-((5-isopropyl-8-((2R,3S)-2-methyl-3-((methanesulfonyl)methyl)azetidin-1-yl)isoquinolin-3-yl)amino)pyrimidin-2-yl)-1-methyl-1,5-dihydro -2H-pyrrol-2-one

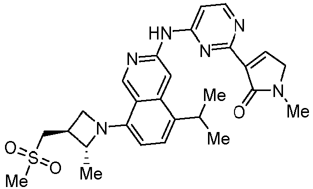

3-(4-((5-Isopropyl-8-((2R,3S)-2-methyl-3-((methanesulfonyl)methyl)azetidin-1-yl)isoquinolin-3-yl)amino)pyrimidin-2-yl)-1-methyl-1,5-dihydro -2H-pyrrol-2-one was prepared with reference to reference example 1.

MS m/z (ESI): 521.2 [M+H]⁺.

### Example 53

### Preparation of 5-isopropyl-N-(2-(1-methyl-1H-1,2,3-triazol-4-yl)pyrimidin-4-yl)-8-((2R,3S)-2-methyl-3-((methylsulfonyl)methyl)azetidin-1-yl)isoquinolin-3-amine

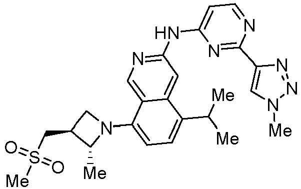

5-Isopropyl-N-(2-(1-methyl-1H-1,2,3-triazol-4-yl)pyrimidin-4-yl)-8-((2R,3S)-2-methyl-3-((methylsulfonyl)methyl)azetidin-1-yl)isoquinolin-3-amine was prepared with reference to reference example 1.

MS m/z (ESI): 507.2 [M+H]⁺.

### Example 54

### Preparation of 5-isopropyl-N-(2-(2-methyl-2H-1,2,3-triazol-4-yl)pyrimidin-4-yl)-8-((2R,3S)-2-methyl-3-((methylsulfonyl)methyl)azetidin-1-yl)isoquinolin-3-amine

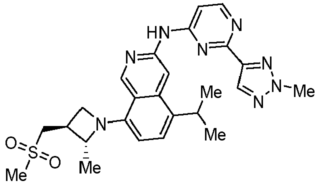

5-Isopropyl-N-(2-(2-methyl-2H-1,2,3-triazol-4-yl)pyrimidin-4-yl)-8-((2R,3S)-2-methyl-3-((methylsulfonyl)methyl)azetidin-1-yl)isoquinolin-3-amine was prepared with reference to reference example 1.

MS m/z (ESI): 507.2 [M+H]⁺.

### Example 55

### Preparation of N-(6-(3-chloro-1-methyl-1H-pyrazol-4-yl)-5-fluoropyridin-2-yl)-5-isopropyl-8-((2R,3S)-2-methyl-3-((methanesulfonyl)methyl)azabut-1-yl)isoquinolin-3-amine

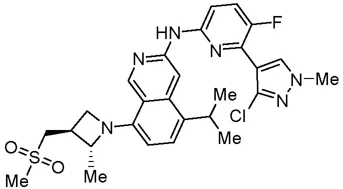

N-(6-(3-chloro-1-methyl-1H-pyrazol-4-yl)-5-fluoropyridin-2-yl)-5-isopropyl-8-((2R,3S)-2-methyl-3-((methanesulfonyl)methyl)azabut-1-yl)isoquinolin-3-amine was prepared with reference to reference example 1 or example 5.

MS m/z (ESI): 557.2 [M+H]⁺.

### Example 56

### Preparation of N-(6-(3-chloro-1-methyl-1H-pyrazol-4-yl)-5-methoxypyridin-2-yl)-5-isopropyl-8-((2R,3S)-2-methyl-3-((methanesulfonyl)methyl)azabut-1-yl)isoquinolin-3-amine

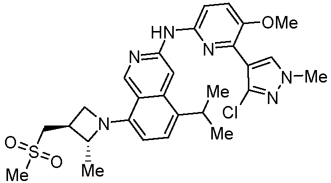

N-(6-(3-chloro-1-methyl-1H-pyrazol-4-yl)-5-methoxy pyridin-2-yl)-5-isopropyl-8-((2R,3S)-2-methyl-3-((methanesulfonyl)methyl)azabut-1-yl)isoquinolin-3-amine was prepared with reference to reference example 1 or example 5.

MS m/z (ESI): 569.2 [M+H]⁺.

### Example 57

### Preparation of 2-(3-chloro-1-methyl-1H-pyrazol-4-yl)-6-((5-isopropyl-8-((2R,3S)-2-methyl-3-((methanesulfonyl)methyl)azabut-1-yl)isoquinolin-3-yl)amino)nicotinonitrile

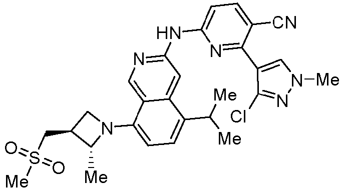

2-(3-Chloro-1-methyl-1H-pyrazol-4-yl)-6-((5-isopropyl-8-((2R,3S)-2-methyl-3-((methanesulfonyl)methyl)azabut-1-yl)isoquinolin-3-yl)amino)nicotinonitrile was prepared with reference to reference example 1 or example 5.

MS m/z (ESI): 564.2 [M+H]⁺.

### Example 58

### Preparation of N-(5-isopropyl-8-((2R,3S)-2-methyl-3-((methanesulfonyl)methyl)azetidin-1-yl)isoquinolin-3-yl)-8-methyl-6,8-dihydropyrazole [4',3': 4,5]pyrano[3,2-b]pyridin-2-amine

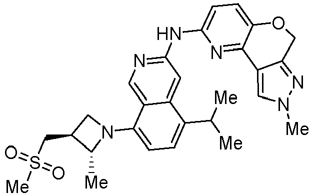

N-(5-isopropyl-8-((2R,3S)-2-methyl-3-((methanesulfonyl)methyl)azetidin-1-yl)isoquinolin-3-yl)-8-methyl-6,8-dihydropyrazole [4',3': 4,5]pyrano[3,2-b]pyridin-2-amine was prepared with reference to reference example 1.

MS m/z (ESI): 533.2 [M+H]⁺.

### Example 59

### Preparation of N-((2R,3S)-1-(7-((2-(3-chloro-1-methyl-1H-pyrazol-4-yl)pyrimidin-4-yl)amino)-1-isopropyl-2,6-diazanaphthalen-4-yl)-2-methylazetidin-3-yl)-N-methyl methanesulfonamide

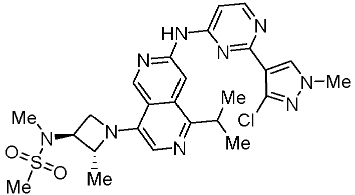

### Step 1 Synthesis of 5-bromo-N-(tert-butyl)-2-chloroisonicotinamide

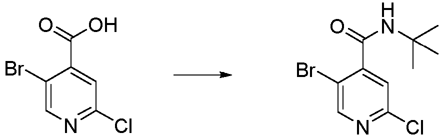

5-Bromo-2-chloroisonicotinic acid (20 g, 84.60 mmol) and 2-methylpropan-2-amine (7.31 g, 100 mmol) were dissolved in DMF (150 mL), HATU (38 g, 100 mmol) was added in batches, and the mixture was reacted at room temperature for 3 hours. The reaction solution was subjected to liquid separation with ethyl acetate and water, and the organic phase was washed with an aqueous saturated NaCl solution. The organic phase was separated and dried over anhydrous sodium sulfate and filtered, and the filtrate was concentrated under reduced pressure and separated by column chromatography to obtain the target compound 5-bromo-N-(tert-butyl)-2-chloroisonicotinamide (15.50 g, 62.8 %).

MS m/z (ESI) :291.0 [M+H]⁺.

### Step 2 Synthesis of (E)-N-(tert-butyl)-2-chloro-5-(2-ethoxyethenyl) isonicotinamide

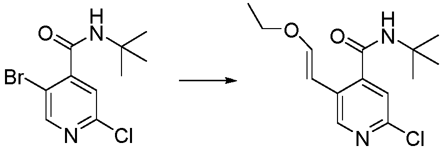

5-Bromo-N-(tert-butyl)-2-chloroisonicotinamide (15.50 g, 53.16 mmol) was dissolved in dioxane (150 mL) and water (30 mL), (E)-2-(2-ethoxyethenyl)-4,4,5,5-tetramethyl-1,3,2-dioxaborolane (11.59 g, 58.50 mmol), cesium carbonate (32.58 g, 100 mmol) and PdCl₂(dppf) (3.89 g, 5.32 mmol) were added, and the mixture was warmed to 80°C and reacted for 2 hours. The reaction solution was cooled to room temperature, and concentrated. The residue was subjected to liquid separation with dichloromethane and water, and the organic phase was dried over anhydrous sodium sulfate, concentrated under reduced pressure. The resulting mixture was separated by column chromatography to obtain the target compound (E)-N-(tert-butyl)-2-chloro-5-(2-ethoxyethenyl)isonicotinamide (9.50 g, 63.2%).

MS m/z (ESI) :283.1 [M+H]⁺.

### Step 3 Synthesis of 7-chloro-2,6-diazanaphthalen-1-ol

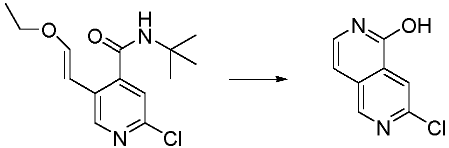

(E)-N-(tert-butyl)-2-chloro-5-(2-ethoxyethenyl)isonicotinamide (9.50 g, 33.59 mmol) was dissolved in TFA (50 mL), and the mixture was warmed to 100°C and reacted for 12 hours. The reaction solution was cooled to room temperature and concentrated under reduced pressure to obtain the crude target compound 7-chloro-2,6-diazanaphthalen-1-ol (5.66 g, 93.3 %).

MS m/z (ESI) :181.0 [M+H]⁺.

### Step 4 Synthesis of 4-bromo-7-chloro-2,6-diazanaphthalen-1-ol

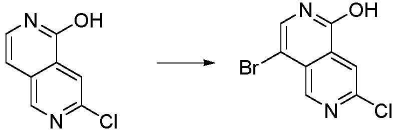

7-Chloro-2,6-diazanaphthalen-1-ol (5.66 g, 31.34 mmol) was dissolved in dichloromethane (75 mL), NBS (6.69 g, 37.61 mmol) was added, and the mixture was reacted at room temperature for 1 hour. The reaction solution was filtered under reduced pressure, and the filter cake was collected and then dried to obtain the crude target compound 4-bromo-7-chloro-2,6-diazanaphthalen-1-ol (5.80 g, 71.3 %).

MS m/z (ESI) :259.0 [M+H]⁺.

### Step 5 Synthesis of 4-bromo-7-chloro-2,6-diazanaphthalen-1-yl trifluoromethanesulfonic acid

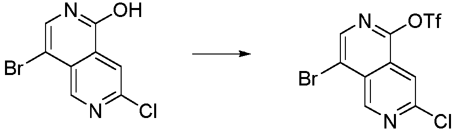

4-Bromo-7-chloro-2,6-diazanaphthalen-1-ol (5.80 g, 22.35 mmol) was dissolved in dichloromethane (80 mL) and TEA (4.52 g, 44.70 mmol), the reaction solution was cooled to - 75°C, (TfO)₂O (25.39 g, 90 mmol) was slowly added dropwise, and the mixture was reacted at - 75°C for 0.5 hours and slowly warmed to room temperature and reacted for 0.5 hours. The reaction was quenched by adding water and extracted with dichloromethane, and the organic phase was dried over anhydrous sodium sulfate and filtered, and the filtrate was concentrated under reduced pressure and separated by column chromatography to obtain the target compound 4-bromo-7-chloro-2,6-diazanaphthalen-1-yl trifluoromethanesulfonic acid (5.71 g, 65.3 %).

MS m/z (ESI) :391.0 [M+H]⁺.

### Step 6 Synthesis of 4-bromo-7-chloro-1-iodo-2,6-diazanaphthalene

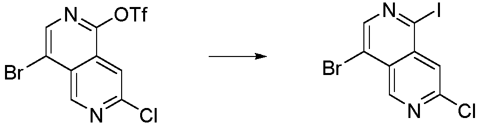

4-Bromo-7-chloro-2,6-diazanaphthalen-1-yl trifluoromethanesulfonic acid (5.71 g, 14.58 mmol) and Nal (10.94 g, 73 mmol) were mixed in ACN (100 mL), the reaction solution was cooled to 0°C, a solution of trifluoromethanesulfonic acid (4.50 g, 30 mmol) in ACN (10 mL) was slowly added dropwise, and the mixture was reacted at room temperature for 2 hours. The reaction solution was subjected to liquid separation with EA and water, and the organic phase was washed with an aqueous saturated NaCl solution. The organic phase was separated and dried over anhydrous sodium sulfate, and filtered, and the filtrate was concentrated under reduced pressure to obtain the crude target compound 4-bromo-7-chloro-1-iodo-2,6-diazanaphthalene (5.28 g, 98.0 %).

MS m/z (ESI) :369.0 [M+H]⁺.

### Step 7 Synthesis of 4-bromo-7-chloro-1-(prop-1-en-2-yl)-2,6-diazanaphthalene

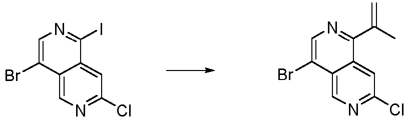

4-Bromo-7-chloro-1-iodo-2,6-diazanaphthalene (5.28 g, 14.29 mmol) was dissolved in dioxane (50 mL) and H₂O (5 mL), isopropenylboronic acid pinacol ester (2.35 g, 14 mmol), potassium carbonate (4.60 g, 33.40 mmol), and PdCl₂(dppf) (610 mg, 0.84 mmol) were added, and the mixture was warmed to 100°C and reacted for 4 hours. The reaction solution was cooled to room temperature, and concentrated. The residue was subjected to liquid separation with dichloromethane and water. The organic phase was dried over anhydrous sodium sulfate, concentrated under reduced pressure and separated by column chromatography to obtain the target compound 4-bromo-7-chloro-1-(prop-1-en-2-yl)-2,6-diazanaphthalene (2.05 g, 50.6%).

MS m/z (ESI) :283.0 [M+H]⁺.

### Step 8 Synthesis of 4-bromo-7-chloro-1-isopropyl-2,6-diazanaphthalene

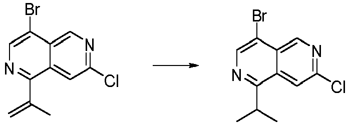

4-Bromo-7-chloro-1-(prop-1-en-2-yl)-2,6-diazanaphthalene (2.05 g, 7.23 mmol) was dissolved in ethyl acetate (80 mL), PtO2 (2.04 g, 9 mmol) was added, and the mixture was stirred at room temperature for 3 hours under hydrogen atmosphere. The reaction solution was filtered, and the filtrate was concentrated under reduced pressure and separated by column chromatography to obtain the target compound 4-bromo-7-chloro-1-isopropyl-2,6-diazanaphthalene (1.20 g, 58.1%).

MS m/z (ESI) :285.0 [M+H]⁺.

### Step 9 Synthesis of N-((2R,3S)-1-(7-chloro-1-isopropyl-2,6-diazanaphthalen-4-yl)-2-methylazetidin-3-yl)-N-methyl methanesulfonamide

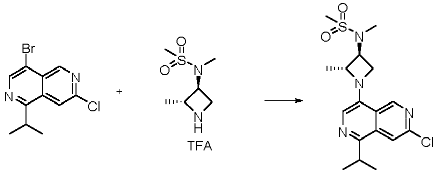

4-Bromo-7-chloro-1-isopropyl-2,6-diazanaphthalene (471 mg, 1.65 mmol) and N-methyl-N-((2R,3S)-2-methylazetidin-3-yl)methanesulfonamide trifluoroacetate (470 mg, 1.70 mmol) were mixed in dioxane (10 mL), cesium carbonate (1.15 g, 3.54 mmol) and Xantphos Pd G4 (164 mg, 0.17 mmol) were added, and the mixture was warmed to 100°C and stirred for 5 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound N-((2R,3S)-1-(7-chloro-1-isopropyl-2,6-diazanaphthalen-4-yl)-2-methylazetidin-3-yl)-N-methyl methanesulfonamide (230 mg, 36.4%).

MS m/z (ESI) :383.1 [M+H]⁺.

### Step 10 Synthesis of N-((2R,3S)-1-(7-((2-(3-chloro-1-methyl-1H-pyrazol-4-yl)pyrimidin-4-yl)amino)-1-isopropyl-2,6-diazanaphthalen-4-yl)-2-methylazetidin-3-yl)-N-methyl methanesulfonamide

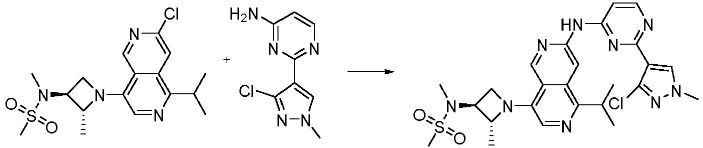

N-((2R,3S)-1-(7-chloro-1-isopropyl-2,6-diazanaphthalen-4-yl)-2-methylazetidin-3-yl)-N-methyl methanesulfonamide (100 mg, 0.26 mmol) and 2-(3-chloro-1-methyl-1H-pyrazol-4-yl)pyrimidin-4-amine (55 mg, 0.26 mmol) were dissolved in dioxane (5 mL), cesium carbonate (274 mg, 0.84 mmol) and BrettPhos Pd G4 (24 mg, 26 µmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate and filtered, and the filtrate was concentrated under reduced pressure to remove the organic solvent and separated by column chromatography to obtain the target compound N-((2R,3S)-1-(7-((2-(3-chloro-1-methyl-1H-pyrazol-4-yl)pyrimidin-4-yl)amino)-1-isopropyl-2,6-diazanaphthalen-4-yl)-2-methylazetidin-3-yl)-N-methyl methanesulfonamide (56 mg, 38.7%).

MS m/z (ESI) :556.2 [M+H]⁺.

### Example 60

### Preparation of N-(1-(7-((2-(1,3-dimethyl-1H-pyrazol-4-yl)pyrimidin-4-yl)amino)-1-isopropyl-2,6-diazanaphthalen-4-yl)azetidin-3-yl)-N-methyl methanesulfonamide

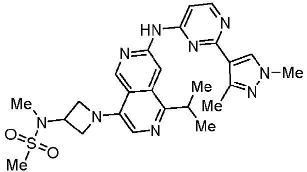

N-(1-(7-((2-(1,3-Dimethyl-1H-pyrazol-4-yl)pyrimidin-4-yl)amino)-1-isopropyl-2,6-diazanaphthalen-4-yl)azetidin-3-yl)-N-methyl methanesulfonamide was prepared with reference to example 59.

MS m/z (ESI):522.2 [M+H]⁺.

### Example 61

### Preparation of N-((2R,3S)-1-(7-((2-(1,3-dimethyl-1H-pyrazol-4-yl)pyrimidin-4-yl)amino)-1-isopropyl-2,6-diazanaphthalen-4-yl)-2-methylazetidin-3-yl)-N-methyl methanesulfonamide

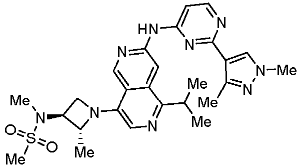

N-((2R,3S)-1-(7-((2-(1,3-dimethyl-1H-pyrazol-4-yl)pyrimidin-4-yl)amino)-1-isopropyl-2,6-diazanaphthalen-4-yl)-2-methylazetidin-3-yl)-N-methyl methanesulfonamide was prepared with reference to example 59.

MS m/z (ESI):536.2 [M+H]⁺.

### Example 62

### Preparation of 2-(3-fluoro-4-(4-((5-isopropyl-8-((2R,3S)-2-methyl-3-((methanesulfonyl)methyl)azabut-1-yl)isoquinolin-3-yl)amino)pyrimidin-2-yl)-1H-pyrazol-1-yl)ethanol

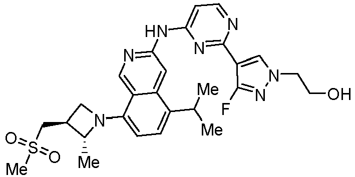

2-(3-Fluoro-4-(4-((5-isopropyl-8-((2R,3S)-2-methyl-3-((methanesulfonyl) methyl)azabut-1-yl)isoquinolin-3-yl)amino)pyrimidin-2-yl)-1H-pyrazol-1-yl)ethanol was prepared with reference to reference example 1 or 2.

MS m/z (ESI): 554.2 [M+H]⁺.

### Example 63

Preparation of (S) -N-(2-(3-chloro-1-methyl-1H-pyrazol-4-yl)pyrimidin-4-yl)-9-methyl-5-((2R,3S)-2-methyl-3-((methanesulfonyl)methyl)azetidin-1-yl)-8,9-dihydro-7H-cyclopentane [f]isoquinolin-2-amine

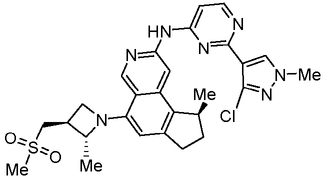

(S) -N-(2-(3-Chloro-1-methyl-1H-pyrazol-4-yl)pyrimidin-4-yl)-9-methyl-5-((2R,3S)-2-methyl-3-((methanesulfonyl)methyl)azetidin-1-yl)-8,9-dihydro-7H-cyclopentane[f]isoquinolin-2-amine was prepared with reference to reference example 1 or example 5.

MS m/z (ESI): 552.2 [M+H]⁺.

### Example 64

### Preparation of (R) -N-(2-(3-chloro-1-methyl-1H-pyrazol-4-yl)pyrimidin-4-yl)-9-methyl-5-((2R,3S)-2-methyl-3-((methanesulfonyl)methyl)azetidin-1-yl)-8,9-dihydro-7H-cyclopentane [f]isoquinolin-2-amine

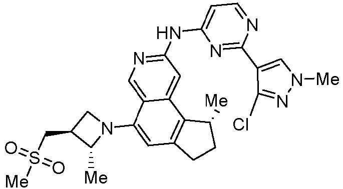

(R) -N-(2-(3-Chloro-1-methyl-1H-pyrazol-4-yl)pyrimidin-4-yl)-9-methyl-5-((2R,3S)-2-methyl-3-((methanesulfonyl)methyl)azetidin-1-yl)-8,9-dihydro-7H-cyclopentane[f]isoquinolin-2-amine was prepared with reference to reference example 1 or example 5.

MS m/z (ESI): 552.2 [M+H]⁺.

### Example 65

### Preparation of N-(2-(3-chloro-1-methyl-1H-pyrazol-4-yl)pyrimidin-4-yl)-5,5-dimethyl-8-((2R,3S)-2-methyl-3-((methanesulfonyl)methyl)azetidin-1-yl)-4,5-dihydrocyclopentane [de]isoquinolin-3-amine

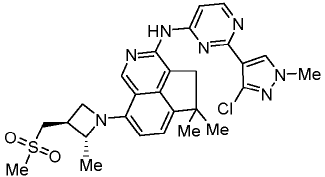

N-(2-(3-Chloro-1-methyl-1H-pyrazol-4-yl)pyrimidin-4-yl)-5,5-dimethyl-8-((2R,3S)-2-methyl-3-((methanesulfonyl)methyl)azetidin-1-yl)-4,5-dihydrocyclopentane [de]isoquinolin-3-amine was prepared with reference to reference example 1 or example 5.

MS m/z (ESI): 552.2 [M+H]⁺.

### Example 66

### Preparation of N-((2R,3S)-1-(5-isopropyl-3-((2-(1-methyl-1H-pyrazol-4-yl)pyrimidin-4-yl)amino)isoquinolin-8-yl)-2-methylazetidin-3-yl)methanesulfonimide

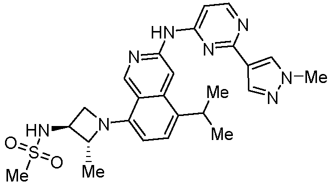

N-((2R,3S)-1-(5-Isopropyl-3-((2-(1-methyl-1H-pyrazol-4-yl)pyrimidin-4-yl)amino)isoquinolin-8-yl)-2-methylazetidin-3-yl)methanesulfonimide was prepared with reference to reference example 1 or example 44.

MS m/z (ESI): 507.2 [M+H]⁺.

### Example 67

### Preparation of N-(1-(3-((2-(3-chloro-1-methyl-1H-pyrazol-4-yl)pyrimidin-4-yl)amino)-5-isopropylisoquinolin-8-yl)azetidin-3-yl)-N-methyl methanesulfonamide

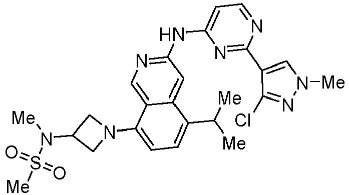

### Step 1 Synthesis of N-(1-(3-chloro-5-isopropylisoquinolin-8-yl)azetidin-3-yl)-N-methyl methanesulfonamide

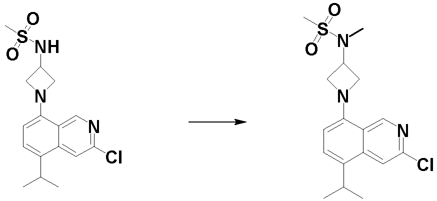

N-(1-(3-Chloro-5-isopropylisoquinolin-8-yl)azetidin-3-yl)methanesulfonamide (300 mg, 0.85 mmol) was dissolved in DMF (6 mL), cesium carbonate (552 mg, 1.70 mmol) and iodomethane (144 mg, 1.02 mmol) were added, and the mixture was stirred at room temperature for 12 hours. The reaction solution was concentrated under reduced pressure. The residue was subjected to liquid separation with dichloromethane and water, and the organic phase was dried over anhydrous sodium sulfate and filtered, and the organic solvent was concentrated under reduced pressure. The resulting mixture was separated by column chromatography to obtain the target compound N-(1-(3-chloro-5-isopropylisoquinolin-8-yl)azetidin-3-yl)-N-methyl methanesulfonamide (150 mg, 48.1%).

MS m/z (ESI) :368.1 [M+H]⁺.

### Step 2 Synthesis of N-(1-(3-((2-(3-chloro-1-methyl-1H-pyrazol-4-yl)pyrimidin-4-yl)amino)-5-isopropylisoquinolin-8-yl)azetidin-3-yl)-N-methyl methanesulfonamide

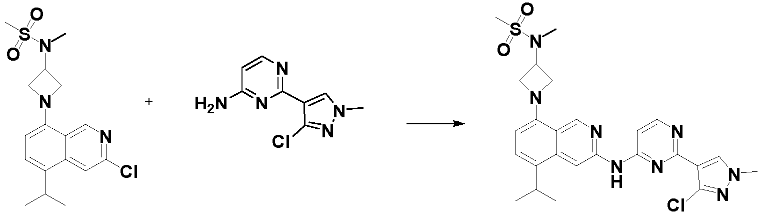

N-(1-(3-chloro-5-isopropylisoquinolin-8-yl)azetidin-3-yl)-N-methyl methanesulfonamide (60 mg, 0.16 mmol) and 2-(3-chloro-1-methyl-1H-pyrazol-4-yl)pyrimidin-4-amine (34 mg, 0.16 mmol) were dissolved in dioxane (5 mL), cesium carbonate (104 mg, 0.32 mmol) and Xantphos Pd G4 (26 mg, 27 µmol) were added, and the mixture was reacted under microwave at 120°C for 2 hours. The reaction solution was cooled to room temperature, and concentrated under reduced pressure. The residue was subjected to liquid separation with dichloromethane and water. The organic phase was dried over anhydrous sodium sulfate and filtered, and the organic solvent was concentrated under reduced pressure. The resulting mixture was separated by column chromatography to obtain the target compound N-(1-(3-((2-(3-chloro-1-methyl-1H-pyrazol-4-yl)pyrimidin-4-yl)amino)-5-isopropylisoquinolin-8-yl)azetidin-3-yl)-N-methyl methanesulfonamide (18 mg, 20.4%).
¹H NMR (400 MHz, DMSO-*d*₆) *δ* 10.15 (s, 1H), 9.12 (s, 1H), 8.56 (s, 1H), 8.41-8.39 (m, 1H), 8.33 (s, 1H), 7.43 (d, *J* = 7.6 Hz, 1H), 7.32-7.27 (m, 1H), 6.47 (d, *J* = 8.2 Hz, 1H), 4.70-4.65 (m, 1H), 4.43-4.38 (m, 2H), 4.25-4.21 (m, 2H), 3.90 (s, 3H), 3.61-3.54 (m, 1H), 2.96 (s, 3H), 2.93 (s, 3H), 1.31-1.28 (m, 6H);
MS m/z (ESI): 541.2 [M+H]⁺.

### Example 68

### Preparation of N-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)-N-methyl methanesulfonamide

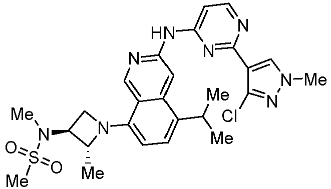

N-((2R,3S)-1-(3-((2-(3-Chloro-1-methyl-1H-pyrazol-4-yl)pyrimidin-4-yl)amino)-5-isopropylisoquinolin-8-yl)-2-methylazetidin-3-yl)-N-methyl methanesulfonamide was prepared with reference to example 67.

MS m/z (ESI): 555.2 [M+H]⁺.

### Example 69

### Preparation of N-(1-(3-((2-(3-chloro-1-(methyl-d3)-1H-pyrazol-4-yl)pyrimidin-4-yl)amino)-5-isopropylisoquinolin-8-yl)azetidin-3-yl)-N-methyl methanesulfonamide

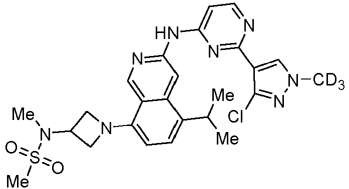

N-(1-(3-((2-(3-Chloro-1-(methyl-d3)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 544.2 [M+H]⁺.

### Example 70

### Preparation of N-((2R,3S)-1-(3-((2-(3-chloro-1-(methyl-d3)-1H-pyrazol-4-yl)pyrimidin-4-yl)amino)-5-isopropylisoquinolin-8-yl)-2-methylazetidin-3-yl)-N-methyl methanesulfonamide

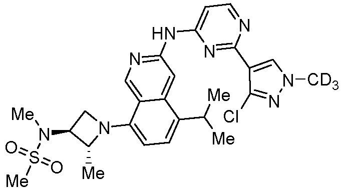

N-((2R,3S)-1-(3-((2-(3-Chloro-1-(methyl-d3)-1H-pyrazol-4-yl)pyrimidin-4-yl)amino)-5-isopropylisoquinolin-8-yl)-2-methylazetidin-3-yl)-N-methyl methanesulfonamide was prepared with reference to example 67.

MS m/z (ESI): 558.2 [M+H]⁺.

### Example 71

### Preparation of N-(1-(3-((2-(1,3-dimethyl-1H-pyrazol-4-yl)pyrimidin-4-yl)amino)-5-isopropylisoquinolin-8-yl)azetidin-3-yl)-N-methyl methanesulfonamide

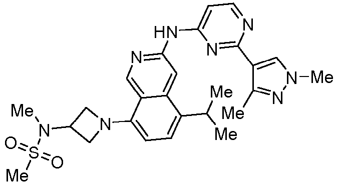

N-(1-(3-((2-(1,3-Dimethyl-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 521.2 [M+H]⁺.

### Example 72

### Preparation of N-((2R,3S)-1-(3-((2-(1,3-dimethyl-1H-pyrazol-4-yl)pyrimidin-4-yl)amino)-5-isopropylisoquinolin-8-yl)-2-methylazetidin-3-yl)-N-methyl methanesulfonamide

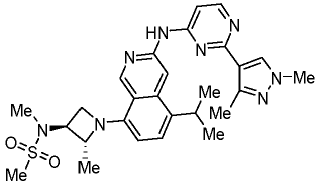

N-((2R,3S)-1-(3-((2-(1,3-Dimethyl-1H-pyrazol-4-yl)pyrimidin-4-yl)amino)-5-isopropylisoquinolin-8-yl)-2-methylazetidin-3-yl)-N-methyl methanesulfonamide was prepared with reference to example 67.

MS m/z (ESI): 535.2 [M+H]⁺.

### Example 73

### Preparation of N-(2-(3-chloro-1-(2-(methylamino)ethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

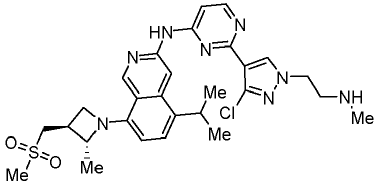

N-(2-(3-Chloro-1-(2-(methylamino)ethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1 or 2.

MS m/z (ESI): 583.2 [M+H]⁺.

### Example 74

### Preparation of N-(2-(3-chloro-1-(2-(dimethylamino)ethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

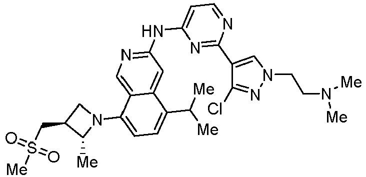

N-(2-(3-Chloro-1-(2-(dimethylamino)ethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1 or 2.

MS m/z (ESI): 597.2 [M+H]⁺.

### Example 75

### Preparation of N-(1-(3-((2-(1-(2-aminoethyl)-3-chloro-1H-pyrazol-4-yl)pyrimidin-4-yl)amino)-5-isopropylisoquinolin-8-yl)azetidin-3-yl)-N-methyl methanesulfonamide

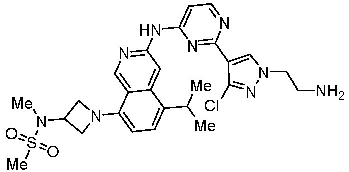

N-(1-(3-((2-(1-(2-Aminoethyl)-3-chloro-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 570.2 [M+H]⁺.

### Example 76

### Preparation of N-(1-(3-((2-(3-chloro-1-(2-(methylamino)ethyl)-1H-pyrazol-4-yl)pyrimidin-4-yl)amino)-5-isopropylisoquinolin-8-yl)azetidin-3-yl)-N-methyl methanesulfonamide

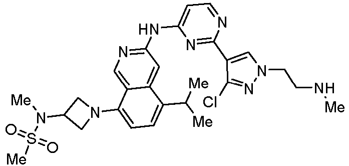

N-(1-(3-((2-(3-Chloro-1-(2-(methylamino)ethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 584.2 [M+H]⁺.

### Example 77

### Preparation of N-(1-(3-((2-(3-chloro-1-(2-(dimethylamino)ethyl)-1H-pyrazol-4-yl)pyrimidin-4-yl)amino)-5-isopropylisoquinolin-8-yl)azetidin-3-yl)-N-methyl methanesulfonamide

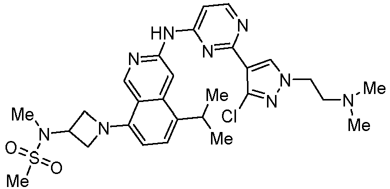

N-(1-(3-((2-(3-Chloro-1-(2-(dimethylamino)ethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 598.2 [M+H]⁺.

### Example 78

### Preparation of N-((2R,3S)-1-(3-((2-(3-chloro-1-(2-hydroxyethyl)-1H-pyrazol-4-yl)pyrimidin-4-yl)amino)-5-isopropylisoquinolin-8-yl)-2-methylazetidin-3-yl)-N-methyl methanesulfonamide

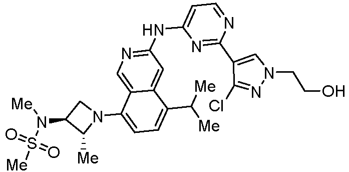

N-((2R,3S)-1-(3-((2-(3-Chloro-1-(2-hydroxyethyl)-1H-pyrazol-4-yl)pyrimidin-4-yl)amino)-5-isopropylisoquinolin-8-yl)-2-methylazetidin-3-yl)-N-methyl methanesulfonamide was prepared with reference to example 67.

MS m/z (ESI): 585.2 [M+H]⁺.

### Example 79

### Preparation of N-(1-(3-((2-(3-chloro-1-(oxetan-3-yl)-1H-pyrazol-4-yl)pyrimidin-4-yl)amino)-5-isopropylisoquinolin-8-yl)azetidin-3-yl)-N-methyl methanesulfonamide

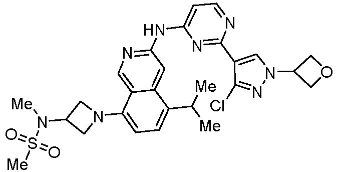

N-(1-(3-((2-(3-Chloro-1-(oxetan-3-yl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 583.2 [M+H]⁺.

### Example 80

### Preparation of N-(2-(3-chloro-1-(((S)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

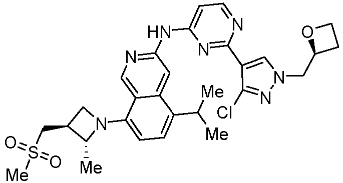

N-(2-(3-Chloro-1-(((S)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1 or 2.

MS m/z (ESI): 596.2 [M+H]⁺.

### Example 81

### Preparation of N-(2-(3-chloro-1-(((R)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

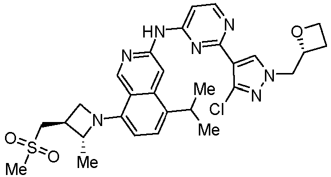

N-(2-(3-chloro-1-(((R)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1 or 2.

MS m/z (ESI): 596.2 [M+H]⁺.

### Example 82

### Preparation of (S)-N-(1-(3-((2-(3-chloro-1-(oxetan-2-ylmethyl)-1H-pyrazol-4-yl)pyrimidin-4-yl)amino)-5-isopropylisoquinolin-8-yl)azetidin-3-yl)-N-methyl methanesulfonamide

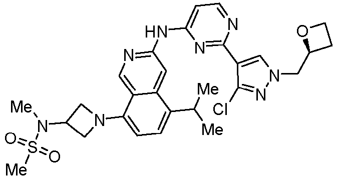

(S)-N-(1-(3-((2-(3-Chloro-1-(oxetan-2-ylmethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 597.2 [M+H]⁺.

### Example 83

### Preparation of (R)-N-(1-(3-((2-(3-chloro-1-(oxetan-2-ylmethyl)-1H-pyrazol-4-yl)pyrimidin-4-yl)amino)-5-isopropylisoquinolin-8-yl)azetidin-3-yl)-N-methyl methanesulfonamide

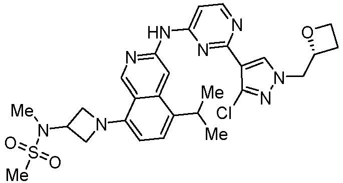

(R)-N-(1-(3-((2-(3-Chloro-1-(oxetan-2-ylmethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 597.2 [M+H]⁺.

### Example 84

### Preparation of N-(1-(3-((2-(3-chloro-1-(difluoromethyl)-1H-pyrazol-4-yl)pyrimidin-4-yl)amino)-5-isopropylisoquinolin-8-yl)azetidin-3-yl)-N-methyl methanesulfonamide

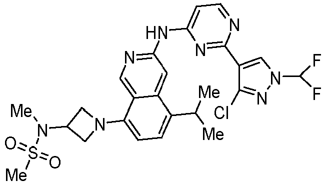

N-(1-(3-((2-(3-Chloro-1-(difluoromethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 577.2 [M+H]⁺.

### Example 85

### Preparation of N-(2-(3-chloro-1-(difluoromethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

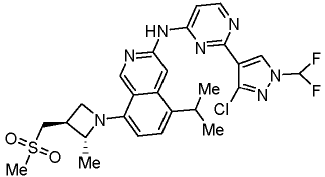

N-(2-(3-chloro-1-(difluoromethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1 or 2.

MS m/z (ESI): 576.2 [M+H]⁺.

### Example 86

### Preparation of N-(1-(3-((2-(3-chloro-1-(2-methoxyethyl)-1H-pyrazol-4-yl)pyrimidin-4-yl)amino)-5-isopropylisoquinolin-8-yl)azetidin-3-yl)-N-methyl methanesulfonamide

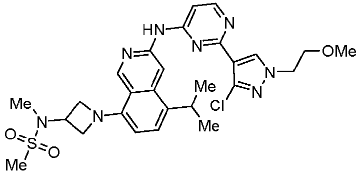

N-(1-(3-((2-(3-Chloro-1-(2-methoxyethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 585.2 [M+H]⁺.

### Example 87

### Preparation of N-(1-(3-((2-(3-chloro-1-(2-cyanoethyl)-1H-pyrazol-4-yl)pyrimidin-4-yl)amino)-5-isopropylisoquinolin-8-yl)azetidin-3-yl)-N-methyl methanesulfonamide

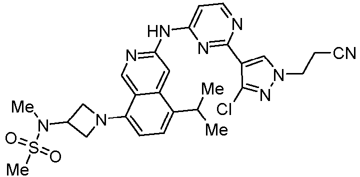

N-(1-(3-((2-(3-Chloro-1-(2-cyanoethyl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 580.2 [M+H]⁺.

### Example 88

### Preparation of N-(1-(3-((2-(3-chloro-1-(tetrahydro-2H-pyran-4-yl)-1H-pyrazol-4-yl)pyrimidin-4-yl)amino)-5-isopropylisoquinolin-8-yl)azetidin-3-yl)-N-methyl methanesulfonamide

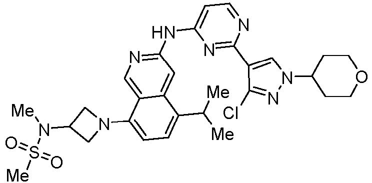

N-(1-(3-((2-(3-Chloro-1-(tetrahydro-2H-pyran-4-yl)-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 611.2 [M+H]⁺.

### Example 89

### Preparation of N-(1-(3-((2-(5-fluoro-1-methyl-1H-pyrazol-4-yl)pyrimidin-4-yl)amino)-5-isopropylisoquinolin-8-yl)azetidin-3-yl)-N-methyl methanesulfonamide

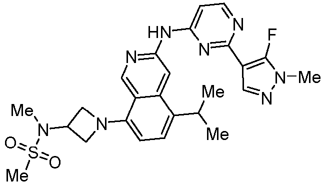

N-(1-(3-((2-(5-Fluoro-1-methyl-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 525.2 [M+H]⁺.

### Example 90

### Preparation of N-(2-(5-fluoro-1-methyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

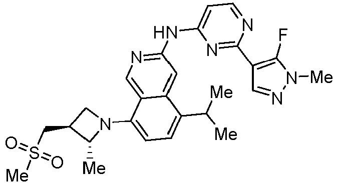

N-(2-(5-fluoro-1-methyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1.

MS m/z (ESI): 524.2 [M+H]⁺.

### Example 91

### Preparation of N-(1-(3-((2-(5-chloro-1-methyl-1H-pyrazol-4-yl)pyrimidin-4-yl)amino)-5-isopropylisoquinolin-8-yl)azetidin-3-yl)-N-methyl methanesulfonamide

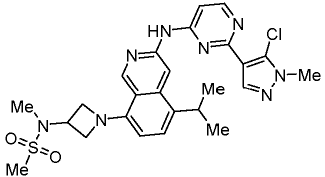

N-(1-(3-((2-(5-Chloro-1-methyl-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 541.2 [M+H]⁺.

### Example 92

### Preparation of N-(2-(5-chloro-1-methyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

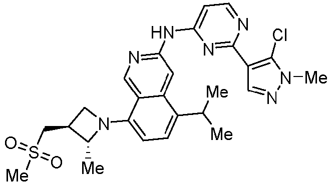

N-(2-(5-chloro-1-methyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1.
¹H NMR (400 MHz, DMSO-*d*₆) *δ* 10.24 (s, 1H), 9.10 (s, 1H), 8.67 (s, 1H), 8.42 (d, *J* = 5.8 Hz, 1H), 8.18 (s, 1H), 7.45 (d, *J* = 8.0 Hz, 1H), 7.24 (d, *J* = 5.6 Hz, 1H), 6.58 (d, *J* = 8.1 Hz, 1H), 4.72-4.64 (m, 1H), 4.24-4.18 (m, 1H), 3.90 (s, 3H), 3.69-3.63 (m, 1H), 3.60-3.50 (m, 3H), 3.00 (s, 3H), 2.92-2.86 (m, 1H), 1.43 (d, *J* = 6.0 Hz, 3H), 1.35-1.30 (m, 6H);
MS m/z (ESI): 540.2 [M+H]⁺.

### Example 93

### Preparation of N-(1-(3-((2-(5-fluoro-1,3-dimethyl-1H-pyrazol-4-yl)pyrimidin-4-yl)amino)-5-isopropylisoquinolin-8-yl)azetidin-3-yl)-N-methyl methanesulfonamide

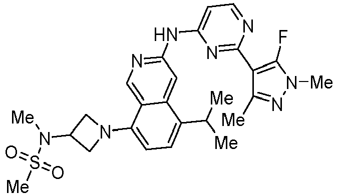

N-(1-(3-((2-(5-Fluoro-1,3-dimethyl-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 539.2 [M+H]⁺.

### Example 94

### Preparation of N-(1-(3-((2-(5-chloro-1,3-dimethyl-1H-pyrazol-4-yl)pyrimidin-4-yl)amino)-5-isopropylisoquinolin-8-yl)azetidin-3-yl)-N-methyl methanesulfonamide

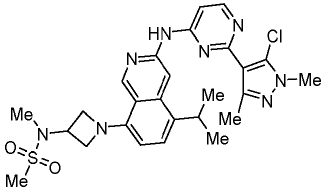

N-(1-(3-((2-(5-Chloro-1,3-dimethyl-1H-pyrazol-4-yl)pyrimidin-4-yl)amino)-5-isopropylisoquinolin-8-yl)azetidin-3-yl)-N-methyl methanesulfonamide was prepared with reference to example 67.

MS m/z (ESI): 555.2 [M+H]⁺.

### Example 95

### Preparation of N-(2-(5-chloro-1,3-dimethyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

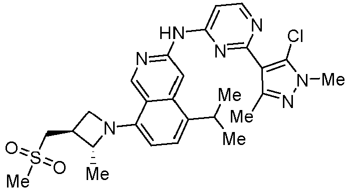

N-(2-(5-chloro-1,3-dimethyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 1.

MS m/z (ESI): 554.2 [M+H]⁺.

### Example 96

### Preparation of (R)-2-(1-(3-((2-(3-chloro-1-methyl-1H-pyrazol-4-yl)pyrimidin-4-yl)amino)-5-isopropylisoquinolin-8-yl)azetidin-3-yl)tetrahydrothiophene 1,1-dioxide

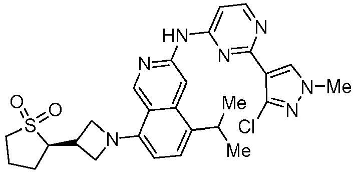

(R)-2-(1-(3-((2-(3-Chloro-1-methyl-1H-pyrazol-4-yl)pyrimidin-4-yl)amino)-5-isopropylisoquinolin-8-yl)azetidin-3-yl)tetrahydrothiophene 1,1-dioxide was prepared with reference to reference example 1 or example 5.

MS m/z (ESI): 552.2 [M+H]⁺.

### Example 97

### Preparation of (S)-2-(1-(3-((2-(3-chloro-1-methyl-1H-pyrazol-4-yl)pyrimidin-4-yl)amino)-5-isopropylisoquinolin-8-yl)azetidin-3-yl)tetrahydrothiophene 1,1-dioxide

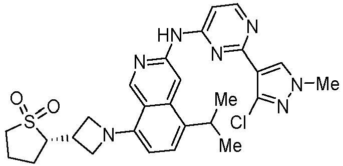

(S)-2-(1-(3-((2-(3-Chloro-1-methyl-1H-pyrazol-4-yl)pyrimidin-4-yl)amino)-5-isopropylisoquinolin-8-yl)azetidin-3-yl)tetrahydrothiophene 1,1-dioxide was prepared with reference to reference example 1 or example 5.

MS m/z (ESI): 552.2 [M+H]⁺.

### Example 98

### Preparation of (R)-2-(1-(3-((2-(1,3-dimethyl-1H-pyrazol-4-yl)pyrimidin-4-yl)amino)-5-isopropylisoquinolin-8-yl)azetidin-3-yl)tetrahydrothiophene 1,1-dioxide

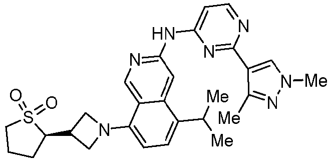

(R)-2-(1-(3-((2-(1,3-Dimethyl-1H-pyrazol-4-yl)pyrimidin-4-yl)amino)-5-isopropylisoquinolin-8-yl)azetidin-3-yl)tetrahydrothiophene 1,1-dioxide was prepared with reference to reference example 1.

MS m/z (ESI): 532.2 [M+H]⁺.

### Example 99

### Preparation of (S)-2-(1-(3-((2-(1,3-dimethyl-1H-pyrazol-4-yl)pyrimidin-4-yl)amino)-5-isopropylisoquinolin-8-yl)azetidin-3-yl)tetrahydrothiophene 1,1-dioxide

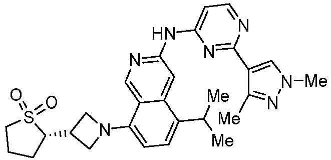

(S)-2-(1-(3-((2-(1,3-Dimethyl-1H-pyrazol-4-yl)pyrimidin-4-yl)amino)-5-isopropylisoquinolin-8-yl)azetidin-3-yl)tetrahydrothiophene 1,1-dioxide was prepared with reference to reference example 1.

MS m/z (ESI): 532.2 [M+H]⁺.

### Example 100

### Preparation of N-(((1-(3-((2-(3-chloro-1-methyl-1H-pyrazol-4-yl)pyrimidin-4-yl)amino)-5-isopropylisoquinolin-8-yl)azetidin-3-yl)methyl)(methyl)(carbonyl)-l6-sulfanylidene)cyanamide

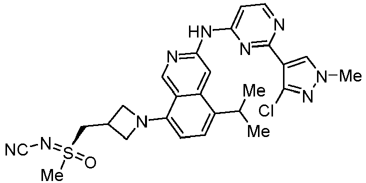

N-(((1-(3-((2-(3-Chloro-1-methyl-1H-pyrazol-4-yl)pyrimidin-4-yl)amino)-5-isopropylisoquinolin-8-yl)azetidin-3-yl)methyl)(methyl)(carbonyl)-l6-sulfanylidene)cyanamide was prepared with reference to example 67 or example 5.

MS m/z (ESI): 550.2 [M+H]⁺.

### Example 101

### Preparation of N-(((1-(3-((2-(3-chloro-1-methyl-1H-pyrazol-4-yl)pyrimidin-4-yl)amino)-5-isopropylisoquinolin-8-yl)azetidin-3-yl)methyl)(methyl)(carbonyl)-l6-sulfanylidene)cyanamide

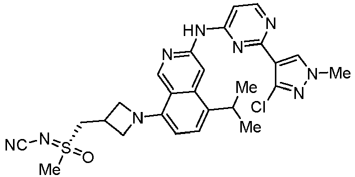

N-(((1-(3-((2-(3-Chloro-1-methyl-1H-pyrazol-4-yl)pyrimidin-4-yl)amino)-5-isopropylisoquinolin-8-yl)azetidin-3-yl)methyl)(methyl)(carbonyl)-l6-sulfanylidene)cyanamide was prepared with reference to example 67 or example 5.

MS m/z (ESI): 550.2 [M+H]⁺.

### Example 102

### Preparation of ((1-(3-((2-(3-chloro-1-methyl-1H-pyrazol-4-yl)pyrimidin-4-yl)amino)-5-isopropylisoquinolin-8-yl)azetidin-3-yl)imino)dimethyl-l6-sulfanone

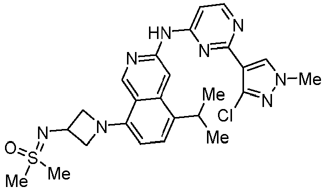

((1-(3-((2-(3-Chloro-1-methyl-1H-pyrazol-4-yl)pyrimidin-4-yl)amino)-5-isopropylisoquinolin-8-yl)azetidin-3-yl)imino)dimethyl-l6-sulfanone was prepared with reference to example 67 or example 5.
¹H NMR (400 MHz, DMSO-*d₆*) *δ* 10.14 (s, 1H), 9.09 (s, 1H), 8.55 (s, 1H), 8.40 (d, *J* = 5.8 Hz, 1H), 8.33 (s, 1H), 7.41 (d, *J* = 8.0 Hz, 1H), 7.27 (d, *J* = 6.0 Hz, 1H), 6.41 (d, *J* = 8.0 Hz, 1H), 4.48-4.39 (m, 3H), 3.90 (s, 3H), 3.82-3.78 (m, 2H), 3.58-3.55 (m, 1H), 3.05 (s, 6H), 1.30 (d, *J* = 6.8 Hz, 6H);
MS m/z (ESI): 525.2 [M+H]⁺.

### Example 103

### Preparation of N-(2-(3-chloro-1-methyl-1H-pyrazol-4-yl)pyrimidin-4-yl)-5-isopropyl-8-((1s,3s)-3-((methanesulfonyl)methyl)cyclobutyl)isoquinolin-3-amine

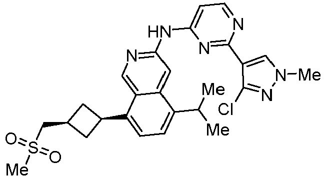

N-(2-(3-Chloro-1-methyl-1H-pyrazol-4-yl)pyrimidin-4-yl)-5-isopropyl-8-((1s,3s)-3-((methanesulfonyl)methyl)cyclobutyl)isoquinolin-3-amine was prepared with reference to example 67 or example 5.

MS m/z (ESI): 525.2 [M+H]⁺.

### Example 104

### Preparation of N-(2-(3-chloro-1-methyl-1H-pyrazol-4-yl)pyrimidin-4-yl)-5-isopropyl-8-((1r,3r)-3-((methanesulfonyl)methyl)cyclobutyl)isoquinolin-3-amine

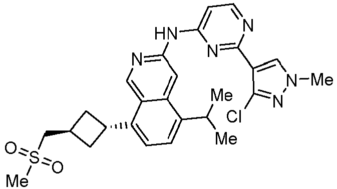

N-(2-(3-Chloro-1-methyl-1H-pyrazol-4-yl)pyrimidin-4-yl)-5-isopropyl-8-((1r,3r)-3-((methanesulfonyl)methyl)cyclobutyl)isoquinolin-3-amine was prepared with reference to example 67 or example 5.

MS m/z (ESI): 525.2 [M+H]⁺.

### Example 105

### Preparation of N-((1s,3s)-3-(3-((2-(3-chloro-1-methyl-1H-pyrazol-4-yl)pyrimidin-4-yl)amino)-5-isopropylisoquinolin-8-yl)cyclobutyl)-N-methyl methanesulfonamide

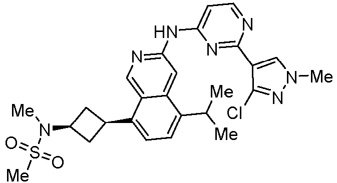

N-((1s,3s)-3-(3-((2-(3-Chloro-1-methyl-1H-pyrazol-4-yl)pyrimidin-4-yl)amino)-5-isopropylisoquinolin-8-yl)cyclobutyl)-N-methyl methanesulfonamide was prepared with reference to example 67.

MS m/z (ESI): 540.2 [M+H]⁺.

### Example 106

### Preparation of N-((1r,3r)-3-(3-((2-(3-chloro-1-methyl-1H-pyrazol-4-yl)pyrimidin-4-yl)amino)-5-isopropylisoquinolin-8-yl)cyclobutyl)-N-methyl methanesulfonamide

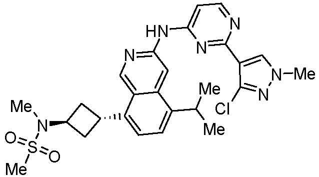

N-((1r,3r)-3-(3-((2-(3-Chloro-1-methyl-1H-pyrazol-4-yl)pyrimidin-4-yl)amino)-5-isopropylisoquinolin-8-yl)cyclobutyl)-N-methyl methanesulfonamide was prepared with reference to example 67.

MS m/z (ESI): 540.2 [M+H]⁺.

### Example 107

### Preparation of 3-(1-(3-((2-(3-chloro-1-methyl-1H-pyrazol-4-yl)pyrimidin-4-yl)amino)-5-isopropylisoquinolin-8-yl)azetidin-3-yl)-1,2,3-oxathiazolidine 2,2-dioxide

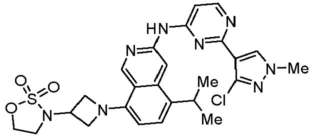

3-(1-(3-((2-(3-Chloro-1-methyl-1H-pyrazol-4-yl)pyrimidin-4-yl)amino)-5-isopropylisoquinolin-8-yl)azetidin-3-yl)-1,2,3-oxathiazolidine 2,2-dioxide was prepared with reference to reference example 1 or example 5.

MS m/z (ESI): 555.2 [M+H]⁺.

### Example 108

### Preparation of 3-(1-(3-(2-(3-chloro-1-methyl-1H-pyrazol-4-yl)pyrimidin-4-yl)amino)-5-isopropylisoquinolin-8-yl)azetidin-3-yl)oxazolidin-2-one

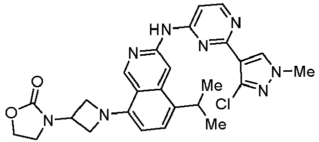

3-(1-(3-(2-(3-Chloro-1-methyl-1H-pyrazol-4-yl)pyrimidin-4-yl)amino)-5-isopropylisoquinolin-8-yl)azetidin-3-yl)oxazolidin-2-one was prepared with reference to reference example 1 or example 5.

MS m/z (ESI): 519.2 [M+H]⁺.

### Example 109

### Preparation of 3-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)oxazolidin-2-one

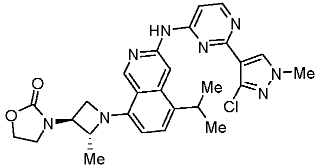

3-((2R,3S)-1-(3-((2-(3-Chloro-1-methyl-1H-pyrazol-4-yl)pyrimidin-4-yl)amino)-5-isopropylisoquinolin-8-yl)-2-methylazetidin-3-yl)oxazolidin-2-one was prepared with reference to example 5.
¹H NMR (400 MHz, DMSO-*d*₆) *δ* 10.26 (s, 1H), 9.08 (s, 1H), 8.52 (s, 1H), 8.35 (d, *J* = 6.0 Hz, 1H), 8.28 (s, 1H), 7.40 (d, *J* = 8.0 Hz, 1H), 7.20 (d, *J* = 5.2 Hz, 1H), 6.60 (d, *J* = 8.1 Hz, 1H), 4.58 (t, *J* = 7.5 Hz, 1H), 4.40-4.36 (m, 1H), 4.30-4.18 (m, 2H), 3.84 (s, 3H), 3.79 (t, *J* = 7.2 Hz, 1H), 3.68-3.58 (m, 2H), 3.55-3.48 (m, 2H), 1.34 (d, *J* = 6.0Hz, 3H), 1.24(t, *J* = 6.0Hz ,6H);
MS m/z (ESI):533.2 [M+H]⁺.

### Example 110

### Preparation of 1-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropan-2-ol

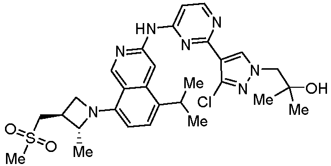

1-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-2-methylpropan-2-ol was prepared with reference to reference example 2.

MS m/z (ESI):598.2 [M+H]⁺.

### Example 111

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2,2-dimethylpropanenitrile

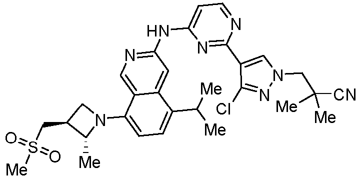

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-2,2-dimethylpropanenitrile was prepared with reference to reference example 2.

MS m/z (ESI):607.2 [M+H]⁺.

### Example 112

### Preparation of 2-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropanenitrile

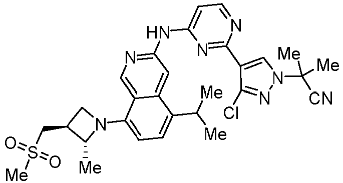

2-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-2-methylpropanenitrile was prepared with reference to reference example 2.
¹H NMR (400 MHz, DMSO-*d*₆) *δ* 10.20 (s, 1H), 9.11 (s, 1H), 8.51-8.40 (m, 3H), 7.43 (d, *J* = 7.8 Hz, 1H), 7.36 (s, 1H), 6.58 (d, *J* = 8.0 Hz, 1H), 4.68 (t, *J* = 7.6 Hz, 1H), 4.46-4.40 (m, 2H), 4.21 (t, *J* = 6.4 Hz, 1H), 3.65 (t, *J* = 7.0 Hz, 1H), 3.59-3.50 (m, 4H), 3.00 (s, 3H), 2.96-2.85 (m, 1H), 1.43 (d,*J* = 6.0 Hz, 3H), 1.33-1.26 (m, 9H);
MS m/z (ESI):593.2 [M+H]⁺.

### Example 113

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1,1,1-trifluoropropan-2-ol

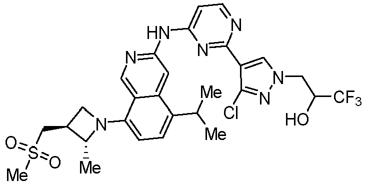

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-1,1,1-trifluoropropan-2-ol was prepared with reference to reference example 2.
¹H NMR (400 MHz, DMSO-*d*₆) *δ* 10.20 (s, 1H), 9.10 (s, 1H), 8.51-8.41 (m, 3H), 7.45 (d,*J* = 8.0 Hz, 1H), 6.86-6.81 (m ,1H), 6.58 (d, *J* = 8.0 Hz, 1H), 5.34-5.30 (m, 1H), 4.70-4.66 (m, 1H), 4.30-4.19 (m, 2H), 3.66-3.48 (m, 5H), 3.00 (s, 3H), 2.92-2.87 (m, 1H), 2.03-1.95 (m, 1H), 1.33-1.27 (m, 9H);
MS m/z (ESI):638.2 [M+H]⁺.

### Example 114

### Preparation of 1-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)propan-2-one

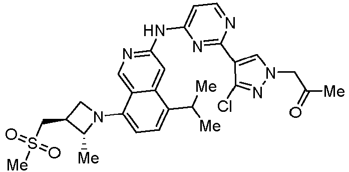

1-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)propan-2-one was prepared with reference to reference example 2.

MS m/z (ESI):582.2 [M+H]⁺.

### Example 115

### Preparation of 2-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)propanenitrile

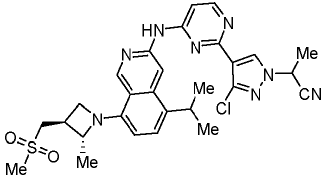

2-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)propanenitrile was prepared with reference to reference example 2.
¹H NMR (400 MHz, DMSO) *δ* 10.24 (s, 1H), 9.10 (s, 1H), 8.55 (s, 1H), 8.48 (s, 1H), 8.44 (d, *J* = 5.9 Hz, 1H), 7.43 (d, *J* = 8.0 Hz, 1H), 7.36 (d, *J* = 4.3 Hz, 1H), 6.58 (d, *J* = 8.1 Hz, 1H), 5.93 (q, *J* = 7.1 Hz, 1H), 4.68 (t, *J* = 7.5 Hz, 1H), 4.23-4.17 (m, 1H), 3.65 (t, *J* = 7.1 Hz, 1H), 3.60-3.50 (m, 3H), 3.00 (s, 3H), 2.95-2.82 (m, 1H), 1.86 (d, *J* = 7.1 Hz, 3H), 1.43 (d, *J* = 6.0 Hz, 3H),1.30-1.26 (m, 6H);
MS m/z (ESI):579.2 [M+H]⁺.

### Example 116A

### Preparation of N-(2-(3-chloro-1-(2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

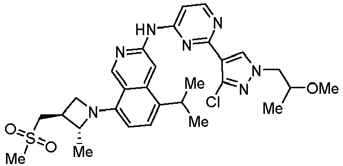

N-(2-(3-chloro-1-(2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d₆*) *δ* 10.19 (s, 1H), 9.10 (s, 1H), 8.53 (s, 1H), 8.40 (s, 1H), 8.30 (s, 1H), 7.43 (d, *J* = 8.0 Hz, 1H), 7.30 (d, *J* = 5.1 Hz, 1H), 6.58 (d, *J* = 8.1 Hz, 1H), 4.68 (t, *J* = 7.5 Hz, 1H), 4.35-4.06 (m, 3H), 3.88-3.68 (m, 1H), 3.68-3.43 (m, 4H), 3.20 (s, 3H), 3.00 (s, 3H), 2.90 (dt, J = 14.6, 7.2 Hz, 1H), 1.43 (d, *J* = 6.0 Hz, 3H), 1.30 (dd, *J* = 12.1, 6.3 Hz, 6H), 1.11 (d, *J* = 6.2 Hz, 3H);
MS m/z (ESI):598.2 [M+H]⁺.

### Example 116

### Preparation of N-(2-(3-chloro-1-((S)-2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

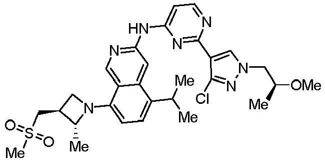

N-(2-(3-chloro-1-((S)-2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2. MS m/z (ESI):598.2 [M+H]⁺.

### Example 117

### Preparation of N-(2-(3-chloro-1-((R)-2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

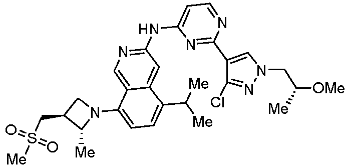

N-(2-(3-chloro-1-((R)-2-methoxypropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2. MS m/z (ESI):598.2 [M+H]⁺.

### Example 118

### Preparation of (S)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropanenitrile

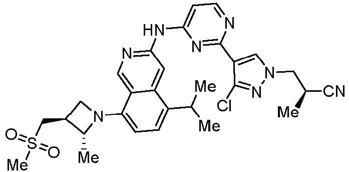

(S)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropanenitrile was prepared with reference to reference example 2.
¹H NMR (400 MHz, DMSO-*d*₆) *δ* 10.20 (s, 1H), 9.10 (s, 1H), 8.47-8.41 (m, 3H), 7.43 (d, *J* = 8.0 Hz, 1H), 7.35 (d, *J* = 4.6 Hz, 1H), 6.58 (d, *J* = 8.1 Hz, 1H), 4.68 (t, *J* = 7.5 Hz, 1H), 4.47-4.38 (m, 2H), 4.22-4.19 (m,1H), 3.65 (t, *J* = 7.1 Hz, 1H), 3.60-3.50 (m, 4H), 3.00 (s, 3H), 2.94-2.87 (m, 1H), 1.43 (d, *J* = 6.1 Hz, 3H), 1.30-1.27 (m, 9H);
MS m/z (ESI):593.2 [M+H]⁺.

### Example 119

### Preparation of (R)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropanenitrile

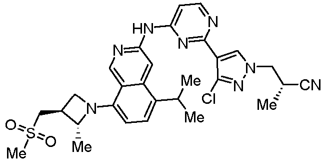

(R)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-2-methylpropanenitrile was prepared with reference to reference example 2.
¹H NMR (400 MHz, DMSO-*d*₆) *δ* 10.20 (s, 1H), 9.10 (s, 1H), 8.47-8.41 (m, 3H), 7.43 (d,*J* = 8.0 Hz, 1H), 7.35 (d, *J* = 4.6 Hz, 1H), 6.58 (d, *J* = 8.1 Hz, 1H), 4.68 (t, *J* = 7.5 Hz, 1H), 4.47-4.38 (m, 2H), 4.22-4.19 (m,1H), 3.65 (t, *J* = 7.1 Hz, 1H), 3.60-3.50 (m, 4H), 3.00 (s, 3H), 2.94-2.87 (m, 1H), 1.43 (d, *J* = 6.1 Hz, 3H), 1.30-1.27 (m, 9H);
MS m/z (ESI):593.2 [M+H]⁺.

### Example 120

### Preparation of N-(2-(3-chloro-1-methyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine

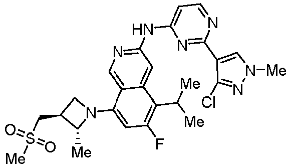

N-(2-(3-Chloro-1-methyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine was prepared with reference to example 5 or prepared as follows:

### Step 1 Synthesis of 3-chloro-6-fluoro-5-isopropyl-8-((2R,3S)-2-methyl-3-((methanesulfonyl)methyl)azetidin-1-yl)isoquinoline

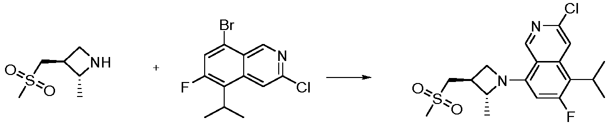

8-Bromo-3-chloro-6-fluoro-5-isopropylisoquinoline (500 mg, 1.65 mmol) and (2R,3S)-2-methyl-3-((methanesulfonyl)methyl)azetidine (297 mg, 1.82 mmol) were dissolved in dioxane (10 mL), cesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound 3-chloro-6-fluoro-5-isopropyl-8-((2R,3S)-2-methyl-3-((methanesulfonyl)methyl)azetidin-1-yl)isoquinoline (418 mg, 65.8%).

MS m/z (ESI) :385.1 [M+H]⁺.

### Step 2 Synthesis of N-(2-(3-chloro-1-methyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine

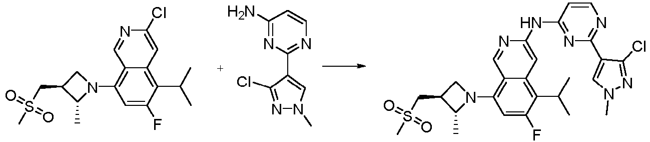

3-Chloro-6-fluoro-5-isopropyl-8-((2R,3S)-2-methyl-3-((methanesulfonyl) methyl)azetidin-1-yl)isoquinoline (100 mg, 0.26 mmol) and 2-(3-chloro-1-methyl-1H-pyrazol-4-yl)pyrimidin-4-amine (54 mg, 0.26 mmol) were dissolved in dioxane (5 mL), cesium carbonate (274 mg, 0.84 mmol) and BrettPhos Pd G4 (24 mg, 26 µmol) were added, and the mixture was warmed to 100°C and stirred for 12 hours. The reaction solution was cooled to room temperature, and concentrated under reduced pressure. The residue was subjected to liquid separation with dichloromethane and water. The organic phase was dried over anhydrous sodium sulfate, and the organic solvent was concentrated under reduced pressure. The resulting mixture was separated by column chromatography to obtain the target compound N-(2-(3-chloro-1-methyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine (61 mg, 42%).
¹H NMR (400 MHz, DMSO-*d*₆) *δ* 10.14 (s, 1H), 8.96 (s, 1H), 8.53 (s, 1H), 8.35 (d, *J* = 5.8 Hz, 1H), 8.24 (s, 1H), 7.19 (d, *J* = 5.8 Hz, 1H), 6.27 (d, *J* = 14.4 Hz, 1H), 4.70-4.62 (m, 1H), 4.21-4.14 (m, 1H), 3.83 (s, 3H), 3.72-3.64 (m, 1H), 3.57-3.41 (m, 3H), 2.93 (s, 3H), 2.87-2.77 (m, 1H), 1.39-1.26 (m, 9H);
MS m/z (ESI):558.2 [M+H]⁺.

### Example 121

### Preparation of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methyl methanesulfonamide

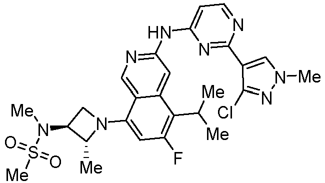

N-((2R,3S)-1-(3-((2-(3-Chloro-1-methyl-1H-pyrazol-4-yl)pyrimidin-4-yl)amino)-6-fluoro-5-isopropylisoquinolin-8-yl)-2-methylazetidin-3-yl)-N-methyl methanesulfonamide was prepared with reference to example 67.
or prepared as follows:

### Step 1 Synthesis of N-((2R,3S)-1-(3-chloro-6-fluoro-5-isopropylisoquinolin-8-yl)-2-methylazetidin-3-yl)-N-methyl methanesulfonamide

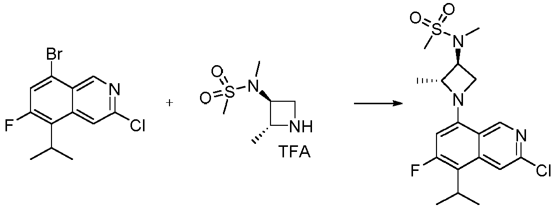

8-Bromo-3-chloro-6-fluoro-5-isopropylisoquinoline (500 mg, 1.65 mmol) and N-methyl-N-((2R,3S)-2-methylazetidin-3-yl)methanesulfonamide trifluoroacetate (470 mg, 1.70 mmol) were mixed in dioxane (10 mL), cesium carbonate (1.15 g, 3.54 mmol) and Xantphos Pd G4 (164 mg, 0.17 mmol) were added, and the mixture was warmed to 100°C and stirred for 5 hours. The reaction solution was cooled to room temperature, and concentrated under reduced pressure. The residue was subjected to liquid separation with dichloromethane and water, and the organic phase was dried over anhydrous sodium sulfate and filtered. The filtrate was concentrated under reduced pressure and separated by column chromatography to obtain the target compound N-((2R,3S)-1-(3-chloro-6-fluoro-5-isopropylisoquinolin-8-yl)-2-methylazetidin-3-yl)-N-methyl methanesulfonamide (330 mg, 50.0%).
¹H NMR(400 MHz, DMSO-*d₆*) *δ* 9.08 (s, 1H), 8.07 (s, 1H), 6.68-6.65 (m, 1H), 4.76-4.69 (m, 1H), 4.59-4.55 (m, 1H), 4.26-4.22 (m, 1H), 3.98-3.96 (m, 1H), 3.67-3.59 (m, 1H), 2.95 (s, 3H), 2.83 (s, 3H), 1.40 (d, *J* = 6.0 Hz, 3H), 1.37-1.31 (m, 6H) ;
MS m/z (ESI) :400.1 [M+H]⁺.

### Step 2 Synthesis of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methyl methanesulfonamide

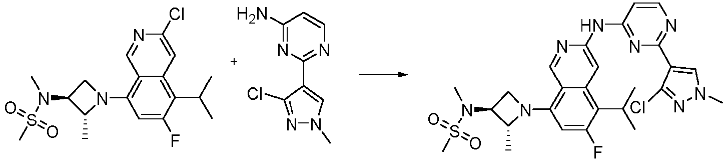

N-((2R,3S)-1-(3-Chloro-6-fluoro-5-isopropylisoquinolin-8-yl)-2-methylazetidin-3-yl)-N-methyl methanesulfonamide (100 mg, 0.25 mmol) and 2-(3-chloro-1-methyl-1H-pyrazol-4-yl)pyrimidin-4-amine (55 mg, 0.25 mmol) were dissolved in dioxane (5 mL), cesium carbonate (274 mg, 0.84 mmol) and BrettPhos Pd G4 (24 mg, 26 µmol) were added, and the mixture was warmed to 100°C and stirred for 12 hours. The reaction solution was cooled to room temperature, and concentrated under reduced pressure. The residue was subjected to liquid separation with dichloromethane and water, and the organic phase was dried over anhydrous sodium sulfate and filtered. The filtrate was concentrated under reduced pressure and separated by column chromatography to obtain the target compound N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methyl methanesulfonamide (73 mg, 50.9%).
¹H NMR (400 MHz, DMSO-*d₆*) *δ* 10.21 (s, 1H), 9.05 (s, 1H), 8.60 (s, 1H), 8.42 (d, *J* = 5.8 Hz, 1H), 8.30 (s, 1H), 7.28 (d, *J* = 6.0 Hz, 1H), 6.47-6.43 (m, 1H), 4.71 -4.69(m, 1H), 4.53-4.49 (m, 1H), 4.23-4.18 (m, 1H), 3.94-3.91 (m, 1H), 3.89 (s, 3H), 3.63 -3.58 (m, 1H), 2.95 (s, 3H), 2.84 (s, 3H), 1.40 (d, *J* = 6.0 Hz, 6H), 1.36-1.32 (m, 3H);
MS m/z (ESI):573.2 [M+H]⁺.

### Example 122

### Preparation of N-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)-N-methyl methanesulfonamide

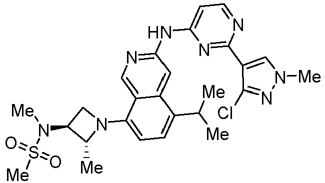

N-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)-N-methyl methanesulfonamide was prepared with reference to example 67 or 121.
¹H NMR (400 MHz, DMSO-*d*₆) *δ* 10.21 (s, 1H), 9.12 (s, 1H), 8.61 (s, 1H), 8.41 (d, *J* = 5.8 Hz, 1H), 8.34 (s, 1H), 7.46 (d, *J* = 8.0 Hz, 1H), 7.26 (d, *J* = 5.6 Hz, 1H), 6.67 (d, *J* = 8.0 Hz, 1H), 4.66-4.63 (m, 1H), 4.45-4.41 (m, 1H), 4.21-4.18 (m, 1H), 3.91 (s, 3H), 3.82-3.79 (m, 1H), 3.59-3.55 (m, 1H), 2.95 (s, 3H), 2.83 (s, 3H), 1.41 (d, *J* = 6.0 Hz, 3H), 1.33-1.31 (m, 6H);
MS m/z (ESI):555.2 [M+H]⁺.

### Example 123

### Preparation of N-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)methanesulfonamide

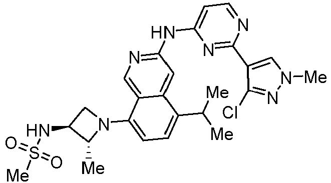

N-((2R,3S)-1-(3-((2-(3-Chloro-1-methyl-1H-pyrazol-4-yl)pyrimidin-4-yl)amino)-5-isopropylisoquinolin-8-yl)-2-methylazetidin-3-yl)methanesulfonamide was prepared with reference to example 44.
¹H NMR (400 MHz, DMSO-*d₆*) *δ*10.14 (s, 1H), 9.03 (s, 1H), 8.54 (s, 1H), 8.34 (d, *J* = 5.8 Hz, 1H), 8.27 (s, 1H), 7.70 (d, *J* = 8.0 Hz, 1H), 7.38 (d, *J* = 8.0 Hz, 1H), 7.18 (d, *J* = 5.8 Hz, 1H), 6.57 (d,*J* = 8.0 Hz, 1H), 4.73-4.71 (m, 1H), 4.08-4.06 (m, 1H), 3.89-3.87 (m, 1H), 3.84 (s, 3H), 3.62 -3.59 (m, 2H), 2.89 (s, 3H), 1.36 (d, *J* = 6.0 Hz, 3H), 1.24-1.22 (m, 6H);
MS m/z (ESI):541.2 [M+H]⁺.

### Example 124

### Preparation of N-(1-(3-((2-(3-chloro-1-methyl-1H-pyrazol-4-yl)pyrimidin-4-yl)amino)-6-fluoro-5-isopropylisoquinolin-8-yl)azetidin-3-yl)-N-methyl methanesulfonamide

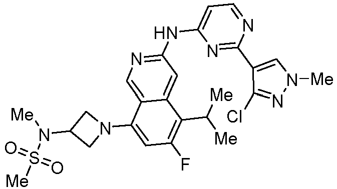

N-(1-(3-((2-(3-Chloro-1-methyl-1H-pyrazol-4-yl)pyrimidin-4-yl)amino)-6-fluoro-5-isopropylisoquinolin-8-yl)azetidin-3-yl)-N-methyl methanesulfonamide was prepared with reference to example 67 or 120.
¹H NMR (400 MHz, DMSO-*d₆*) *δ* 10.18 (s,1H), 9.06 (s, 1H), 8.56 (s, 1H), 8.42 (d, *J* = 5.8 Hz, 1H), 8.30 (s, 1H), 7.31 (d, *J* = 5.8 Hz, 1H), 6.28-6.25 (m, 1H), 4.70 -4.68(m, 1H), 4.46-4.43 (m, 2H), 4.32-4.29 (m, 2H), 3.89 (s, 3H), 3.59-3.56 (m, 1H), 2.95-2.93 (m, 6H), 1.38-1.36 (m, 6H);
MS m/z (ESI):559.2 [M+H]⁺.

### Example 125

### Preparation of N-(2-(1,3-dimethyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine

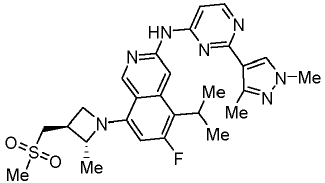

N-(2-(1,3-Dimethyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine was prepared with reference to example 5 or 120.
¹H NMR (400 MHz, DMSO-*d*₆) *δ* 10.04 (s, 1H), 8.96 (s, 1H), 8.47 (s, 1H), 8.31 (d, *J* = 5.8 Hz, 1H), 8.07 (s, 1H), 7.17-7.09 (m, 1H), 6.31-6.23 (m, 1H), 4.66 (t, *J* = 7.6 Hz, 1H), 4.18 (t, *J* = 6.2 Hz, 1H), 3.77 (s, 3H), 3.68 (t, *J* = 7.2 Hz, 1H), 3.56-3.44 (m, 3H), 2.93 (s, 3H), 2.86-2.77 (m, 1H), 2.44 (s, 3H), 1.39-1.31 (m, 9H);
MS m/z (ESI):538.2 [M+H]⁺.

### Example 126

### Preparation of N-((2R,3S)-1-(3-((2-(1,3-dimethyl-1H-pyrazol-4-yl)pyrimidin-4-yl)amino)-5-isopropylisoquinolin-8-yl)-2-methylazetidin-3-yl)-N-methyl methanesulfonamide

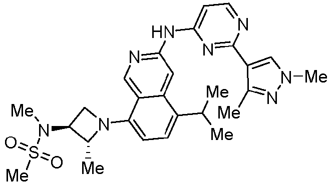

N-((2R,3S)-1-(3-((2-(1,3-Dimethyl-1H-pyrazol-4-yl)pyrimidin-4-yl)amino)-5-isopropylisoquinolin-8-yl)-2-methylazetidin-3-yl)-N-methyl methanesulfonamide was prepared with reference to example 67 or 121.
¹H NMR (400 MHz, DMSO-*d₆*) *δ* 10.11 (s, 1H), 9.12 (s, 1H), 8.58 (s, 1H), 8.37 (d, *J* = 5.8 Hz, 1H), 8.19 (s, 1H), 7.46 (d, *J* = 7.8 Hz, 1H), 7.20 (d, *J* = 5.8 Hz, 1H), 6.67 (d, *J* = 8.0 Hz, 1H), 4.66-4.63 (m, 1H), 4.44-4.42 (m, 1H), 4.21-4.19 (m, 1H), 3.85 (s, 3H), 3.82-3.79 (m, 1H), 3.58-3.56 (m, 1H), 2.96 (s, 3H), 2.83 (s, 3H), 2.52(s, 3H), 1.41 (d, *J* = 6.0 Hz, 3H), 1.34-1.31 (m, 6H);
MS m/z (ESI):535.2 [M+H]⁺.

### Example 127

### Preparation of N-((2R,3S)-1-(3-((2-(1,3-dimethyl-1H-pyrazol-4-yl)pyrimidin-4-yl)amino)-6-fluoro-5-isopropylisoquinolin-8-yl)-2-methylazetidin-3-yl)-N-methyl methanesulfonamide

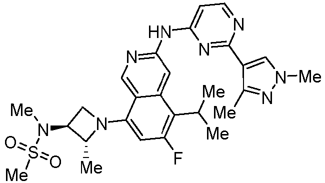

N-((2R,3S)-1-(3-((2-(1,3-Dimethyl-1H-pyrazol-4-yl)pyrimidin-4-yl)amino)-6-fluoro-5-isopropylisoquinolin-8-yl)-2-methylazetidin-3-yl)-N-methyl methanesulfonamide was prepared with reference to example 67 or 121.
¹H NMR (400 MHz, DMSO-*d*₆) *δ* 10.12 (s, 1H), 9.05 (s, 1H), 8.55 (s, 1H), 8.38 (d, *J* = 5.8 Hz, 1H), 8.14 (s, 1H), 7.22 (d, *J* = 5.6 Hz, 1H), 6.47 (d, *J* = 14.3 Hz, 1H), 4.75-4.67 (m, 1H), 4.57-4.48 (m, 1H), 4.27-4.19 (m, 1H), 3.97-3.91 (m, 1H), 3.84 (s, 3H), 3.64-3.52 (m, 1H), 2.95 (s, 3H), 2.84 (s, 3H), 2.51 (s, 3H), 1.46-1.37 (m, 9H);
MS m/z (ESI):553.2 [M+H]⁺.

### Example 128

### Preparation of N-((2R,3S)-1-(3-((2-(1,3-dimethyl-1H-pyrazol-4-yl)pyrimidin-4-yl)amino)-5-isopropylisoquinolin-8-yl)-2-methylazetidin-3-yl)methanesulfonamide

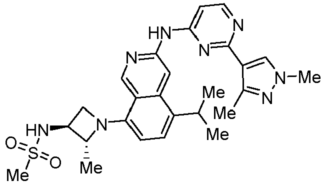

N-((2R,3S)-1-(3-((2-(1,3-Dimethyl-1H-pyrazol-4-yl)pyrimidin-4-yl)amino)-5-isopropylisoquinolin-8-yl)-2-methylazetidin-3-yl)methanesulfonamide was prepared with reference to example 44.

MS m/z (ESI):521.2 [M+H]⁺.

### Example 129

### Preparation of N-(1-(3-((2-(1,3-dimethyl-1H-pyrazol-4-yl)pyrimidin-4-yl)amino)-6-fluoro-5-isopropylisoquinolin-8-yl)azetidin-3-yl)-N-methyl methanesulfonamide

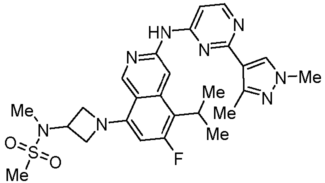

N-(1-(3-((2-(1,3-Dimethyl-1H-pyrazol-4-yl)pyrimidin-4-yl)amino)-6-fluoro-5-isopropylisoquinolin-8-yl)azetidin-3-yl)-N-methyl methanesulfonamide was prepared with reference to example 67 or 120.
¹H NMR (400 MHz, DMSO-*d*₆) *δ* 10.00 (s, 1H), 9.00 (s, 1H), 8.43 (s, 1H), 8.32 (d, *J* = 5.8 Hz, 1H), 8.07 (s, 1H), 7.21-7.17 (m, 1H), 6.23-6.15 (m, 1H), 4.67-4.60 (m, 1H), 4.39 (t, *J* = 8.2 Hz, 2H), 4.29-4.22 (m, 2H), 3.77 (s, 3H), 3.55-3.47 (m, 1H), 2.91-2.84 (m, 6H), 2.44 (s, 3H), 1.34 (d, *J* = 6.6 Hz, 6H);
MS m/z (ESI):539.2 [M+H]⁺.

### Example 130

### Preparation of N-(1-(6-((2-(3-chloro-1-methyl-1H-pyrazol-4-yl)pyrimidin-4-yl)amino)-4-isopropyl-2,7-diazanaphthalen-1-yl)azetidin-3-yl)-N-methyl methanesulfonamide

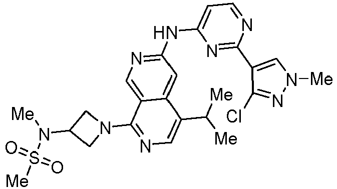

N-(1-(6-((2-(3-Chloro-1-methyl-1H-pyrazol-4-yl)pyrimidin-4-yl)amino)-4-isopropyl-2,7-diazanaphthalen-1-yl)azetidin-3-yl)-N-methyl methanesulfonamide was prepared with reference to example 67.
¹H NMR (400 MHz, DMSO-d₆) *δ* 10.37 (s, 1H), 9.12 (s, 1H), 8.48-8.46 (m, 2H), 8.34 (s, 1H), 8.00 (s, 1H), 7.35 (d, *J* = 8.0 Hz, 1H), 4.74-4.49 (m, 5H), 3.91 (s, 3H), 3.45-3.40 (m, 1H), 2.95 (s, 6H), 1.31 (d, *J* = 7.6Hz, 6H);
MS m/z (ESI):542.2 [M+H]⁺.

### Example 131

### Preparation of N-(1-(7-((2-(3-chloro-1-methyl-1H-pyrazol-4-yl)pyrimidin-4-yl)amino)-1-isopropyl-2,6-diazanaphthalen-4-yl)azetidin-3-yl)-N-methyl methanesulfonamide

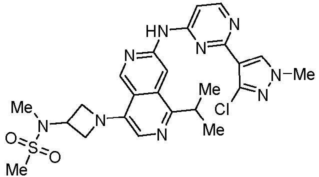

N-(1-(7-((2-(3-Chloro-1-methyl-1H-pyrazol-4-yl)pyrimidin-4-yl)amino)-1-isopropyl-2,6-diazanaphthalen-4-yl)azetidin-3-yl)-N-methyl methanesulfonamide was prepared with reference to example 67.
¹H NMR (400 MHz, DMSO-*d*₆) *δ* 10.38 (s, 1H), 9.21 (s, 1H), 8.73 (s, 1H), 8.44 (d, *J* = 5.8 Hz, 1H), 8.37 (s, 1H), 7.65 (s, 1H), 7.23 (d, *J* = 5.8 Hz, 1H), 4.73 (t, *J* = 7.0 Hz, 1H), 4.55-4.48 (m, 2H), 4.39-4.33 (m, 2H), 3.91 (s, 3H), 3.84-3.77 (m, 1H), 2.96 (s, 6H), 1.31 (d, *J* = 6.8 Hz, 6H);
MS m/z (ESI):542.2 [M+H]⁺.

### Example 132

### Preparation of N-(2-(3-chloro-1-methyl-1H-pyrazol-4-yl)pyrimidin-4-yl)-5-isopropyl-8-((2R,3S)-2-methyl-3-((methanesulfonyl)methyl)azetidin-1-yl)-2,7-diazanaphthalen-3-amine

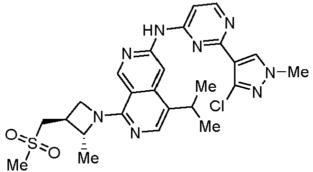

N-(2-(3-Chloro-1-methyl-1H-pyrazol-4-yl)pyrimidin-4-yl)-5-isopropyl-8-((2R,3S)-2-methyl-3-((methanesulfonyl)methyl)azetidin-1-yl)-2,7-diazanaphthalen-3-amine was prepared with reference to example 5.
¹H NMR (400 MHz, DMSO-d₆) *δ* 10.39 (s, 1H), 9.09 (s, 1H), 8.48-8.45 (m, 2H), 8.34 (s, 1H), 8.01 (s, 1H), 7.32 (d, J = 8.0 Hz, 1H), 4.88-4.86 (m, 1H), 4.58-4.55 (m, 1H), 4.02-3.98 (m,1H), 3.91 (s, 3H), 3.57-3.54 (m, 2H), 3.44-3.36 (m,1H), 3.00 (s, 3H), 2.91-2.88 (m, 1H), 1.50 (d, J = 7.6 Hz, 3H), 1.31 (d, *J* = 7.6Hz, 6H);
MS m/z (ESI):541.2 [M+H]⁺.

### Example 133

### Preparation of N-(2-(3-chloro-1-methyl-1H-pyrazol-4-yl)pyrimidin-4-yl)-5-isopropyl-8-((2R,3S)-2-methyl-3-((methanesulfonyl)methyl)azetidin-1-yl)-2,6-diazanaphthalen-3-amine

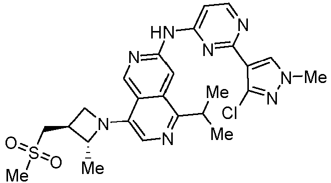

N-(2-(3-Chloro-1-methyl-1H-pyrazol-4-yl)pyrimidin-4-yl)-5-isopropyl-8-((2R,3S)-2-methyl-3-((methanesulfonyl)methyl)azetidin-1-yl)-2,6-diazanaphthalen-3-amine was prepared with reference to example 5.
¹H NMR (400 MHz, DMSO-*d*₆) *δ* 10.42 (s, 1H), 9.17 (s, 1H), 8.75 (s, 1H), 8.44 (d, *J* = 5.8 Hz, 1H), 8.37 (s, 1H), 7.75 (s, 1H), 7.224-7.15 (m, 1H), 4.80-4.73 (m, 1H), 4.36-4.30 (m, 1H), 3.91 (s, 3H), 3.85-3.76 (m, 2H), 3.60-3.55 (m, 2H), 3.01 (s, 3H), 2.95-2.91 (m, 1H), 1.49 (d, *J* = 6.2 Hz, 3H), 1.31 (d, *J* = 6.6 Hz, 6H);
MS m/z (ESI):541.2 [M+H]⁺.

### Example 134

### Preparation of N-(1-(3-((2-(3-chloro-1-methyl-1H-pyrazol-4-yl)pyrimidin-4-yl)amino)-5-(dimethylamino)-6-fluoroisoquinolin-8-yl)azetidin-3-yl)-N-methyl methanesulfonamide

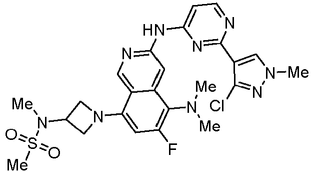

N-(1-(3-((2-(3-Chloro-1-methyl-1H-pyrazol-4-yl)pyrimidin-4-yl)amino)-5-(dimethylamino)-6-fluoroisoquinolin-8-yl)azetidin-3-yl)-N-methyl methanesulfonamide was prepared with reference to example 67 or 120.

MS m/z (ESI):560.2 [M+H]⁺.

### Example 135

### Preparation of (R)-N-(1-(3-((2-(3-chloro-1-methyl-1H-pyrazol-4-yl)pyrimidin-4-yl)amino)-6-fluoro-5-(1-methoxyethyl)isoquinolin-8-yl)azetidin-3-yl)-N-methyl methanesulfonamide

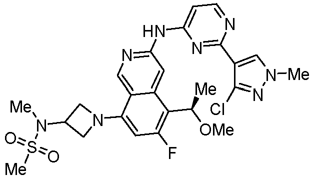

(R)-N-(1-(3-((2-(3-Chloro-1-methyl-1H-pyrazol-4-yl)pyrimidin-4-yl)amino)-6-fluoro-5-(1-methoxyethyl)isoquinolin-8-yl)azetidin-3-yl)-N-methyl methanesulfonamide was prepared with reference to example 67 or 120.

MS m/z (ESI):575.2 [M+H]⁺.

### Example 136

### Preparation of (S)-N-(1-(3-((2-(3-chloro-1-methyl-1H-pyrazol-4-yl)pyrimidin-4-yl)amino)-6-fluoro-5-(1-methoxyethyl)isoquinolin-8-yl)azetidin-3-yl)-N-methyl methanesulfonamide

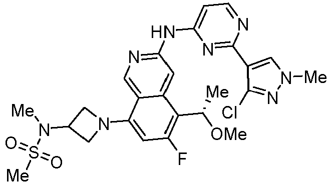

(S)-N-(1-(3-((2-(3-Chloro-1-methyl-1H-pyrazol-4-yl)pyrimidin-4-yl)amino)-6-fluoro-5-(1-methoxyethyl)isoquinolin-8-yl)azetidin-3-yl)-N-methyl methanesulfonamide was prepared with reference to example 67 or 120.

MS m/z (ESI):575.2 [M+H]⁺.

### Example 137

### Preparation of N3-(2-(3-chloro-1-methyl-1H-pyrazol-4-yl)pyrimidin-4-yl)-6-fluoro-N5,N5-dimethyl-8-((2R,3S)-2-methyl-3-((methanesulfonyl)methyl)azetidin-1-yl)isoquinolin-3,5-diamine

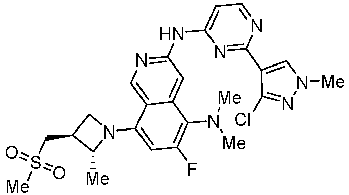

N3-(2-(3-Chloro-1-methyl-1H-pyrazol-4-yl)pyrimidin-4-yl)-6-fluoro-N5,N5-dimethyl-8-((2R,3S)-2-methyl-3-((methanesulfonyl)methyl)azetidin-1-yl)isoquinoline -3,5-diamine was prepared with reference to example 5 or 120.

MS m/z (ESI):559.2 [M+H]⁺.

### Example 138

### Preparation of N-(2-(3-chloro-1-methyl-1H-pyrazol-4-yl)pyrimidin-4-yl)-6-fluoro-5-((R)-1-methoxyethyl)-8-((2R,3S)-2-methyl-3-((methanesulfonyl)methyl)azetidin-1-yl)isoquinolin-3-amine

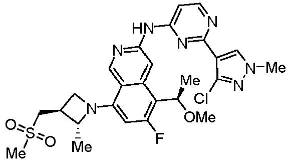

N-(2-(3-Chloro-1-methyl-1H-pyrazol-4-yl)pyrimidin-4-yl)-6-fluoro-5-((R)-1-methoxyethyl)-8-((2R,3S)-2-methyl-3-((methanesulfonyl)methyl)azetidin-1-yl)isoquinolin-3-amine was prepared with reference to example 5 or 120.

MS m/z (ESI):574.2 [M+H]⁺.

### Example 139

### Preparation of N-(2-(3-chloro-1-methyl-1H-pyrazol-4-yl)pyrimidin-4-yl)-6-fluoro-5-((S)-1-methoxyethyl)-8-((2R,3S)-2-methyl-3-((methanesulfonyl)methyl)azetidin-1-yl)isoquinolin-3-amine

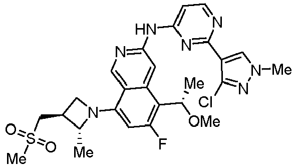

N-(2-(3-Chloro-1-methyl-1H-pyrazol-4-yl)pyrimidin-4-yl)-6-fluoro-5-((S)-1-methoxyethyl)-8-((2R,3S)-2-methyl-3-((methanesulfonyl)methyl)azetidin-1-yl)isoquinolin-3-amine was prepared with reference to example 5 or 120.

MS m/z (ESI):574.2 [M+H]⁺.

### Example 140

### Preparation of N-(2-(3-chloro-1-(2-methyltetrahydro-2H-pyran-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

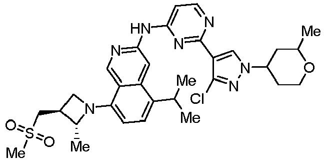

N-(2-(3-Chloro-1-(2-methyltetrahydro-2H-pyran-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.11 (s, 1H), 9.03 (s, 1H), 8.46-8.32 (m, 3H), 7.35 (d, *J* = 8.0 Hz, 1H), 7.24 (s, 1H), 6.50 (d, *J* = 8.1 Hz, 1H), 4.62-4.59 (m, 1H), 4.17-4.11 (m, 1H), 3.80-3.41 (m, 7H), 2.93 (s, 3H), 2.85-2.78 (m, 1H), 2.27-1.90 (m, 4H), 1.76-1.68 (m, 1H), 1.36 (d, *J* = 6.1 Hz, 3H), 1.23-1.18 (m, 6H), 1.09 (d, *J* = 6.4 Hz, 3H);
MS m/z (ESI):624.2 [M+H]⁺.

### Example 141

### Preparation of N-(2-(3-chloro-1-(2-oxaspiro[3.3]heptan-6-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

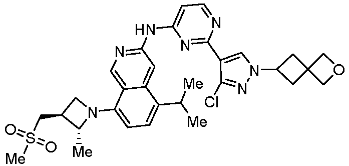

N-(2-(3-Chloro-1-(2-oxaspiro[3.3]heptan-6-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.18 (s, 1H), 9.09 (s, 1H), 8.63-8.39 (m, 3H), 7.43 (d, *J* = 8.0 Hz, 1H), 7.31-7.23 (m, 1H), 6.71-6.65(m, 1H), 4.88-4.51 (m, 5H), 4.25-4.20 (m, 1H), 3.61-3.50 (m, 2H), 3.00 (s, 3H), 2.90-2.60 (m, 4H), 2.14-2.00 (m, 4H), 1.44-1.21 (m, 9H);
MS m/z (ESI):622.2 [M+H]⁺.

### Example 142

### Preparation of N-(2-(3-chloro-1-((S)-tetrahydrofuran-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

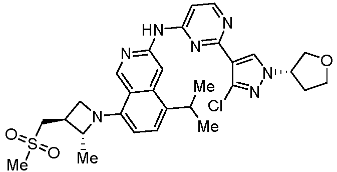

N-(2-(3-Chloro-1-((S)-tetrahydrofuran-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.13 (s, 1H), 9.10 (s, 1H), 8.47-8.40 (m, 3H), 7.43 (d,*J* = 8.0 Hz, 1H), 7.33-7.26 (m ,1H), 6.58 (d, *J* = 8.0 Hz, 1H), 5.35-5.30 (m, 1H), 5.10-5.06 (m, 1H), 4.65-4.60 (m, 1H), 4.22-4.19 (m, 1H), 4.01-3.95 (m, 3H), 3.86-3.82 (m, 1H), 3.65-3.45 (m, 2H), 2.99 (s, 3H), 2.03-1.97 (m, 4H), 1.31-1.27 (m, 9H);
MS m/z (ESI):596.2 [M+H]⁺.

### Example 143

### Preparation of N-(2-(3-chloro-1-((R)-tetrahydrofuran-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

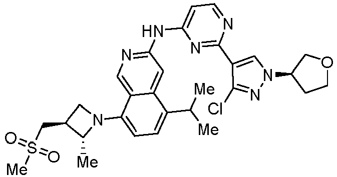

N-(2-(3-Chloro-1-((R)-tetrahydrofuran-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.19 (s, 1H), 9.10 (s, 1H), 8.47-8.40 (m, 3H), 7.43 (d, *J* = 7.2 Hz, 1H), 7.30 (br s, 1H), 6.56 (d, *J* = 7.6 Hz, 1H), 5.09-5.06 (m, 1H), 4.70- 4.65 (m, 1H), 4.23-4.18 (m, 1H), 4.03-3.83 (m, 4H), 3.86-3.80 (m, 1H), 3.65-3.52 (m, 2H), 2.99 (s, 3H), 2.91-2.88 (m, 1H), 2.35-2.30 (m, 1H), 2.03-1.95 (m, 2H), 1.37-1.16 (m, 9H);
MS m/z (ESI):596.2 [M+H]⁺.

### Example 144

### Preparation of N-(2-(3-chloro-1-(((R)-tetrahydrofuran-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

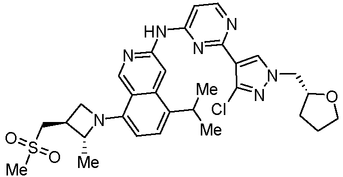

N-(2-(3-Chloro-1-(((R)-tetrahydrofuran-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.12 (s, 1H), 9.03 (s, 1H), 8.44 (s, 1H), 8.34 (d, *J* = 5.8 Hz, 1H), 8.25 (s, 1H), 7.36 (d, *J* = 8.0 Hz, 1H), 7.24 (s, 1H), 6.51 (d, *J* = 8.2 Hz, 1H), 4.61 (t, *J* = 7.5 Hz, 1H), 4.23-4.03 (m, 4H), 3.73-3.63 (m, 1H), 3.63-3.39 (m, 5H), 2.93 (s, 3H), 2.82 (q, *J* = 7.3 Hz, 1H), 1.98-1.86 (m, 1H), 1.83-1.64 (m, 2H), 1.62-1.49 (m, 1H), 1.36 (d, *J* = 6.0 Hz, 3H), 1.23 (dd, *J* = 9.2, 6.8 Hz, 6H);
MS m/z (ESI):610.2 [M+H]⁺.

### Example 145

### Preparation of N-(2-(3-chloro-1-(((S)-tetrahydrofuran-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

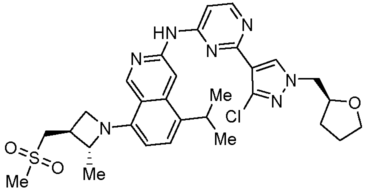

N-(2-(3-Chloro-1-(((S)-tetrahydrofuran-2-yl)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.
¹H NMR (400 MHz, DMSO-*d*₆) *δ* 10.19 (s, 1H), 9.10 (s, 1H), 8.51 (s, 1H), 8.41 (d, J = 5.8 Hz, 1H), 8.32 (s, 1H), 7.43 (d, *J* = 8.0 Hz, 1H), 7.33-7.28 (m, 1H), 6.58 (d, *J* = 8.1 Hz, 1H), 4.68 (t, *J* = 7.5 Hz, 1H), 4.31-4.10 (m, 4H), 3.82-3.70 (m, 1H), 3.68-3.61 (m, 2H), 3.61-3.50 (m, 3H), 3.00 (s, 3H), 2.90 (q, *J* = 7.3 Hz, 1H), 2.05-1.93 (m, 1H), 1.88-1.74 (m, 2H), 1.69-1.56 (m, 1H), 1.43 (d, *J* = 6.0 Hz, 3H), 1.35-1.21 (m, 6H);
MS m/z (ESI):610.2 [M+H]⁺.

### Example 146

### Preparation of N-(2-(3-chloro-1-(((R)-1-methylazetidin-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

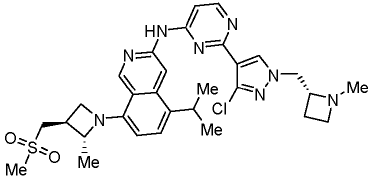

N-(2-(3-Chloro-1-(((R)-1-methylazetidin-2-yl)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.
¹H NMR (400 MHz, DMSO-*d*₆) *δ* 10.20 (s, 1H), 9.10 (s, 1H), 8.54 (s, 1H), 8.41 (d, *J* = 6.0 Hz, 1H), 8.33 (s, 1H), 7.44 (d, *J* = 8.0 Hz, 1H), 7.32-7.26 (m, 1H), 6.58 (d, *J* = 8.0 Hz, 1H), 4.72-4.66 (m, 1H), 4.23-4.15 (m, 3H), 3.67-3.61 (m, 1H), 3.59-3.50 (m, 4H), 3.00 (s, 3H), 2.93-2.87 (m, 1H), 2.73-2.66 (m, 1H), 2.03 (s, 3H), 2.01-1.97 (m, 2H), 1.86-1.80 (m, 1H), 1.43 (d, *J* = 6.4 Hz, 3H), 1.31 (d, *J* = 6.8 Hz, 6H);
MS m/z (ESI):609.2 [M+H]⁺.

### Example 147

### Preparation of N-(2-(3-chloro-1-(((S)-1-methylazetidin-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

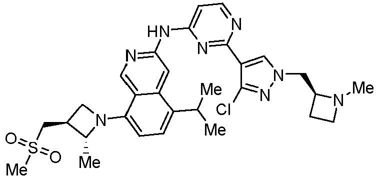

N-(2-(3-Chloro-1-(((S)-1-methylazetidin-2-yl)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.
¹H NMR (400 MHz, DMSO-*d*₆) *δ* 10.13 (s, 1H), 9.03 (s, 1H), 8.47 (s, 1H), 8.34 (d, *J* = 6.0 Hz, 1H), 8.27 (s, 1H), 7.37 (d, *J* = 8.0 Hz, 1H), 7.22 (s, 1H), 6.51 (d, *J* = 8.0 Hz, 1H), 4.64-4.59 (m, 1H), 4.16-4.09 (m, 3H), 3.61-3.55 (m, 1H), 3.52-3.44 (m, 4H), 2.93 (s, 3H), 2.86-2.80 (m, 1H), 2.66-2.61 (m, 1H), 1.96 (s, 3H), 1.93-1.88 (m, 2H), 1.79-1.73 (m, 1H), 1.36 (d, *J* = 6.0 Hz, 3H), 1.24 (d, J = 6.8 Hz, 6H);
MS m/z (ESI):609.2 [M+H]⁺.

### Example 148

### Preparation of N-(2-(3-chloro-1-(((R)-1-methylpyrrolidin-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

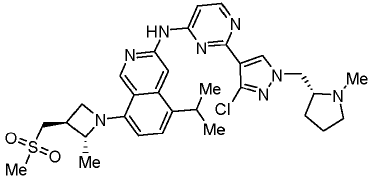

N-(2-(3-Chloro-1-(((R)-1-methylpyrrolidin-2-yl)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.
¹H NMR (400 MHz, DMSO-d₆) *δ* 10.19 (s, 1H), 9.10 (s, 1H), 8.57-8.20 (m, 3H), 7.43 (d, *J* = 7.2 Hz, 1H), 7.31 (br s, 1H), 6.56 (d, *J* = 7.2 Hz, 1H), 4.70-4.65 (m, 1H), 4.23-4.02 (m, 3H), 3.73-3.53 (m, 5H), 2.99-2.80 (m, 5H), 2.35-2.20 (m, 4H), 2.03-1.95 (m, 2H), 1.75-1.17 (m, 11H);
MS m/z (ESI):623.3 [M+H]⁺.

### Example 149

### Preparation of N-(2-(3-chloro-1-(((S)-1-methylpyrrolidin-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

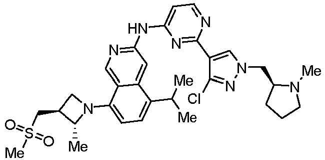

N-(2-(3-Chloro-1-(((S)-1-methylpyrrolidin-2-yl)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.

MS m/z (ESI):623.3 [M+H]⁺.

### Example 150

### Preparation of N-(2-(3-chloro-1-(2-methyloctahydrocyclopentadieno[c]pyrrol-5-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

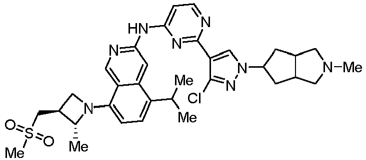

N-(2-(3-Chloro-1-(2-methyloctahydrocyclopentadieno[c]pyrrol-5-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.

MS m/z (ESI):649.3 [M+H]⁺.

### Example 151A

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutane-1-carbonitrile

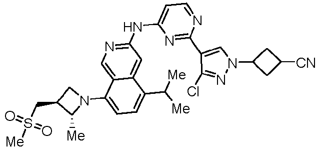

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)cyclobutane-1-carbonitrile was prepared with reference to reference example 2.
¹H NMR (400 MHz, DMSO-*d₆*) *δ* 10.13 (s, 1H), 9.03 (s, 1H), 8.46 (s, 1H), 8.42 (s, 1H), 8.35 (d, *J* = 5.8 Hz, 1H), 7.36 (d, *J* = 8.0 Hz, 1H), 7.22 (s, 1H), 6.51 (d, *J* = 8.0 Hz, 1H), 4.90-4.84 (m, 1H), 4.63-4.59 (m, 1H), 4.16-4.12 (m, 1H), 3.60-3.55 (m, 1H), 3.49-3.43 (m, 4H), 2.93 (s, 3H), 2.84-2.76 (m, 5H), 1.36 (d, *J* = 6.0 Hz, 3H), 1.25-1.20 (m, 6H);
MS m/z (ESI):605.2 [M+H]⁺.

### Example 151

### Preparation of (1r,3r)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutane-1-carbonitrile

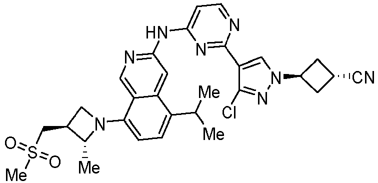

(1r,3r)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutane-1-carbonitrile was prepared with reference to reference example 2.

MS m/z (ESI):605.2 [M+H]⁺.

### Example 152

### Preparation of (1s,3s)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutane-1-carbonitrile

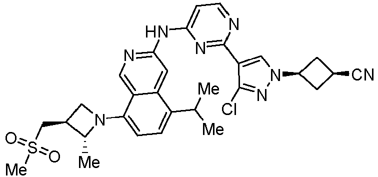

(1s,3s)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutane-1-carbonitrile was prepared with reference to reference example 2.

MS m/z (ESI):605.2 [M+H]⁺.

### Example 153

### Preparation of N-(2-(3-chloro-1-(3-methoxy cyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

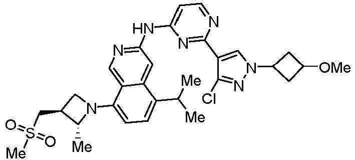

N-(2-(3-Chloro-1-(3-methoxy cyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.20 (s, 1H), 9.09 (s, 1H), 8.57 (s, 1H), 8.44-8.39 (m, 2H), 7.43 (d, *J* = 7.8 Hz, 1H), 7.27 (s, 1H), 6.58 (d, *J* = 8.0 Hz, 1H), 5.01 (t, *J* = 7.0 Hz, 1H), 4.68 (t,*J* = 7.2 Hz, 1H), 4.24-4.15 (m, 1H), 3.64 (t, *J* = 7.2 Hz, 1H), 3.59-3.51 (m, 2H), 3.21 (s, 3H), 3.00 (s, 3H), 2.94-2.86 (m, 1H), 2.76-2.65 (m, 2H), 2.05-1.95 (m, 1H), 1.43 (d, *J* = 6.0 Hz, 3H), 1.37-1.20 (m, 9H);
MS m/z (ESI):610.2 [M+H]⁺.

### Example 154A

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-ol

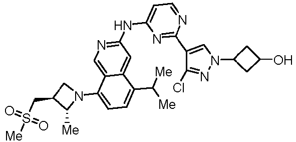

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)cyclobutan-1-ol was prepared with reference to reference example 2.
¹H NMR (400 MHz, DMSO-*d₆*) *δ* 10.11 (s, 1H), 9.03 (s, 1H), 8.46 (s, 1H), 8.34 (d, *J* = 5.8 Hz, 1H), 8.32 (s, 1H), 7.36 (d, *J* = 7.8 Hz, 1H), 7.21 (s, 1H), 6.51 (d, *J* = 8.0 Hz, 1H), 5.27 (d, *J* = 6.8 Hz, 1H), 4.63-4.59 (m, 1H), 4.39- 4.32 (m, 1H), 4.16-4.10 (m, 1H), 3.96-3.88 (m, 1H), 3.58 (t, *J* = 7.2 Hz, 1H), 3.53-3.43 (m, 3H), 2.93 (s, 3H), 2.85 -2.80 (m, 1H), 2.72-2.65 (m, 2H), 2.36-2.28 (m, 2H), 1.36 (d, *J* = 6.0 Hz, 3H), 1.24-1.19 (m, 6H);
MS m/z (ESI):596.2 [M+H]⁺.

### Example 154

### Preparation of (1s,3s)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-ol

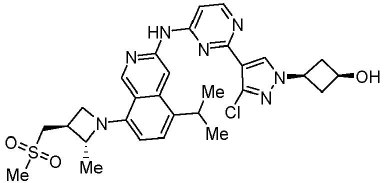

(1s,3s)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-ol was prepared with reference to reference example 2.

MS m/z (ESI):596.2 [M+H]⁺.

### Example 155

### Preparation of (1r,3r)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-ol

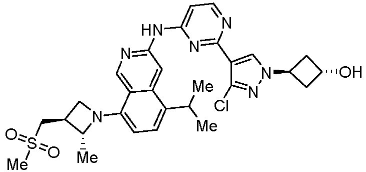

(1r,3r)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-ol was prepared with reference to reference example 2.

MS m/z (ESI):596.2 [M+H]⁺.

### Example 156

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclobutan-1-one

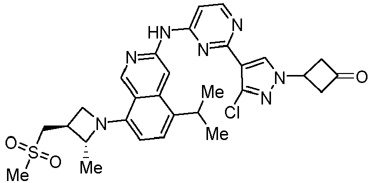

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)cyclobutan-1-one was prepared with reference to reference example 2.
¹H NMR (400 MHz, DMSO-*d*₆) *δ* 10.29 (s, 1H), 9.11 (s, 1H), 8.88 (s, 1H), 8.46 (d, *J* = 5.9 Hz, 1H), 8.19 (d, *J* = 14.2 Hz, 1H), 7.43 (d, *J* = 8.0 Hz, 1H), 6.75 (d, *J* = 14.1 Hz, 1H), 6.58 (d, *J* = 8.1 Hz, 1H), 4.68 (t, *J* = 7.5 Hz, 1H), 4.26-4.15 (m, 1H), 3.65 (t, *J* = 7.2 Hz, 1H), 3.60-3.49 (m, 3H), 3.00 (s, 3H), 2.94-2,85 (m, 1H), 2.33 (s, 3H), 2.05-1.93 (m, 2H), 1.43 (d, *J* = 6.1 Hz, 3H), 1.33-1.13 (m, 6H);
MS m/z (ESI):594.2 [M+H]⁺.

### Example 157

### Preparation of N-(2-(3-chloro-1-(5-methyl-5-azaspiro [3.4]octan-2-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

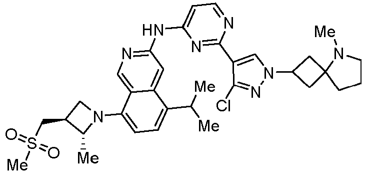

N-(2-(3-Chloro-1-(5-methyl-5-azaspiro [3.4]octan-2-yl)-1H-pyrazol-4-yl) pyrimidin-4-yl)-5-isopropyl-8-((2R,3S)-2-methyl-3-((methanesulfonyl)methyl) azetidin-1-yl)isoquinolin-3-amine was prepared with reference to reference example 2.
¹H NMR (400 MHz, DMSO-*d₆*) *δ* 10.19 (s, 1H), 9.09 (s, 1H), 8.45-8.38 (m, 3H), 7.43 (d, *J* = 8.0 Hz, 1H), 7.27 (s, 1H), 6.59 (d, *J* = 8.0 Hz, 1H), 5.36-5.30 (m, 1H), 4.73-4.67 (m, 1H), 4.223-4.17 (m, 1H), 3.67-3.61 (m, 1H), 3.58-3.52 (m, 4H), 3.00 (s, 3H), 2.94-2.88 (m, 1H), 2.57-2.52 (m, 2H), 2.45-2.40 (m, 2H), 2.21 (s, 3H), 2.04-1.94 (m, 2H), 1.43 (d, *J* = 6.0 Hz, 6H), 1.34-1.26 (m, 6H);
MS m/z (ESI):649.3 [M+H]⁺.

### Example 158

### Preparation of N-(2-(3-chloro-1-(1-methylpiperidin-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

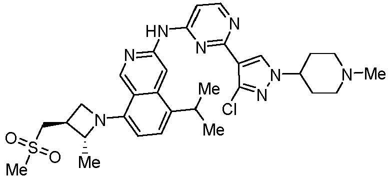

N-(2-(3-Chloro-1-(1-methyl piperidin-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.19 (s, 1H), 9.10 (s, 1H), 8.55-8.40 (m, 3H), 7.43 (d, *J* = 8.0 Hz, 1H), 7.26-7.21 (m ,1H), 6.58 (d, *J* = 8.0 Hz, 1H), 5.35-5.30 (m, 1H), 4.70-4.65 (m, 1H), 4.22-4.17 (m, 2H), 3.66-3.50 (m, 4H), 2.99 (s, 3H), 2.92-2.87 (m, 4H), 2.21 (s, 3H), 2.03-1.95 (m, 4H), 1.33-1.27 (m, 9H);
MS m/z (ESI):623.3 [M+H]⁺.

### Example 159

### Preparation of N-(2-(3-chloro-1-(3,3-difluorocyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

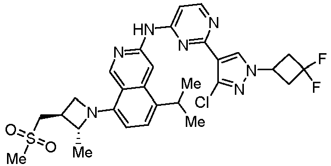

N-(2-(3-Chloro-1-(3,3-difluorocyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.13 (s, 1H), 9.03 (s, 1H), 8.43 (d, *J* = 4.0 Hz, 2H), 8.35 (d, *J* = 5.9 Hz, 1H), 7.36 (d, *J* = 8.0 Hz, 1H), 7.26 (d, *J* = 5.9 Hz, 1H), 6.51 (d, *J* = 8.0 Hz, 1H), 4.99-4.84 (m, 1H), 4.61 (t, *J* = 7.5 Hz, 1H), 4.16-4.09 (m, 1H), 3.58 (t, *J* = 7.2 Hz, 1H), 3.53-3.42 (m, 3H), 3.17-3.10 (m, 4H), 2.93 (s, 3H), 2.82 (q, *J* = 7.2 Hz, 1H), 1.36 (d, *J* = 6.1 Hz, 3H), 1.21 (t, *J* = 4.0 Hz, 6H);
MS m/z (ESI):616.2 [M+H]⁺.

### Example 160

### Preparation of N-(2-(3-chloro-1-(3-(dimethylamino)cyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

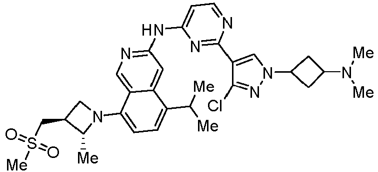

N-(2-(3-Chloro-1-(3-(dimethylamino)cyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.

MS m/z (ESI):623.3 [M+H]⁺.

### Example 161

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methylcyclobutane-1-carbonitrile

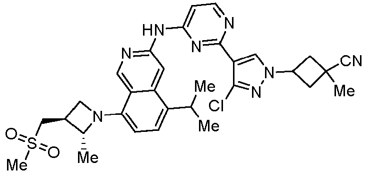

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-1-methylcyclobutane-1-carbonitrile was prepared with reference to reference example 2.
¹H NMR (400 MHz, DMSO-*d₆*) *δ* 10.13 (s, 1H), 9.03 (s, 1H), 8.45 (s, 1H), 8.41 (s, 1H), 8.34 (d, *J* = 6.0 Hz, 1H), 7.38 (s, 1H), 7.24 (s, 1H), 6.51 (d, *J* = 8.0 Hz, 1H), 5.28 -5.24 (m, 1H), 5.11-5.06 (m, 1H), 4.64-4.59 (m, 1H), 4.14 (t, *J* = 6.4 Hz, 1H), 3.60-3.52 (m, 1H), 3.53-3.46 (m, 4H), 2.93 (s, 3H), 2.86-2.78 (m, 1H), 2.66-2.58 (m, 2H), 1.52 (s, 3H), 1.36 (d, *J* = 6.0 Hz, 3H), 1.26-1.19 (m, 6H);
MS m/z (ESI):619.2 [M+H]⁺.

### Example 162

### Preparation of N-(2-(3-chloro-1-(3-(methoxymethyl)cyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

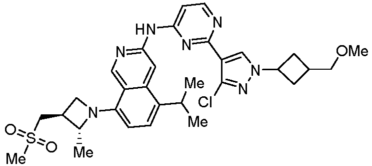

N-(2-(3-Chloro-1-(3-(methoxymethyl)cyclobutyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine was prepared with reference to reference example 2.
¹H NMR (400 MHz, DMSO) *δ* 10.20 (s, 1H), 9.10 (s, 1H), 8.59 (s, 1H), 8.44-8.37 (m, 2H), 7.43 (d, *J* = 8.0 Hz, 1H), 7.26 (d, *J* = 5.0 Hz, 1H), 6.58 (d, *J* = 8.1 Hz, 1H), 4.99 (p, *J* = 7.8 Hz, 1H), 4.68 (t, *J* = 7.5 Hz, 1H), 4.21 (p, *J* = 5.9 Hz, 1H), 3.65 (t, *J* = 7.1 Hz, 1H), 3.55 (qt, *J* = 9.9, 5.1 Hz, 3H), 3.47 (d, *J* = 6.0 Hz, 2H), 3.32 (s, 3H), 3.00 (s, 3H), 2.95-2.81 (m, 1H), 2.68-2.53 (m, 3H), 2.30 (dt,*J* = 15.4, 6.0 Hz, 2H), 1.43 (d, *J* = 6.0 Hz, 3H), 1.29 (dd, *J* = 6.7, 4.1 Hz, 6H);
MS m/z (ESI):624.2 [M+H]⁺.

### Example 163

### Preparation of (1s,3s)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methyl cyclobutan-1-ol

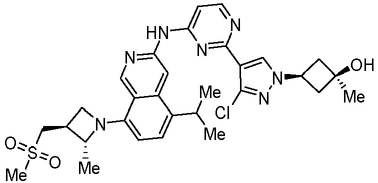

(1s,3s)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methylcyclobutan-1-ol was prepared with reference to reference example 2.

MS m/z (ESI):610.2 [M+H]⁺.

### Example 164

### Preparation of (1r,3r)-3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methylcyclobutan-1-ol

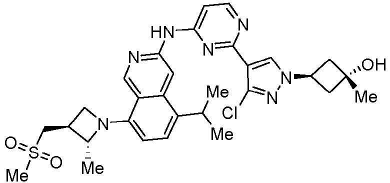

(1r,3r)-3-(3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methylcyclobutan-1-ol was prepared with reference to reference example 2.

MS m/z (ESI):610.2 [M+H]⁺.

### Example 165

### Preparation of N-(2-(3-chloro-1-(oxetan-2-ylmethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

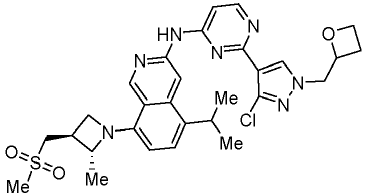

N-(2-(3-Chloro-1-(oxetan-2-ylmethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.20 (s, 1H), 9.10 (s, 1H), 8.51 (s, 1H), 8.42 (d, *J* = 5.8 Hz, 1H), 8.36 (s, 1H), 7.44 (d, *J* = 8.0 Hz, 1H), 7.32 (s, 1H), 6.58 (d, *J* = 8.0 Hz, 1H), 5.33 (t, *J* = 4.8 Hz, 1H), 5.08-5.04 (m, 1H), 4.68 (t, *J* = 7.6 Hz, 1H), 4.57-4.28 (m, 5H), 4.25-4.17 (m, 1H), 3.72-3.44 (m, 4H), 3.00 (s, 3H), 2.95-2.85 (m, 1H), 1.43 (d, *J* = 6.0 Hz, 3H), 1.36-1.28 (m, 6H);
MS m/z (ESI):596.2 [M+H]⁺.

### Example 166

### Preparation of ((1-(3-((2-(1,3-dimethyl-1H-pyrazol-4-yl)pyrimidin-4-yl)amino)-5-isopropylisoquinolin-8-yl)azetidin-3-yl)methyl)(imino)(methyl)-l6-sulfanone

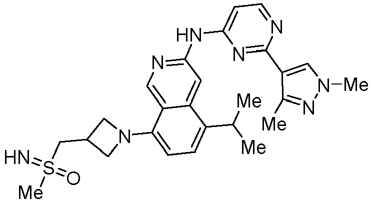

((1-(3-((2-(1,3-Dimethyl-1H-pyrazol-4-yl)pyrimidin-4-yl)amino)-5-isopropylisoquinolin-8-yl)azetidin-3-yl)methyl)(imino)(methyl)-I6-sulfanone was prepared with reference to example 5.
¹H NMR (400 MHz, DMSO-*d*₆) *δ* 10.05 (s, 1H), 9.10 (s, 1H), 8.53 (s, 1H), 8.36 (d, *J* = 5.8 Hz, 1H), 8.18 (s, 1H), 7.43 (d, *J* = 8.0 Hz, 1H), 7.21 (d, *J* = 5.9 Hz, 1H), 6.41 (d, *J* = 8.0 Hz, 1H), 4.42-4.35 (m, 2H), 4.05-3.93 (m, 2H), 3.85 (s, 3H), 3.74 (s, 1H), 3.58-3.46 (m, 3H), 3.31-3.27 (m, 1H), 2.93 (s, 3H), 2.51 (s, 3H), 1.33 (d, *J* = 6.8 Hz, 6H);
MS m/z (ESI):505.2 [M+H]⁺.

### Example 167

### Preparation of N-(((1-(3-((2-(1,3-dimethyl-1H-pyrazol-4-yl)pyrimidin-4-yl)amino)-5-isopropylisoquinolin-8-yl)azetidin-3-yl)methyl)(methyl)(carbonyl)-I6-sulfanylidene)cyanamide

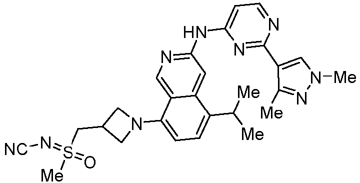

N-(((1-(3-((2-(1,3-Dimethyl-1H-pyrazol-4-yl)pyrimidin-4-yl)amino)-5-isopropylisoquinolin-8-yl)azetidin-3-yl)methyl)(methyl)(carbonyl)-I6-sulfanylidene)cyanamide was prepared with reference to example 5.
¹H NMR (400 MHz, DMSO-*d*₆) *δ* 10.07 (s, 1H), 9.09 (s, 1H), 8.54 (s, 1H), 8.37 (d, *J* = 5.8 Hz, 1H), 8.18 (s, 1H), 7.44 (d, *J* = 8.0 Hz, 1H), 7.21 (d, *J* = 5.5 Hz, 1H), 6.44 (d, *J* = 8.1 Hz, 1H), 4.46-4.36 (m, 2H), 4.13 (d, *J* = 7.2 Hz, 2H), 4.06-3.97 (m, 2H), 3.85 (s, 3H), 3.58-3.51 (m, 4H), 3.42-3.39 (m, 1H), 2.51 (s, 3H), 1.33 (d, *J* = 6.8 Hz, 6H);
MS m/z (ESI):530.2 [M+H]⁺.

### Example 168

### Preparation of (R)-3-(1-(3-((2-(3-chloro-1-methyl-1H-pyrazol-4-yl)pyrimidin-4-yl)amino)-5-isopropylisoquinolin-8-yl)azetidin-3-yl)-5-methyloxazolidin-2-one

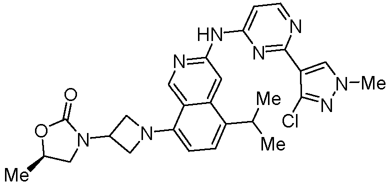

(R)-3-(1-(3-((2-(3-Chloro-1-methyl-1H-pyrazol-4-yl)pyrimidin-4-yl)amino)-5-isopropylisoquinolin-8-yl)azetidin-3-yl)-5-methyl oxazolidin-2-one was prepared with reference to example 5.
¹H NMR (400 MHz, DMSO-*d*₆) *δ* 10.10 (s, 1H), 9.06 (s, 1H), 8.50 (s, 1H), 8.34 (d, *J* = 5.9 Hz, 1H), 8.26 (s, 1H), 7.37 (d, *J* = 8.0 Hz, 1H), 7.21 (d, *J* = 5.7 Hz, 1H), 6.41 (d, *J* = 8.0 Hz, 1H), 4.74-4.57 (m, 2H), 4.32 (q, *J* = 8.1 Hz, 2H), 4.25-4.12 (m, 2H), 3.87 (t, *J* = 8.4 Hz, 1H), 3.84 (s, 3H), 3.51 (dt, J = 13.7, 6.7 Hz, 1H), 1.90-1.96 (m, 1H), 1.29 (d, *J* = 6.2 Hz, 3H), 1.23 (d, *J* = 6.8 Hz, 6H);
MS m/z (ESI):533.2 [M+H]⁺.

### Example 169

### Preparation of N3-(2-(3-chloro-1-methyl-1H-pyrazol-4-yl)pyrimidin-4-yl)-5-isopropyl-8-((2R,3S)-2-methyl-3-((methanesulfonyl)methyl)azetidin-1-yl)isoquinoline-3,6-diamine

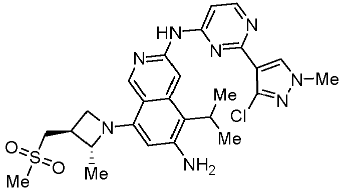

N3-(2-(3-Chloro-1-methyl-1H-pyrazol-4-yl)pyrimidin-4-yl)-5-isopropyl-8-((2R,3S)-2-methyl-3-((methanesulfonyl)methyl)azetidin-1-yl)isoquinoline-3,6-diamine was prepared with reference to example 5.

MS m/z (ESI):555.2 [M+H]⁺.

### Example 170

### Preparation of N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

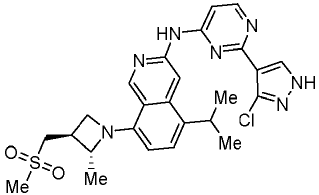

N-(2-(3-Chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d₆*) *δ* 13.50 (s, 1H), 10.22 (s, 1H), 9.09 (s, 1H), 8.68 (s, 1H), 8.41 (d, *J* = 6.0 Hz, 1H), 8.35 (s, 1H), 7.44 (d, *J* = 8.0 Hz, 1H), 7.22 (s, 1H), 6.58 (d, *J* = 8.0 Hz, 1H), 4.68 (t, *J* = 7.6 Hz, 1H), 4.24-4.17 (m, 1H), 3.69-3.62 (m, 1H), 3.60-3.51 (m, 3H), 3.00 (s, 3H), 2.93-2.85 (m, 1H), 1.43 (d, *J* = 6.0 Hz, 3H), 1.34-1.28 (m, 6H);
MS m/z (ESI):526.2 [M+H]⁺.

### Example 171

### Preparation of (S)-3-(1-(3-((2-(3-chloro-1-methyl-1H-pyrazol-4-yl)pyrimidin-4-yl)amino)-5-isopropylisoquinolin-8-yl)azetidin-3-yl)-5-methyloxazolidin-2-one

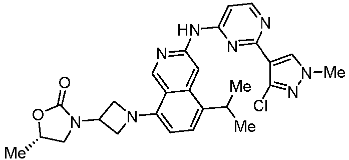

(S)-3-(1-(3-((2-(3-Chloro-1-methyl-1H-pyrazol-4-yl)pyrimidin-4-yl)amino)-5-isopropylisoquinolin-8-yl)azetidin-3-yl)-5-methyl oxazolidin-2-one was prepared with reference to example 5.
¹H NMR (400 MHz, DMSO-*d*₆) *δ* 10.17 (s, 1H), 9.12 (s, 1H), 8.57 (s, 1H), 8.40 (d, *J* = 5.9 Hz, 1H), 8.33 (s, 1H), 7.43 (d, *J* = 8.0 Hz, 1H), 7.27 (d, *J* = 5.3 Hz, 1H), 6.47 (d, *J* = 8.0 Hz, 1H), 4.85-4.62 (m, 2H), 4.38 (q, *J* = 8.1 Hz, 2H), 4.31-4.17 (m, 2H), 3.97-3.82 (m, 4H), 3.57 (dt, *J* = 13.6, 6.7 Hz, 2H), 1.35 (d, *J* = 6.2 Hz, 3H), 1.29 (d, *J* = 6.8 Hz, 6H);
MS m/z (ESI):533.2 [M+H]⁺.

### Example 172

### Preparation of 1-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-3-fluoropropan-2-ol

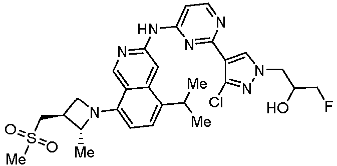

1-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-3-fluoropropan-2-ol was prepared with reference to reference example 2.
¹H NMR (400 MHz, DMSO-*d₆*) *δ* 10.20 (s, 1H), 9.10 (s, 1H), 8.53 (s, 1H), 8.42 (d, *J* = 6.0 Hz, 1H), 8.32 (s, 1H), 7.44 (d, *J* = 8.0 Hz, 1H), 7.29 (s, 1H), 6.58 (d, *J* = 8.0 Hz, 1H), 5.65-5.58 (m, 1H), 4.71-4.66 (m, 1H), 4.49-4.13 (m, 6H), 3.70-3.62 (m, 1H), 3.60-3.53 (m, 3H), 3.00 (s, 3H), 2.92-2.86 (m, 1H), 1.43 (d, *J* = 6.0 Hz, 3H), 1.35-1.27 (m, 6H);
MS m/z (ESI):602.2 [M+H]⁺.

### Example 173

### Preparation of 5-isopropyl-N-(2-(3-methyl-1H-pyrazol-4-yl)pyrimidin-4-yl)-8-((2R,3S)-2-methyl-3-((methanesulfonyl)methyl)azetidin-1-yl)isoquinolin-3-amine

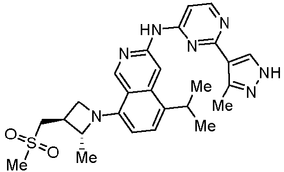

5-Isopropyl-N-(2-(3-methyl-1H-pyrazol-4-yl)pyrimidin-4-yl)-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 12.84 (s, 1H), 10.10 (s, 1H), 9.09 (s, 1H), 8.66 (s, 1H), 8.37 (d, *J* = 5.8 Hz, 1H), 8.10 (s, 1H), 7.44 (d, *J* = 8.0 Hz, 1H), 7.16 (s, 1H), 6.57 (d, *J* = 8.0 Hz, 1H), 4.71-4.66 (m, 1H), 4.22-4.18 (m, 1H), 3.68-3.63 (m, 1H),3.59-3.49 (m, 3H), 2.99 (s, 3H), 2.92-2.87 (m, 1H), 2.62 (s, 3H),1.43 (d, *J* = 6.0 Hz, 3H), 1.31 (d, *J* = 6.8 Hz, 6H);
MS m/z (ESI):506.2 [M+H]⁺.

### Example 174

### Preparation of 2-(1-(3-((2-(3-chloro-1-methyl-1H-pyrazol-4-yl)pyrimidin-4-yl)amino)-5-isopropylisoquinolin-8-yl)azetidin-3-yl)tetrahydrothiophene 1,1-dioxide

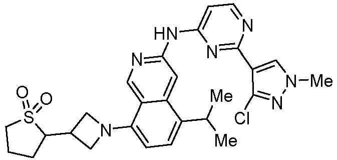

2-(1-(3-((2-(3-Chloro-1-methyl-1H-pyrazol-4-yl)pyrimidin-4-yl)amino)-5-isopropylisoquinolin-8-yl)azetidin-3-yl)tetrahydrothiophene 1,1-dioxide was prepared with reference to example 5.
¹H NMR (400 MHz, DMSO-*d*₆) *δ* 10.14 (s, 1H), 9.02 (s, 1H), 8.53 (s, 1H), 8.34 (d, *J* = 5.9 Hz, 1H), 8.27 (s, 1H), 7.38 (d, *J* = 8.0 Hz, 1H), 7.21 (d, *J* = 5.2 Hz, 1H), 6.43 (d, *J* = 8.1 Hz, 1H), 4.41 (d,*J* = 8.6 Hz, 2H), 4.16 (d, *J* = 8.7 Hz, 2H), 3.84 (s, 3H), 3.54-3.48 (m, 1H), 3.18-3.01 (m, 2H), 2.29-2.23 (m, 2H), 1.96-1.91 (m, 1H), 1.86-1.81 (m, 1H), 1.59-1.54 (m, 2H), 1.24 (d, *J* = 6.8 Hz, 6H);
MS m/z (ESI):552.2 [M+H]⁺.

### Example 175

### Preparation of N-(2-(3-chloro-1-methyl-1H-pyrazol-4-yl)-6-methylpyrimidin-4-yl)-5-isopropyl-8-((2R,3S)-2-methyl-3-((methanesulfonyl)methyl)azetidin-1-yl)isoquinolin-3-amine

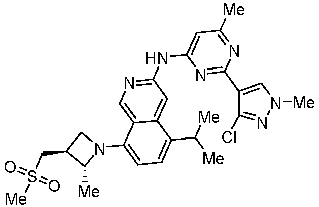

N-(2-(3-Chloro-1-methyl-1H-pyrazol-4-yl)-6-methylpyrimidin-4-yl)-5-isopropyl-8-((2R,3S)-2-methyl-3-((methanesulfonyl)methyl)azetidin-1-yl)isoquinolin-3-amine was prepared with reference to example 5.
¹H NMR (400 MHz, DMSO-*d₆*) *δ* 10.06 (s, 1H), 9.09 (s, 1H), 8.57 (s, 1H), 8.32 (s, 1H), 7.42 (d, *J* = 8.0 Hz, 1H), 7.11 (s, 1H), 6.56 (d, *J* = 8.0 Hz, 1H), 4.68 (t, *J* = 7.6 Hz, 1H), 4.24-4.17 (m, 1H), 3.90 (s, 3H), 3.68-3.63 (m, 1H), 3.60-3.51 (m, 3H), 3.00 (s, 3H), 2.92-2.85 (m, 1H), 2.37 (s, 3H), 1.43 (d, *J* = 6.0 Hz, 3H), 1.32-1.27 (m, 6H);
MS m/z (ESI):554.2 [M+H]⁺.

### Example 176

### Preparation of N-(2-(3-chloro-1-(2,2-dimethyltetrahydro-2H-pyran-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

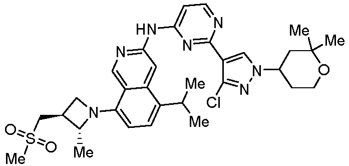

N-(2-(3-Chloro-1-(2,2-dimethyltetrahydro-2H-pyran-4-yl)-1H-pyrazol-4-yl)pyrimidin-4-yl)-5-isopropyl-8-((2R,3S)-2-methyl-3-((methanesulfonyl)methyl) azetidin-1-yl)isoquinolin-3-amine was prepared with reference to reference example 2.

MS m/z (ESI):638.2 [M+H]⁺.

### Example 177

### Preparation of N-(2-(3-chloro-1-(difluoromethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

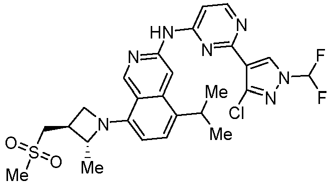

N-(2-(3-Chloro-1-(difluoromethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.

¹H NMR (400 MHz, DMSO-*d*₆) *δ* 10.36 (s, 1H), 9.10 (s, 1H), 8.81 (s, 1H), 8.60 (br s, 1H), 8.46 (d, *J* = 7.2 Hz, 1H), 7.89 (d, *J* = 60 Hz, 1H), 7.42 (d, *J* = 7.2 Hz, 1H), 7.33 (br s, 1H), 6.58 (d, *J* = 7.2 Hz, 1H), 4.70-4.66 (m, 1H), 4.22-4.19 (m, 1H), 3.67-3.52 (m, 4H), 3.00 (s, 3H), 2.92-2.87 (m, 1H), 1.37-1.16 (m, 9H);

MS m/z (ESI):576.2 [M+H]⁺.

### Example 178

### Preparation of N-(1-(3-((2-(3-chloro-1-methyl-1H-pyrazol-4-yl)pyrimidin-4-yl)amino)-6-fluoro-5-isopropylisoquinolin-8-yl)azetidin-3-yl)methanesulfonamide

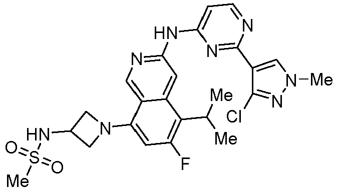

N-(1-(3-((2-(3-Chloro-1-methyl-1H-pyrazol-4-yl)pyrimidin-4-yl)amino)-6-fluoro-5-isopropylisoquinolin-8-yl)azetidin-3-yl)methanesulfonamide was prepared with reference to example 44.
¹H NMR (400 MHz, DMSO-*d*₆) *δ* 10.24 (s, 1H), 9.08 (s, 1H), 8.63 (s, 1H), 8.48 (d, *J* = 5.2 Hz, 1H), 8.36 (s, 1H), 7.92 (d, *J* = 7.2 Hz, 1H), 7.35 (br s, 1H), 6.36 (d, *J* = 8.0 Hz, 1H), 4.69-4.65 (m, 2H), 4.44-4.41 (m, 1H), 4.08-4.04 (m, 2H), 3.95 (s, 3H), 3.58-3.52 (m, 1H), 3.04 (s, 3H), 1.44-1.33 (m, 6H);
MS m/z (ESI):545.2 [M+H]⁺.

### Example 179

### Preparation of N-(2-(3-chloro-1-((2S,4S)-1,2-dimethylpiperidin-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

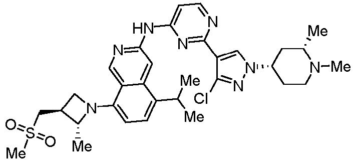

N-(2-(3-Chloro-1-((2S,4S)-1,2-dimethylpiperidin-4-yl)-1H-pyrazol-4-yl)pyrimidin-4-yl)-5-isopropyl-8-((2R,3S)-2-methyl-3-((methanesulfonyl)methyl) azetidin-1-yl)isoquinolin-3-amine was prepared with reference to reference example 2.

¹H NMR (400 MHz, DMSO-*d*₆) *δ* 10.12 (s, 1H), 9.03 (s, 1H), 8.48 (s, 1H), 8.34 (d, *J* = 5.8 Hz, 1H), 8.30 (s, 1H), 7.36 (d, *J* = 8.0 Hz, 1H), 7.16 (s, 1H), 6.51 (d, *J* = 8.0 Hz, 1H), 5.30-5.22 (m, 1H), 4.61 (t, *J* = 7.6 Hz, 1H), 4.23-4.09 (m, 2H), 3.62-3.39 (m, 4H), 2.93 (s, 3H), 2.89-2.77 (m, 2H), 2.13 (s, 3H), 1.97-1.83 (m, 5H), 1.36 (d, *J* = 6.0 Hz, 3H), 1.27-1.20 (m, 6H), 1.01 (d, *J* = 6.0 Hz, 3H);

MS m/z (ESI):637.2 [M+H]⁺.

### Example 180

### Preparation of N-(2-(3-chloro-1-((3S,6R)-1,6-dimethylpiperidin-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

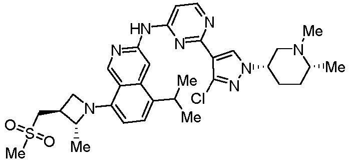

N-(2-(3-Chloro-1-((3S,6R)-1,6-dimethylpiperidin-3-yl)-1H-pyrazol-4-yl)pyrimidin-4-yl)-5-isopropyl-8-((2R,3S)-2-methyl-3-((methanesulfonyl)methyl) azetidin-1-yl)isoquinolin-3-amine was prepared with reference to reference example 2.
¹H NMR (400 MHz, DMSO-d₆) *δ* 10.11 (s, 1H), 9.03 (s, 1H), 8.44 (s, 1H), 8.38-8.31 (m, 1H), 8.28 (s, 1H), 7.36 (d, *J* = 8.0 Hz, 1H), 7.23 (s, 1H), 6.51 (d, *J* = 8.0 Hz, 1H), 5.26 (t, *J* = 4.8 Hz, 1H), 4.61 (t, *J* = 7.6 Hz, 1H), 4.18-4.09 (m, 2H), 3.96-3.87 (m, 1H), 3.58 (d, *J* = 7.2 Hz, 1H), 3.53-3.42 (m, 3H), 2.93 (s, 3H), 2.87-2.77 (m, 1H), 2.19 (s, 3H), 1.99-1.87 (m, 5H), 1.36 (d, *J* = 6.2 Hz, 3H), 1.26-1.19 (m, 6H), 0.83 (d, *J* = 6.4 Hz, 3H);
MS m/z (ESI):637.2 [M+H]⁺.

### Example 181

### Preparation of N-(2-(3-chloro-1-((3R,6S)-1,6-dimethylpiperidin-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

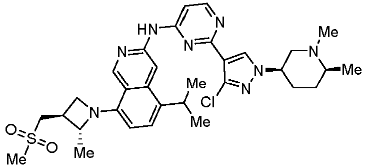

N-(2-(3-Chloro-1-((3R,6S)-1,6-dimethylpiperidin-3-yl)-1H-pyrazol-4-yl)pyrimidin-4-yl)-5-isopropyl-8-((2R,3S)-2-methyl-3-((methanesulfonyl)methyl) azetidin-1-yl)isoquinolin-3-amine was prepared with reference to reference example 2.
¹H NMR (400 MHz, DMSO-*d*₆) *δ* 10.20 (s, 1H), 9.12 (s, 1H), 8.54 (s, 1H), 8.46-8.40 (m, 1H), 8.37 (s, 1H), 7.45 (d, *J* = 8.0 Hz, 1H), 7.24 (s, 1H), 6.60 (d, *J* = 8.0 Hz, 1H), 5.41-5.29 (m, 1H), 4.70 (t, *J* = 7.6 Hz, 1H), 4.28-4.19 (m, 2H), 4.06-3.96 (m, 1H), 3.71-3.45 (m, 4H), 3.02 (s, 3H), 2.96-2.87 (m, 1H), 2.28 (s, 3H), 2.07-1.97 (m, 5H), 1.45 (d, *J* = 6.2 Hz, 3H), 1.41-1.23 (m, 6H), 0.93 (d, *J* = 6.4 Hz, 3H);
MS m/z (ESI):637.2 [M+H]⁺.

### Example 182

### Preparation of N-(2-(3-chloro-1-(((S)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

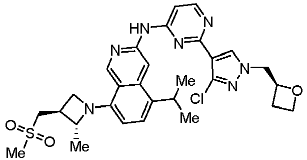

N-(2-(3-Chloro-1-(((S)-oxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.
¹H NMR (400 MHz, DMSO-*d*₆) *δ* 10.20 (s, 1H), 9.10 (s, 1H), 8.51 (s, 1H), 8.41 (d, *J* = 6.0 Hz, 1H), 8.36 (s, 1H), 7.43 (d, *J* = 8.0 Hz, 1H), 7.31 (d, *J* = 5.8 Hz, 1H), 6.58 (d, *J* = 8.0 Hz, 1H), 5.11-5.00 (m, 1H), 4.68 (t, *J* = 7.6 Hz, 1H), 4.56-4.27 (m, 4H), 4.26-4.15 (m, 1H), 3.65 (t, *J* = 7.2 Hz, 1H), 3.60-3.49 (m, 3H), 3.00 (s, 3H), 2.96-2.83 (m, 1H), 2.77-2.64 (m, 1H), 2.49-2.38 (m, 1H), 1.43 (d, *J* = 6.0 Hz, 3H), 1.34-1.27 (m, 6H);
MS m/z (ESI):596.2 [M+H]⁺.

### Example 183

### Preparation of (R)-5-((3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)methyl)pyrrolidin-2-one

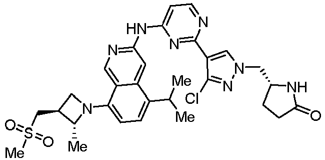

(R)-5-((3-Chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)methyl)pyrrolidin-2-one was prepared with reference to reference example 2.
¹H NMR (400 MHz, DMSO-*d₆*) *δ* 10.25 (s, 1H), 9.10 (s, 1H), 8.66 (s, 1H), 8.51-8.33 (m, 1H), 8.24 (s, 1H), 7.81 (s, 1H), 7.44 (t, *J* = 7.8 Hz, 1H), 7.26 (s, 1H), 6.61 (t, *J* = 16.8 Hz, 1H), 4.68 (t, *J* = 7.5 Hz, 1H), 4.35-4.16 (m, 3H), 4.16-3.92 (m, 1H), 3.66 (t, *J* = 7.1 Hz, 1H), 3.61-3.47 (m, 3H), 3.00 (s, 3H), 2.96-2.79 (m, 1H), 2.18-2.05 (m, 3H), 1.98-1.89 (m, 1H), 1.43 (d, *J* = 6.1 Hz, 3H), 1.32 (t, J = 6.3 Hz, 6H);
MS m/z (ESI): 623.2 [M+H]⁺.

### Example 184

### Preparation of (S)-5-((3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)methyl)pyrrolidin-2-one

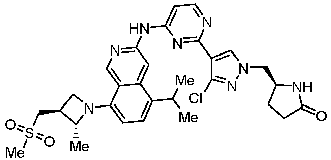

(S)-5-((3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)methyl)pyrrolidin-2-one was prepared with reference to reference example 2.
¹H NMR (400 MHz, DMSO-*d₆*) *δ* 10.24 (s, 1H), 9.10 (s, 1H), 8.65 (s, 1H), 8.43 (d, *J* = 5.9 Hz, 1H), 8.27-8.14 (m, 1H), 7.79 (d, *J* = 12.8 Hz, 1H), 7.44 (d, *J* = 8.0 Hz, 1H), 7.25 (s, 1H), 6.56 (t, *J* = 13.9 Hz, 1H), 4.68 (t, *J* = 7.5 Hz, 1H), 4.29-4.16 (m, 3H), 4.10-3.80 (m, 1H), 3.66 (t, *J* = 7.1 Hz, 1H), 3.61-3.47 (m, 3H), 3.00 (s, 3H), 2.96-2.79 (m, 1H), 2.18-2.05 (m, 3H), 1.94 (dt, *J* = 10.0, 5.7 Hz, 1H), 1.43 (d, *J* = 6.0 Hz, 3H), 1.32 (dd, *J* = 6.7, 2.9 Hz, 6H);
MS m/z (ESI):623.2 [M+H]⁺.

### Example 185

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)butyronitrile

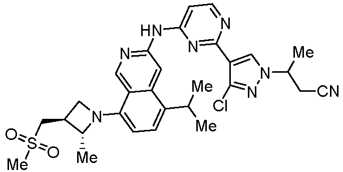

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)butyronitrile was prepared with reference to reference example 2.
¹H NMR (400 MHz, DMSO-*d*₆) *δ* 10.19 (s, 1H), 9.10 (s, 1H), 8.50 (s, 1H), 8.49 (s, 1H), 8.42 (d, *J* = 5.9 Hz, 1H), 7.43 (d, *J* = 8.0 Hz, 1H), 7.33 (s, 1H), 6.58 (d, *J* = 8.0 Hz, 1H), 4.84-4.79 (m, 1H), 4.68 (t, *J* = 7.5 Hz, 1H), 4.20 (t, *J* = 6.2 Hz, 1H), 3.65 (t, *J* = 7.1 Hz, 1H), 3.60-3.48 (m, 3H), 3.18 (d, *J* = 6.7 Hz, 2H), 3.00 (s, 3H), 2.92-2.86 (m, 1H), 1.55 (d, *J* = 6.7 Hz, 3H), 1.43 (d, *J* = 6.0 Hz, 3H), 1.29 (t, J = 6.8 Hz, 6H);
MS m/z (ESI):593.2 [M+H]⁺.

### Example 186

### Preparation of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)methanesulfonamide

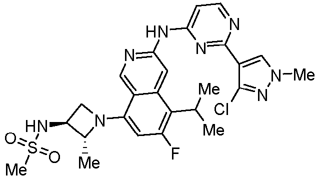

N-((2R,3S)-1-(3-((2-(3-Chloro-1-methyl-1H-pyrazol-4-yl)pyrimidin-4-yl)amino)-6-fluoro-5-isopropylisoquinolin-8-yl)-2-methylazetidin-3-yl)methanesulfonamide was prepared with reference to example 44.
¹H NMR (400 MHz, DMSO-*d*₆) *δ* 10.16 (s, 1H), 8.96 (s, 1H), 8.54 (s, 1H), 8.35 (d, *J* = 7.2 Hz, 1H), 8.24 (s, 1H), 7.71 (d, *J* = 7.6 Hz, 1H), 7.20 (br s, 1H), 6.36 (br s, 1H), 4.82-4.77 (m, 1H), 4.16-4.10 (m, 1H), 3.88-3.83 (m, 4H), 3.62-3.54 (m, 2H), 2.90 (s, 3H), 1.37-1.16 (m, 9H);
MS m/z (ESI):559.2 [M+H]⁺.

### Example 187

### Preparation of N3-(2-(3-chloro-1-methyl-1H-pyrazol-4-yl)pyrimidin-4-yl)-N5,N5-dimethyl-8-((2R,3S)-2-methyl-3-((methanesulfonyl)methyl)azetidin-1-yl)isoquinoline-3,5-diamine

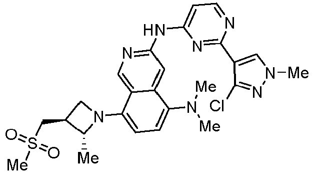

N3-(2-(3-Chloro-1-methyl-1H-pyrazol-4-yl)pyrimidin-4-yl)-N5,N5-dimethyl-8-((2R,3S)-2-methyl-3-((methanesulfonyl)methyl)azetidin-1-yl)isoquinoline-3,5-diamine was prepared with reference to example 5.
¹H NMR (400 MHz, DMSO-*d*₆) *δ* 10.23 (s, 1H), 9.07 (s, 1H), 8.86 (s, 1H), 8.42-8.34 (m, 2H), 7.23 (d, *J* = 8.2 Hz, 1H), 7.13 (d, *J* = 5.8 Hz, 1H), 6.54 (d, *J* = 8.3 Hz, 1H), 4.66-4.59 (m, 1H), 4.21-4.12 (m, 1H), 3.93 (s, 3H), 3.62-3.46 (m, 3H), 3.00 (s, 3H), 2.92-2.84 (m, 1H), 2.75 (s, 6H), 1.41 (d, *J* = 6.0 Hz, 3H);
MS m/z (ESI):541.2 [M+H]⁺.

### Example 188

### Preparation of N-(2-(3-chloro-1-((4-methyloxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

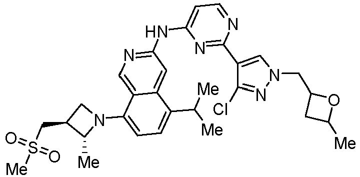

N-(2-(3-Chloro-1-((4-methyloxetan-2-yl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to reference example 2.

MS m/z (ESI):610.2 [M+H]⁺.

### Biological test and evaluation

The present invention will be further described and explained below in conjunction with test examples, but these examples are not meant to limit the scope of the present invention.

### Test example 1. Determination of the inhibitory effect of the compounds of the present invention on the activity of EGFR del19/T790M/C797S and EGFR L858R/T790M/C797S mutant kinases

Experimental objective: The objective of this test example is to test the inhibitory activity of the compounds on the activity of EGFR del19/T790M/C797S and EGFR L858R/T790M/C797S mutant kinases.

Experimental instruments: The centrifuge (5810R) was purchased from Eppendorf, the pipette was purchased from Eppendorf or Rainin, and the microplate reader was purchased from BioTek (United States) with a model of SynergyH1 multifunctional microplate reader.

Experimental method: This experiment used Cisbio's HTRF kinase assay method (Cisbio#62TK0PEB), wherein a catalytic reaction occurred between the substrate polypeptide TK and ATP in the presence of the tyrosine kinase with EGFR del19/T790M/C797S or EGFR L858R/T790M/C797S mutation, the substance was phosphorylated, and the activity of the kinase was characterized by measuring the content of the phosphorylated substrate generated during the reaction, and the half inhibitory concentration IC₅₀ of the compound on the activity of EGFR del19/T790M/C797S or EGFR L858R/T790M/C797S mutant kinase was obtained.

Specific experimental operations were as follows:
the kinase reaction was carried out in a white 384-well plate (Perkin Elmer#6008280), and 1-5 µL of compounds of different concentrations which were diluted with 1% DMSO-containing ddH₂O were added; to the positive control wells were added 1-5 µL of 1% DMSO-containing ddH₂O, followed by 1-5 µL of 0.5-5 nM 4×EGFR del19/T790M/C797S or EGFR L858R/T790M/C797S mutant kinase solution which was diluted with Dilution buffer (5×kinase buffer, MgCl₂ 6.65 Mm, MnCl₂ 1.33 mM, DTT 1.33 mM); to the negative control wells were added 1-5 µL of Dilution buffer; to all the wells were added 1-5 µL of 4 µM 4× substrate TK solution which was prepared with 10× Dilution buffer, and finally added 1-5 µL of 24 µM 4× ATP solution which was diluted with Dilution buffer to start the reaction; after reacting at room temperature for 120 minutes, 10 µL of detection solution (TK antibody 16 nM, XL665 0.5 µM) was added to each well, same was reacted at room temperature in the dark for 20 minutes, and then the chemiluminescence value was detected using a BioTek Synergy H1 microplate reader.

### Experimental data processing method:

The percent inhibition data for compound-treated wells was calculated according to the positive control wells (DMSO control wells) and negative control wells (no kinase added) on the plate {% inhibition rate = 100-[(test compound value-negative control value)] / (positive control value - negative control value) × 100}. GraphPad prism was used to fit different concentrations and corresponding percent inhibition rate data to a 4-parameter nonlinear logistic equation to calculate IC₅₀ values.

Experimental results and conclusions: The compounds in this example of the present invention have good inhibitory effects on EGFR L858R/T790M/C797S and EGFR del19/T790M/C797S drug-resistant mutant kinases.

### Test example 2: Cell proliferation inhibition experiment

Experimental objective: The objective of this test example is to test the inhibitory activity of compounds on the proliferation of different EGFR C797S drug-resistant mutant cell lines Ba/F3 EGFR Del19/T790M/C797S, Ba/F3 EGFR L858R/T790M/C797S, and Ba/F3 EGFR Del19/C797S.

### Experimental instruments:

Centrifuge (Eppendorf 5810R); Microplate reader (BioTek Synergy H1)
Pipette (Eppendorf or Rainin); Carbon dioxide incubator (Thermo 311)
Cell counter (Life Countess II)

### Experimental reagents and consumables:

Ba/F3 EGFR Del19/T790M/C797S cells were purchased from KYinno Biotechnology Co., Ltd.;
Ba/F3 EGFR Del19/C797S cells were purchased from CoBioer Biosciences Co., Ltd.;
Ba/F3 EGFR L858R/T790M/C797S cells were purchased from CoBioer Biosciences Co., Ltd.;
Cell Titer-Glo was purchased from Promega, with a catalog number of G7573;
RPMI 1640 was purchased from Gibco, with a catalog number of 22400089;
FBS was purchased from Gibco, with a catalog number of 10091148;
cell culture plates were purchased from Corning, with a catalog number of 3610.

### Experimental method:

The inhibitory activity of the compound on the proliferation of different EGFR C797S drug-resistant mutant cell lines was detected using the Cell Titer-Glo method. The different cell lines were cultured in RPMI 1640 complete culture medium containing 10% FBS under conditions of 37°C and 5% CO₂, the cells were collected by centrifugation when growing to a certain density, and adjusted to appropriate cell density after counting, the cells were spread on a white 96-well plate at 90 µL/well, and cultured in a 37°C, 5% CO₂ incubator overnight, prepared compound solutions of different concentrations were added at 10 µL/well, corresponding solvent controls were set, and all wells were cultured in a 37°C, 5% CO₂ incubator for 72 hours, 50 µL of CellTiter-Glo solution was added to each well, shook and mixed evenly, and incubated in the dark for 10 minutes, and a BioTek Synergy H1 microplate reader was used for reading.

### Experimental data processing method:

The luminescence signal value was used to calculate the inhibition rate. Graphpad Prism software was used to fit the concentration and inhibition rate to a nonlinear regression curve, so as to obtain the IC₅₀ value, as shown in the Table below for details.

**Table 1**

| Example number | Ba/F3 EGFR Del19_T790M_C797S IC₅₀ (nM) |
|---|---|
| 1 | 4.5 |
| 2 | 6.2 |
| 4 | 7.7 |
| 5 | 3.7 |
| 6 | 5.1 |
| 44 | 10.0 |
| 67 | 4.0 |
| 116A | 7.6 |
| 120 | 7.1 |
| 121 | 4.5 |
| 122 | 2.9 |
| 123 | 3.3 |
| 125 | 6.8 |
| 126 | 2.1 |
| 127 | 4.5 |
| 130 | 8.1 |
| 132 | 5.6 |
| 172 | 5.0 |
| 182 | 4.4 |
| 186 | 5.9 |

**Table 2**

| Example | Ba/F3 EGFR L858R_T790M_C797S IC₅₀ (nM) |
|---|---|
| 5 | 11.0 |
| 6 | 13.0 |
| 120 | 14.0 |
| 121 | 15.0 |
| 122 | 8.2 |
| 123 | 13.0 |
| 126 | 7.5 |
| 132 | 15 |
| 182 | 10 |
| 186 | 8.8 |

**Table 3**

| Example number | Ba/F3 EGFR Del19/C797S IC50 (nM) |
|---|---|
| 1 | 12.0 |
| 2 | 13.0 |
| 3 | 16.0 |
| 4 | 20.0 |
| 5 | 5.1 |
| 6 | 5.1 |
| 9 | 57.0 |
| 44 | 8.4 |
| 59 | 5.0 |
| 60 | 5.6 |
| 61 | 6.0 |
| 67 | 3.2 |
| 71 | 17.0 |
| 102 | 73.0 |
| 109 | 14.0 |
| 112 | 52 |
| 113 | 60 |
| 114 | 39 |
| 115 | 73 |
| 116A | 33 |
| 120 | 11.0 |
| 121 | 6.2 |
| 122 | 5.1 |
| 123 | 6.5 |
| 124 | 14.0 |
| 125 | 18 |
| 126 | 3.0 |
| 127 | 7.8 |
| 129 | 22 |
| 130 | 5.9 |
| 131 | 7.4 |
| 132 | 6.4 |
| 133 | 27 |
| 141 | 64 |
| 142 | 41.0 |
| 143 | 30.0 |
| 144 | 26.0 |
| 145 | 45.0 |
| 146 | 80 |
| 150 | 70 |
| 151A | 41 |
| 154A | 22 |
| 156 | 42.0 |
| 158 | 50.0 |
| 165 | 22 |
| 167 | 74 |
| 169 | 52 |
| 170 | 60 |
| 172 | 33 |
| 177 | 43 |
| 178 | 19 |
| 185 | 80 |
| 186 | 2.6 |
| 188 | 65 |

### Experimental conclusion:

According to the above-mentioned schemes, it can be concluded that the compounds shown in the present invention show a significant inhibitory activity against the proliferation of different EGFR C797S drug-resistant mutant cell lines in the proliferation inhibitory activity test.

### Test example 3: Rat pharmacokinetic evaluation test

### 1. Study objective:

SD rats were used as test animals to study the pharmacokinetic behavior of the compounds of the present invention in the rat body (plasma) after oral administration at a dose of 5 mg/kg.

### 2. Experimental scheme:

2.1 Experimental drugs: the example compound of the present invention, made in house.
2.2 Experimental animals: 3 SD rats/group, male, from Shanghai Jiesijie Experimental Animal Co., Ltd., animal production license number (SCXK (Shanghai) 2013-0006 N0.311620400001794).
2.3 Formulation prescription:

### Oral administration drug preparation: 20% solutol HS15 in 0.5% MC (methylcellulose)

20 g of a Solutol HS15 solid and 0.5 g of a MC solid powder were weighed and dissolved in 80 mL of purified water, and the mixture was vortexed, mixed until uniform, and subjected to ultrasound treatment to obtain a clear solution of 20% solutol HS15 in 0.5% MC.

The example compound was weighed into a 20 mL glass bottle, the solution was added thereto and the mixture was subjected to ultrasound treatment for 10 minutes to obtain a clear solution with a concentration of 0.5 mg/mL.

### 2.4 Administration:

3 male SD rats were administered p.o. respectively after the 3 male SD rats were fasted overnight; the p.o. dose was 5 mg/kg and the administration volume was 10 mL/kg.

### 2.5 Sample collection:

Blood collection: Before and after administration to rats, 0.2 mL of blood was collected from the jugular vein at 0.25, 0.5, 1, 2, 4, 6, 8 and 24 h, and placed in an EDTA-K₂ anticoagulation tube, which was centrifuged at 6000 rpm at 4°C for 6 min to separate plasma which was stored at -80°C; the rats were fed 4 h after administration.

### 2.6 Sample treatment:

1) 160 µL of acetonitrile was added to 40 µL of plasma sample for precipitation, mixed and centrifuged at 3500 × g for 5 to 20 min.
2) the treated supernatant solution was taken and subjected to LC/MS/MS to analyze the concentrations of the test compounds. LC/MS/MS analysis instrument: AB Sciex API 4000 Qtrap.

### 2.7 Liquid phase analysis:

- Liquid phase conditions: Shimadzu LC-20AD pump
- Chromatographic column: Agilent ZORBAX XDB-C18 (50×2.1 mm, 3.5 µm) mobile phase: liquid A is 0.1% aqueous formic acid solution, liquid B is acetonitrile
- Flow rate: 0.4 mL/min
- Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main pharmacokinetic parameters were calculated using WinNonlin 6.1. The results of pharmacokinetic experiments in rats are as shown in Table 4 below:

**Table 4**

| Example | Cmax (ng/mL) | AUC₀₋ₜ (hr*ng/mL) | T_{1/2} (h) |
|---|---|---|---|
| 1 | 287 | 1406 | 2.7 |
| 3 | 458 | 2377 | 4.1 |
| 5 | 239 | 1259 | 4.1 |
| 67 | 186 | 2173 | 2.7 |
| 120 | 178 | 2102 | 2.3 |
| 121 | 142 | 1771 | 3.9 |
| 122 | 97 | 1262 | 3.5 |
| 126 | 240 | 3143 | 3.3 |
| 127 | 157 | 2005 | 3.9 |
| 132 | 180 | 1568 | 3.0 |

### 3.4 Experimental conclusion:

The data in the table show that in the rat pharmacokinetic evaluation experiment, the example compounds of the present invention show high exposure after oral administration.

### Test example 4: In vivo pharmacodynamic study of the compound in a nude mouse subcutaneous transplanted tumor model of mouse primary B cell line Ba/F₃ EGFR Del19/C797S

### 1.1 Experimental objective

The objective of this test example is to evaluate the in vivo efficacy of the compound in a nude mouse subcutaneous transplanted tumor model of mouse primary B cell line Ba/F₃ EGFR Del19/C797S.

### 1.2 Experimental instruments and reagents

### 1.2.1 Instruments

1. Refrigerator (BCD-268TN, Haier)
2. Biological safety cabinet (BSC-1300Il A2, Shanghai Boxun Industrial Co., Ltd. Medical Equipment Factory)
3. Ultra-clean workbench (CJ-2F, Suzhou Fengshi Experimental Animal Equipment Co., Ltd.)
4. Electric pipette-aid (Easypet 3, Eppendorf)
5. Thermostat water bath (HWS-12, Shanghai Yiheng Science)
6. CO₂ incubator (Thermo-311, Thermo)
7. Centrifuge (Centrifuge 5720R, Eppendorf)
8. Full-automatic cell counter (Countess II, Life Technologies)
9. Vernier caliper (CD-6"AX, Mitutoyo)
10. Cell culture flask (T25/T75/T225, Corning)
11. Electronic balance (CPA2202S, Sartorius)
12. Electronic balance (BSA2202S-CW, Sartorius)
13. Ultrasonic cleaner (115F0032, Shanghai Kudos)
14. Water purifier (Pacific TII, Thermo)
15. Magnetic stirrer (08-2G, Chijiu)

### 1.2.2 Reagents

1. RPMI -1640 medium (22400-089, Gibco)
2. Fetal bovine serum (FBS) (10099-141C, Gibco)
3. Phosphate buffer solution (PBS) (10010-023, Gibco)
4. Kolliphor HS15 (42966-1KG, Sigma-Aldrich)
5. Methylcellulose M450 (69016482, Sinopharm Chemical Reagent Co., Ltd.)

### 1.3 Experimental operations and data processing

### 1.3.1 Animals

BALB/c nude mice, 6-8 weeks old, ♀, purchased from the Laboratory Animal Business Department of Shanghai Institute of Planned Parenthood Research

### 1.3.2 Cell culture and cell suspension preparation

a. A Ba/F₃ EGFR Del19/C797S cell line was taken out from the cell bank, and the cells were recovered with RPMI-1640 medium (RPMI-1640 + 10% FBS). The recovered cells were cultured in a CO₂ incubator (incubator temperature: 37°C, CO₂ concentration: 5%).
b. The cells were passaged every three days, and after passage, the cells were placed in a CO₂ incubator to continue the culture. This process was repeated until the cell number meets the needs of in vivo efficacy.
c. The cells in the exponential growth phase were collected, counted using a full-automatic cell counter, resuspended to 2 × 10⁷ cells/mL with PBS according to the counting results, and then placed in an ice box until use.

### 1.3.3 Cell inoculation

a. Nude mouse was marked with a disposable universal ear tag for mice and rats before inoculation.
b. At the time of inoculation, the cell suspension was mixed well, 0.1-1 mL of cell suspension was taken with a 1 mL syringe, and after removing air bubbles, the syringe was placed on an ice bag until use.
c. The nude mouse was secured with the left hand. The position of the right back near the right shoulder of the nude mouse (inoculation site) was disinfected with a 75% alcohol cotton ball, and inoculation was carried out after 30 seconds.
d. The nude mice were inoculated successively (inoculation with 0.1 mL of cell suspension per mouse).

### 1.3.4 Tumor volume measurement, grouping and administration in tumor-bearing mice

a. Tumor was measured on days 9-12 after inoculation depending on the tumor growth, and tumor size was calculated. Tumor volume calculation: tumor volume (mm3) = length (mm) × width (mm) × width (mm)/2
b. According to the weight of tumor-bearing mice and the size of the tumor, the mice were grouped by random grouping.
c. According to the grouping results, the test drug was started to be administered (administration method: oral administration; administration volume: 10 mL/kg; administration frequency: once/day or twice/day; administration cycle: 14 days; vehicle: 10% Solutol HS15/0.5%MC).
d. Tumors were measured and weighed twice a week after starting administration of test drugs.
e. Animals were euthanized at the end of the experiment.
f. The data was processed with software such as Excel. Calculation of tumor growth inhibition (TGI) (%) of a compound: when the tumor did not regress, TGI (%) = [(1-(average tumor volume at the end of administration in a treatment group - average tumor volume at the beginning of administration in the treatment group))/ (average tumor volume at the end of administration in the solvent control group - average tumor volume at the beginning of administration in the solvent control group)] × 100%. When the tumor regressed, TGI(%) = [1-(average tumor volume at the end of administration in a treatment group - average tumor volume at the beginning of administration in the treatment group)/average tumor volume at the beginning of administration in the treatment group] × 100%.

### 1.4 Experimental results and conclusion:

The key example compounds of the present invention exhibit excellent tumor inhibition effects in the model. When administered orally at a dose of 50 mpk QD, the compounds exhibited an excellent tumor inhibition effect, with tumor growth inhibition (TGI) (%) > 80%, and the tumor growth inhibition (TGI) (%) of the key preferred compounds > 100%. When administered orally at a dose of 75 mpk QD, the compounds exhibited an excellent tumor inhibition effect, with tumor growth inhibition (TGI) (%) > 100%, and the tumor growth inhibition (TGI) (%) of the key preferred compounds > 150%. When administered orally at a dose of 120 mpk QD, the compounds exhibited an excellent tumor inhibition effect, with tumor growth inhibition (TGI) (%) > 150%, and the tumor growth inhibition (TGI) (%) of the key preferred compounds > 190%, and there was no significant weight loss.

## Claims

1. A compound as represented by general formula (II-G), or a stereoisomer or pharmaceutically acceptable salt thereof: 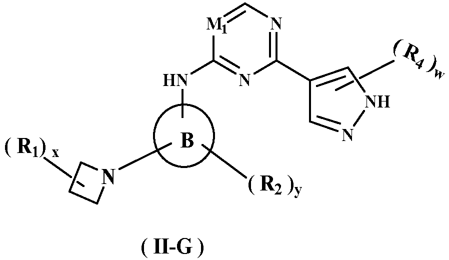
wherein: M₁ is C, N or CH;
ring B is selected from 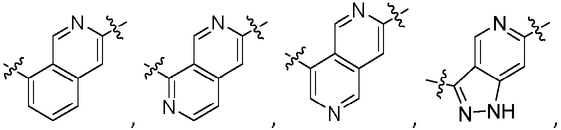 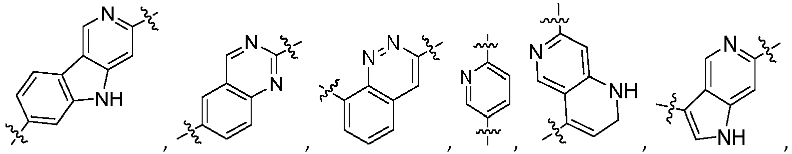 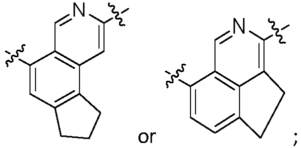
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl and heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
or, wherein one R₂ and one R₄ are connected to the atoms therebetween to form cycloalkyl, heterocyclyl, aryl or heteroaryl, and the cycloalkyl, heterocyclyl, aryl and heteroaryl can be optionally further substituted;
Rₐ is selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
w is 0, 1, 2, 3, 4, 5 or 6;
and p, m and n5 are each independently 0, 1, 2 or 3.

2. The compound or the stereoisomer or pharmaceutically acceptable salt thereof according to claim 1, wherein the compound is further as represented by general formula (VII-1): 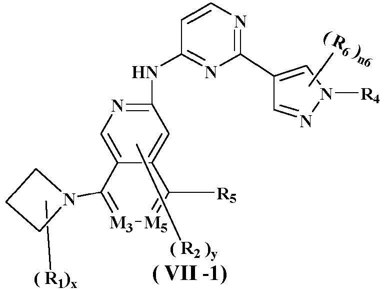 wherein:
M₅ is N or CH;
R₅ and R₆ are independently selected from hydrogen, deuterium, oxo, thio, halogen, amino, hydroxyl, cyano, nitro, C₁₋₆ alkyl, C₁₋₆ deuteroalkyl, C₁₋₆ haloalkyl, C₂₋₆ alkenyl, C₂₋₆ alkynyl, C₁₋₆ alkoxy, C₁₋₆ haloalkoxy, C₁₋₆ deuteroalkoxy or C₁₋₆ hydroxyalkyl;
and n6 is 0, 1 or 2.

3. The compound or the stereoisomer or pharmaceutically acceptable salt thereof according to claim 1 or 2, wherein
R₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -(CH₂)ₙORₐ, -(CH₂)ₙP(O)ₚ(Rₐ)ₙ₅, - (CH₂)ₙS(O)RₐₐN(Rₐₐ), -(CH₂)ₙN=S(O)(Rₐₐ)₂, -(CH₂)ₙS(O)ₘRₐ or -(CH₂)ₙC(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -S(O)RₐₐN(Rₐₐ), -N=S(O)(Rₐₐ)₂. -P(O)ₚ(Rₐₐ)ₙ₅, -S(O)ₘRₐₐ, -Se(O)ₘRₐₐ or -C(O)Rₐₐ; or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy or C₁₋₆ hydroxyalkyl;
preferably, R₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or - C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, 3-ORₐₐ, -P(O)ₚ(Rₐₐ)ₙ₅, -S(O)ₘRₐₐ, -Se(O)ₘRₐₐ or -C(O)Rₐₐ; or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy or C₁₋₆ hydroxyalkyl;
further preferably, R₁ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, C₆₋₁₀ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl, 3- to 10-membered heterocyclyl, C₆₋₁₀ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ;
Rₐₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
and n is independently 0, 1, 2 or 3.

4. The compound as represented by the general formula, or the stereoisomer or pharmaceutically acceptable salt thereof according to any one of claims 1-3, wherein
R₂ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ;
preferably, R₂ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or - C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₂ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ;
Rₐₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted.

5. The compound as represented by the general formula, or the stereoisomer or pharmaceutically acceptable salt thereof according to any one of claims 1-4, wherein
R₄ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more R₄₋₁; R₄₋₁ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, and C₁₋₆ hydroxyalkyl are optionally substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, or C₁₋₆ hydroxyalkyl; preferably, R₄ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 10-membered heteroaryl can be optionally further substituted with one or more R₄₋₁; R₄₋₁ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
or, one R₂ and one R₄ are connected to the atoms therebetween to form C₃₋₁₂ cycloalkyl, 3- to 12-membered heterocyclyl, C₆₋₁₄ aryl or 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
Rₐₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted.

6. The compound as represented by the general formula, or the stereoisomer or pharmaceutically acceptable salt thereof according to any one of claims 1-5, wherein
Rₐ is selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, Rₐ is selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl;
each Rₐₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl.

7. The compound or the stereoisomer or pharmaceutically acceptable salt thereof according to claim 1, wherein the compound is further as represented by general formula (VII-2), (VII-3) or (VII-4): 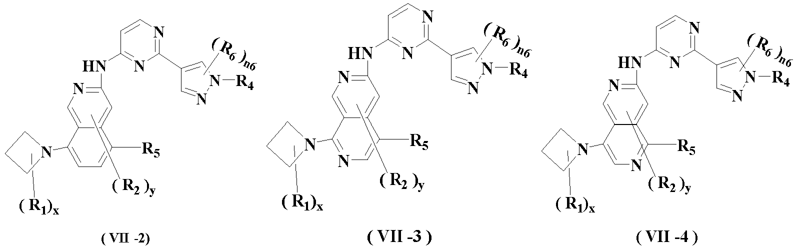
R₁ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, -(CH₂)ₙORₐ, -(CH₂)ₙP(O)ₚ(Rₐ)ₙ₅, -(CH₂)ₙS(O)RₐₐN(Rₐₐ), - (CH₂)ₙN=S(O)(Rₐₐ)₂, -(CH₂)ₙS(O)ₘRₐ or -(CH₂)ₙC(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl and 3- to 10-membered heterocyclyl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃alkoxy, C₁₋₃ hydroxyalkyl, -ORₐₐ, -S(O)RₐₐN(Rₐₐ), -N=S(O)(Rₐₐ)₂, -P(O)ₚ(Rₐₐ)ₙ₅, - S(O)ₘRₐₐ or -C(O)Rₐₐ;
R₂ is independently selected from hydrogen, deuterium, oxo, thio, halogen, amino, hydroxyl, cyano, nitro, C₁₋₃ alkyl, halo C₁₋₃alkyl, C₂₋₃ alkenyl, C₂₋₃ alkynyl, C₁₋₃ alkoxy or C₁₋₃ hydroxyalkyl;
R₄ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, -S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl and 3- to 10-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄₋₁ is selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy or C₁₋₃ hydroxyalkyl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl or 3- to 10-membered heterocyclyl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
R₅ is independently selected from hydrogen, deuterium, oxo, thio, halogen, amino, hydroxyl, cyano, nitro, C₁₋₃ alkyl, halo C₁₋₃alkyl, deuterated C₁₋₃ alkyl, C₂₋₃ alkenyl, C₂₋₃ alkynyl, C₁₋₃ alkoxy or C₁₋₃ hydroxyalkyl, and the amino, C₁₋₃ alkyl, halo C₁₋₃alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃alkyl; preferably, R₅ is selected from hydrogen, hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
R₆ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₐ or -C(O)Rₐ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl; preferably, R₆ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
Rₐ is independently selected from hydrogen, deuterium, halogen, amino, hydroxyl, cyano, nitro, C₁₋₃ alkyl, C₁₋₃ haloalkyl, C₁₋₃ deuteroalkyl C₂₋₃ alkenyl, C₂₋₃ alkynyl, C₁₋₃ alkoxy, C₁₋₃ hydroxyalkyl, C₃₋₁₀ cycloalkyl or 3- to 10-membered heterocyclyl; preferably, Rₐ is selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl or 3- to 10-membered heterocyclyl;
each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl; preferably, each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy or C₁₋₃ hydroxyalkyl;
x is 0, 1, 2 or 3;
y is 0, 1, 2 or 3;
p is 0, 1, 2 or 3;
n is 0, 1, 2 or 3;
m is 0, 1, 2 or 3;
n5 is 0, 1, 2 or 3; and
n6 is 0, 1 or 2.

8. The compound or the stereoisomer or pharmaceutically acceptable salt thereof according to claim 7, wherein the compound is further as represented by general formula (VII-2-1), (VII-3-1) or (VII-4-1): 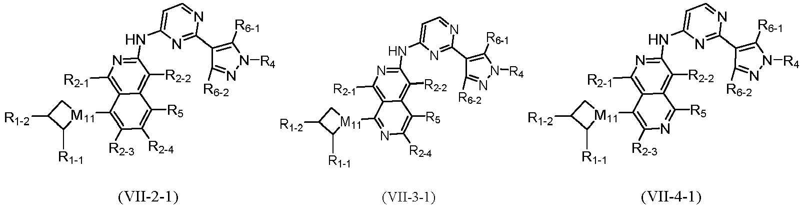 wherein
M₁₁ is independently CH or N;
R₁₋₁ is independently selected from hydrogen, deuterium, fluorine, chlorine, bromine, methyl, ethyl or halomethyl;
R₁₋₂ is independently selected from amino, hydroxyl, cyano, C₁₋₃ alkoxy, C₁₋₃ alkyl, C₃₋₆ cycloalkyl, or 3- to 8-membered heterocyclyl, and the amino, C₁₋₃ alkoxy, C₁₋₃ alkyl, C₃₋₆ cycloalkyl, and 3- to 8-membered heterocyclyl can be optionally further substituted with one or more of hydrogen, deuterium, oxo, thio, halogen, amino, hydroxyl, cyano, nitro, C₁₋₃ alkyl, C₁₋₃ deuteroalkyl, C₁₋₃ haloalkyl, C₁₋₃ alkoxy, halo C₁₋₃ alkoxy, C₁₋₃ hydroxyalkyl, -C(O)Rₑₑ, -ORₑₑ, -P(O)ₚ(Rₑₑ)ₙ₅ and - S(O)ₘRₑₑ; preferably, R₁₋₂ is selected from -NHS(O)₂CH₃, -NCH₃S(O)₂CH₃, -CH₂S(O)₂CH₃, -CH₂SOCH₃, -CH₂NO₂, methyl, hydrogen, 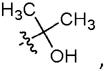 methoxy, cyano, -CH₂OCH₃, -CH₂CN, -CH(CN)₂, hydroxyl, - CH₂COCH₃, 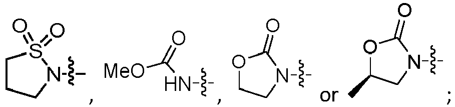
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃ alkyl;
R₄ is independently selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, - S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 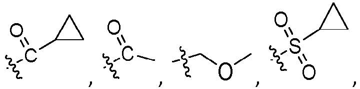 cyclopropyl, 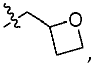 difluoromethyl, 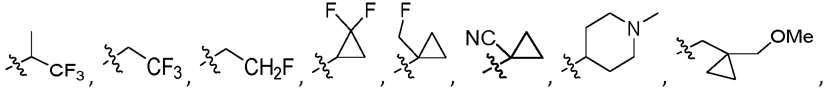 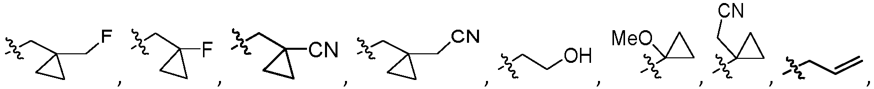 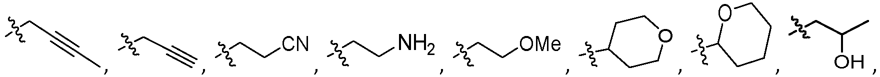 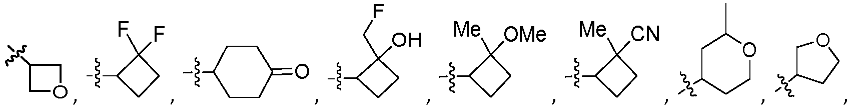 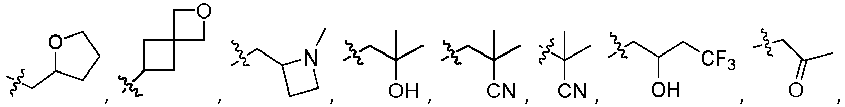 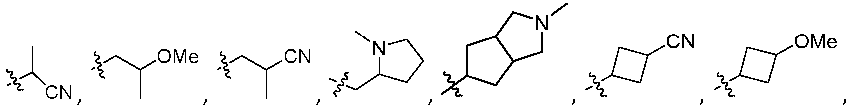 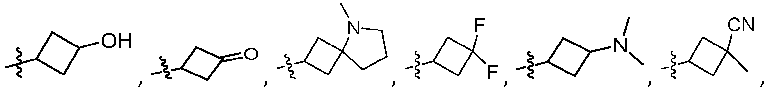 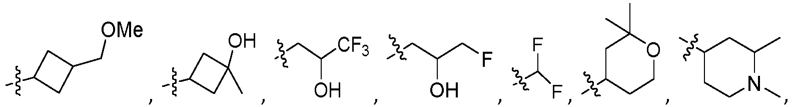 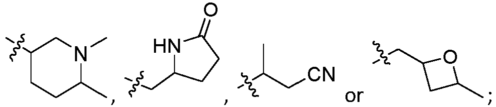
more preferably, R₄ is selected from fluorine, chlorine, cyano, trifluoromethyl, methyl, ethyl, nitro, hydroxyl, methoxy, -OCD₃, hydrogen, 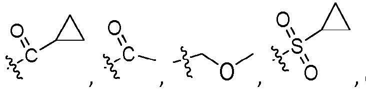 cyclopropyl, 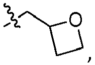 difluoromethyl, 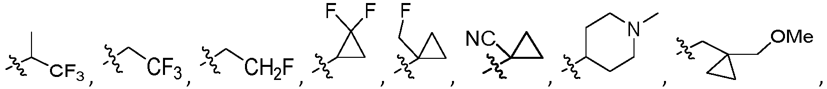 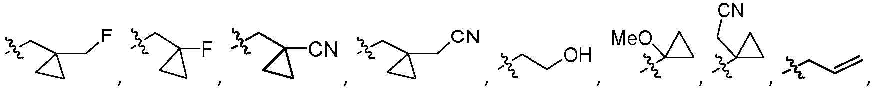 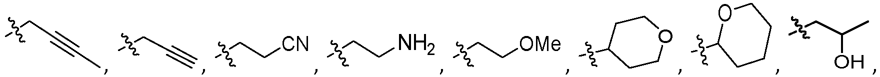 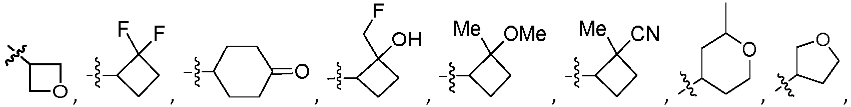 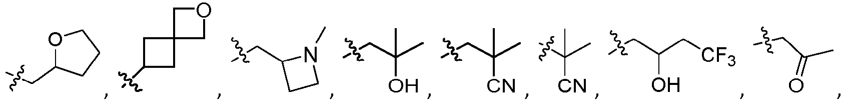 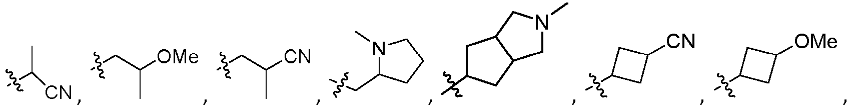 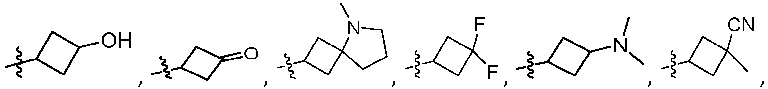 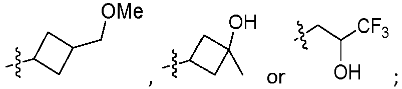
R₆₋₁ is independently selected from hydrogen, deuterium, oxo, thio, halogen, amino, hydroxyl, cyano, nitro, C₁₋₃ alkyl, C₁₋₃ haloalkyl, C₁₋₃ deuteroalkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl;
R₄ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is independently 0, 1 or 2;
p is independently 0, 1 or 2; and
n5 is independently 0, 1 or 2.

9. The compound or the stereoisomer or pharmaceutically acceptable salt thereof according to claim 8, wherein the compound is further as represented by general formula (VII-2-1-1), (VII-3-1-1) or (VII-4-1-1): 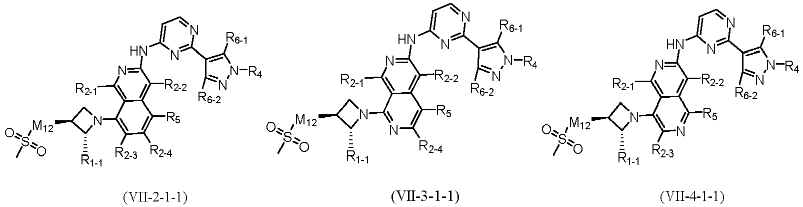 wherein
M₁₂ is independently selected from a bond, NR₉ or CR₁₀R₁₁;
R₉ is independently selected from hydrogen, deuterium, methyl, ethyl, monofluoromethyl, difluoromethyl or trifluoromethyl;
R₁₀ is independently selected from hydrogen, deuterium, fluorine, chlorine, bromine, methyl, ethyl, monofluoromethyl, difluoromethyl or trifluoromethyl;
R₁₁ is independently selected from hydrogen, deuterium, fluorine, chlorine, bromine, methyl, ethyl, monofluoromethyl, difluoromethyl or trifluoromethyl;
R₁₋₁ is independently selected from hydrogen, deuterium, fluorine, chlorine, bromine, methyl, ethyl, monofluoromethyl, difluoromethyl or trifluoromethyl;
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₅ is independently selected from isopropyl, -CH(Me)OMe or -N(Me)₂;
R₄ is independently selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, - S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl and 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 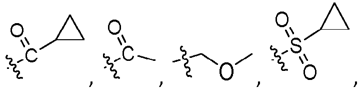 cyclopropyl, 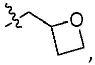 difluoromethyl, 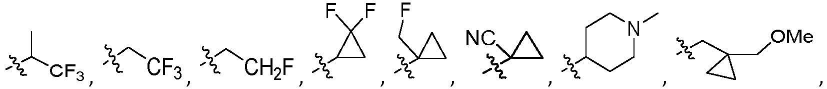 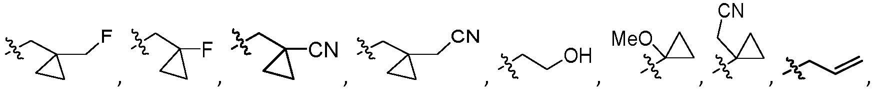 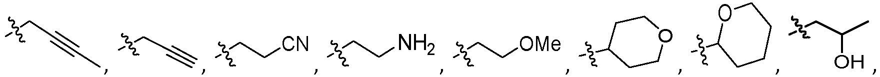 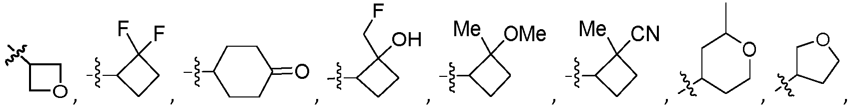 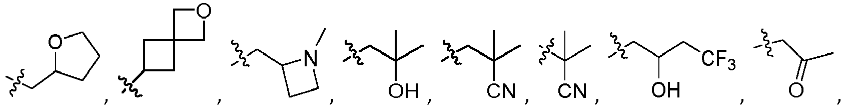 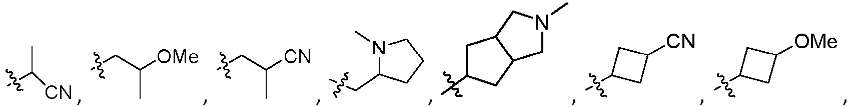 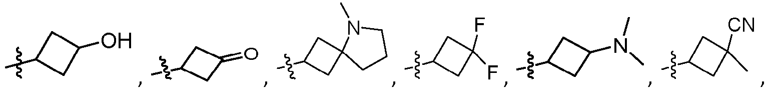 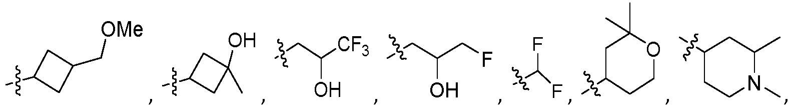 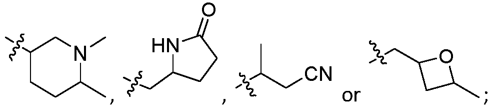 more preferably, R₄ is selected from fluorine, chlorine, cyano, trifluoromethyl, methyl, ethyl, nitro, hydroxyl, methoxy, -OCD₃, hydrogen, 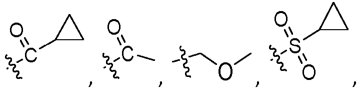 cyclopropyl, 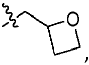
difluoromethyl, 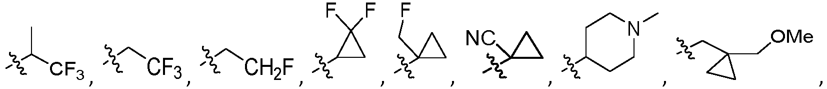 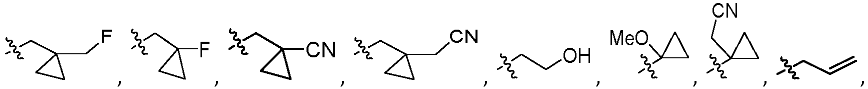 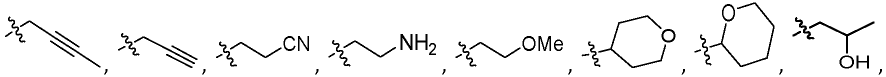 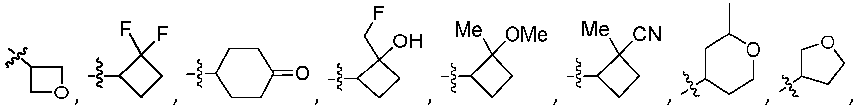 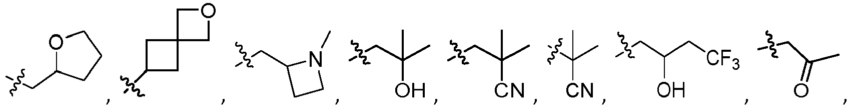 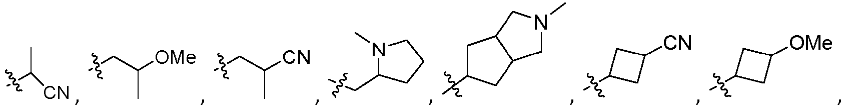 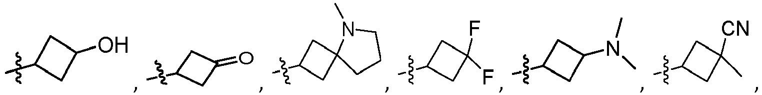 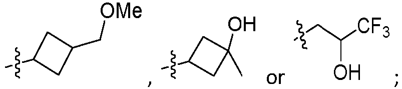
R₆₋₁ is independently selected from hydrogen, deuterium, oxo, halogen, amino, hydroxyl, cyano, nitro, C₁₋₃ alkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro and C₁₋₃ alkyl;
R₄ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl; and
m is independently 0, 1 or 2.

10. The compound or the stereoisomer or pharmaceutically acceptable salt thereof according to claim 1, wherein the compound is further as represented by general formula (VII-2-1-2), (VII-3-1-2) or (VII-4-1-2): 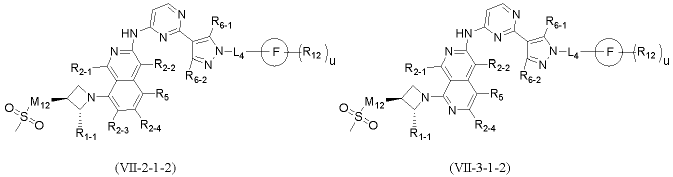 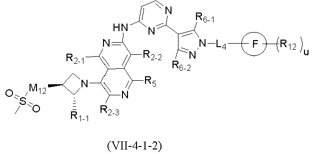 wherein
M₁₂ is independently selected from a bond, NR₉ or CR₁₀R₁₁;
R₉ is independently selected from hydrogen, deuterium, methyl, ethyl, monofluoromethyl, difluoromethyl or trifluoromethyl;
R₁₀ is independently selected from hydrogen, deuterium, fluorine, chlorine, bromine, methyl, ethyl, monofluoromethyl, difluoromethyl or trifluoromethyl;
R₁₁ is independently selected from hydrogen, deuterium, fluorine, chlorine, bromine, methyl, ethyl, monofluoromethyl, difluoromethyl or trifluoromethyl;
R₁₋₁ is independently selected from hydrogen, deuterium, fluorine, chlorine, bromine, methyl, ethyl, monofluoromethyl, difluoromethyl or trifluoromethyl;
R₂₋₁ is independently selected from hydrogen, deuterium, fluorine, chlorine, bromine, methyl, ethyl, halomethyl or haloethyl;
R₂₋₂ is independently selected from hydrogen, deuterium, fluorine, chlorine, bromine, methyl, ethyl, halomethyl or haloethyl;
R₂₋₃ is independently selected from hydrogen, deuterium, fluorine, chlorine, bromine, methyl, ethyl, halomethyl or haloethyl;
R₂₋₄ is independently selected from hydrogen, deuterium, fluorine, chlorine, bromine, methyl, ethyl, halomethyl or haloethyl;
R₅ is independently selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₅ is independently selected from isopropyl, -CH(Me)OMe or -N(Me)₂;
L₄ is independently selected from a bond, C₁₋₃ alkylene, C₂₋₄ alkenylene, C₂₋₄ alkynylene, - (CH₂)ₙ₇NH(CH₂)ₙ₈- or -(CH₂)ₙ₉O(CH₂)ₙ₁₀-, and the C₁₋₃ alkylene, C₂₋₄ alkenylene, C₂₋₄ alkynylene, - (CH₂)ₙ₇NH(CH₂)ₙ₈- and -(CH₂)ₙ₉O(CH₂)ₙ₁₀- are optionally substituted with one or more of halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl;
ring F is independently selected from C₃₋₆ cycloalkyl or 3- to 8-membered heterocyclyl;
preferably, ring F is independently selected from 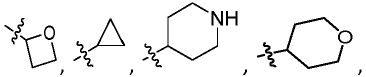 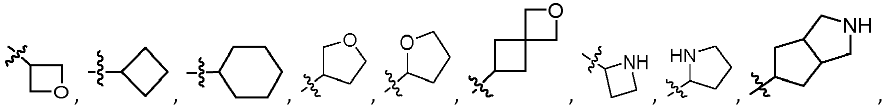 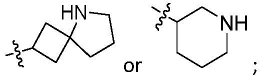 more preferably, ring F is independently selected from 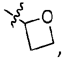 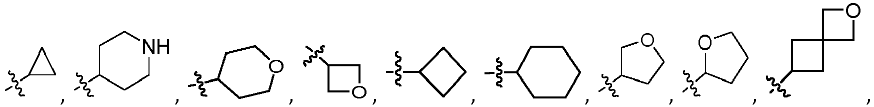 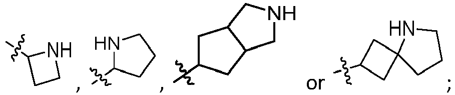
R₁₂ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy or C₁₋₃ hydroxyalkyl; the C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃hydroxyalkyl are optionally substituted with one or more of halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy and C₁₋₃ hydroxyalkyl;
R₆₋₁ is independently selected from hydrogen, deuterium, oxo, halogen, amino, hydroxyl, cyano, nitro, C₁₋₃ alkyl, C₁₋₃ alkoxy or C₁₋₃ hydroxyalkyl;
R₆₋₂ is independently selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro and C₁₋₃ alkyl;
R₄ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is independently 0, 1 or 2;
n7 is independently 0, 1 or 2;
n8 is independently 0, 1 or 2;
n9 is independently 0, 1 or 2;
n10 is independently 0, 1 or 2; and
u is independently 0, 1 or 2.

11. The compound or the stereoisomer or pharmaceutically acceptable salt thereof according to claim 1, wherein the specific compounds are as follows:

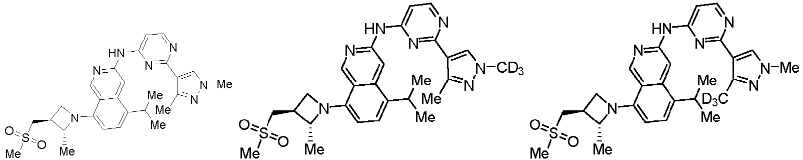

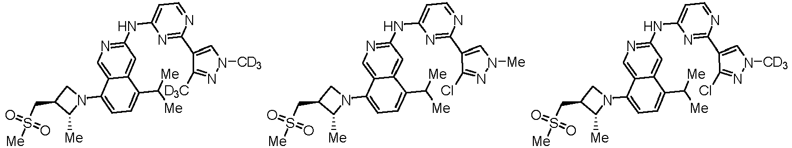

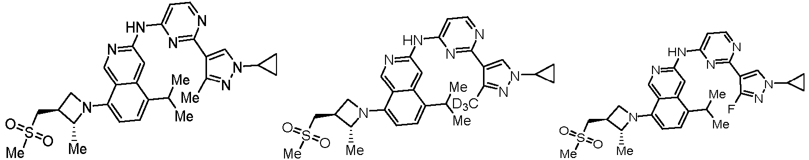

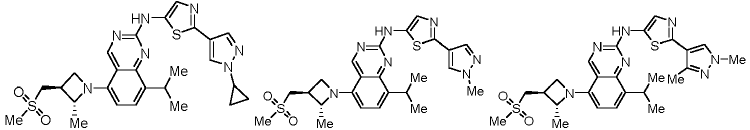

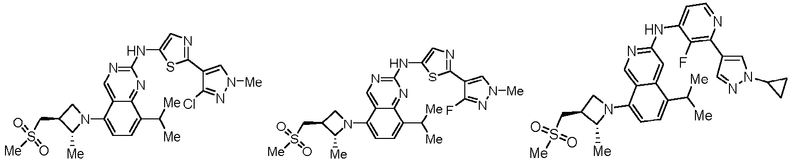

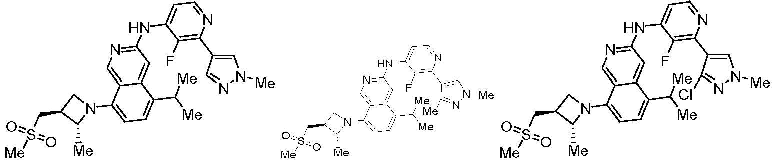

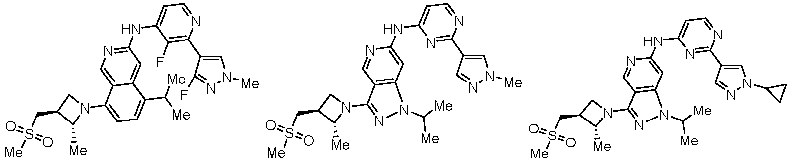

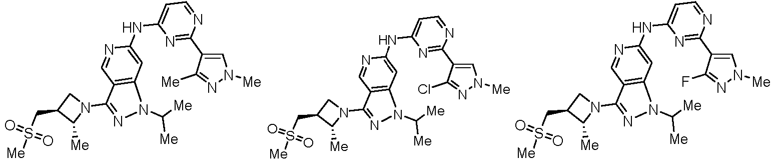

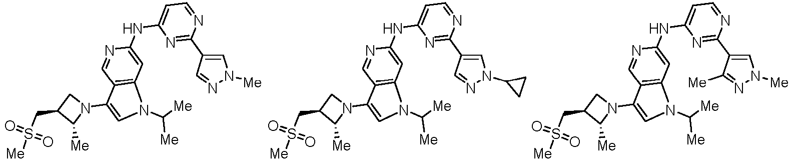

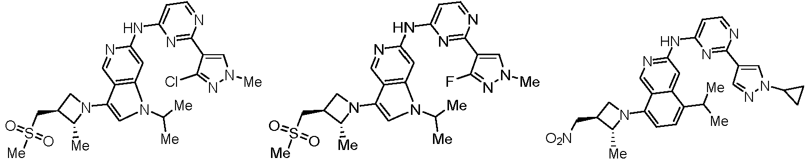

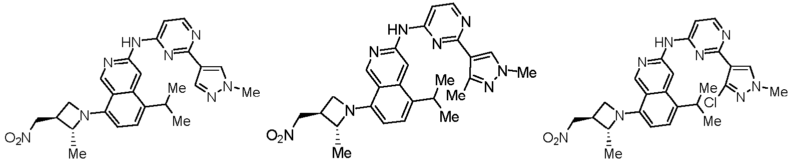

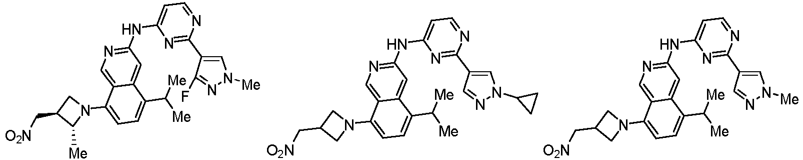

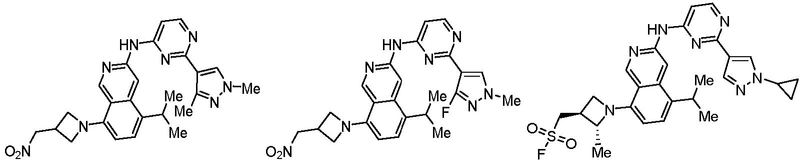

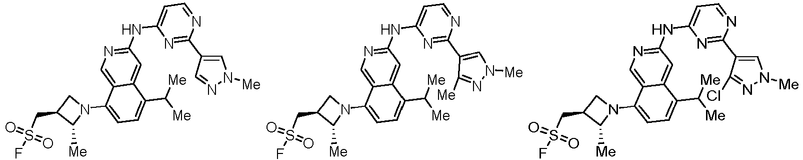

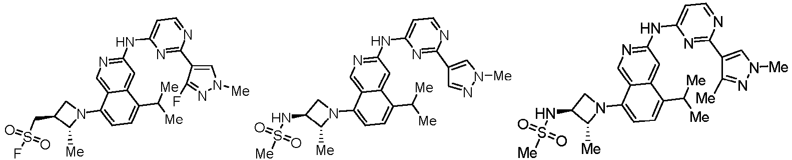

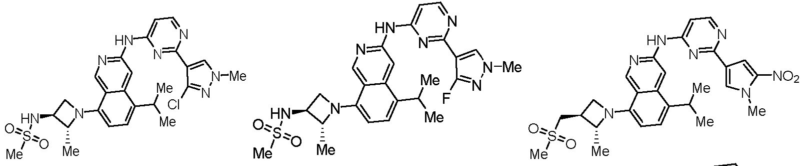

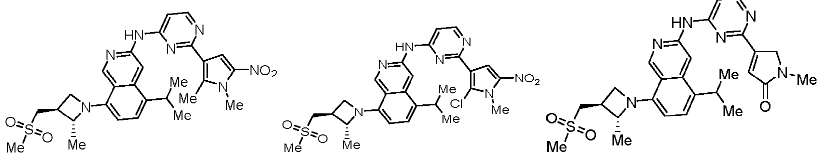

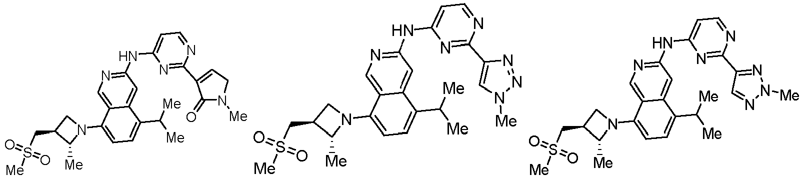

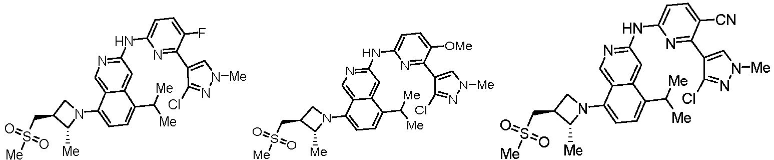

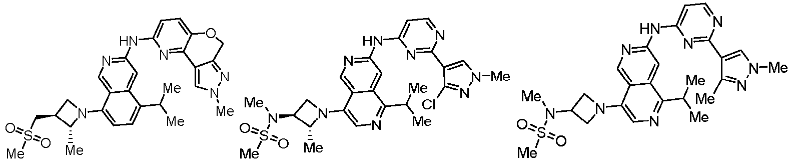

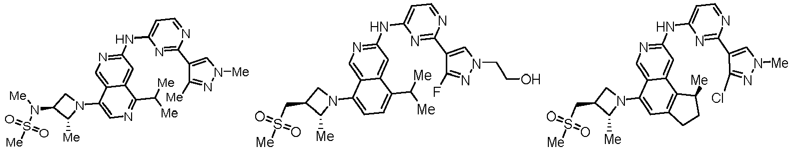

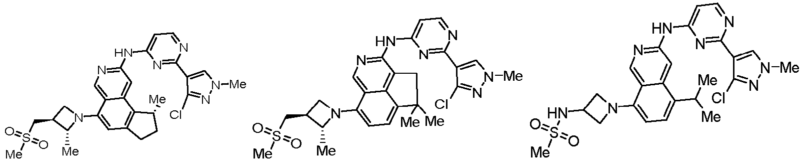

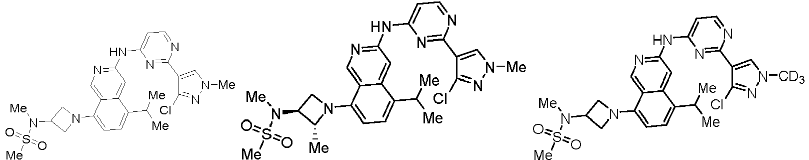

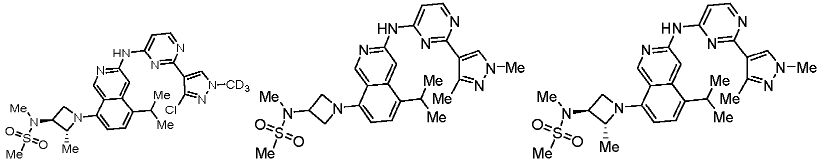

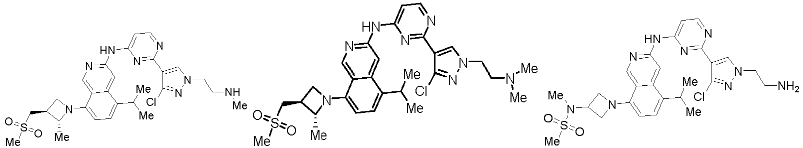

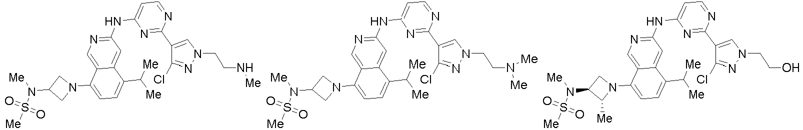

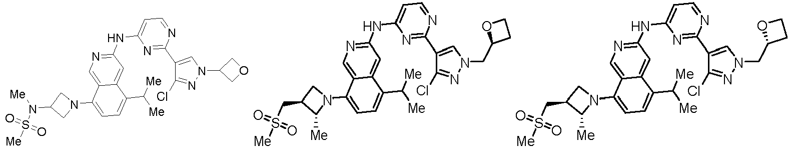

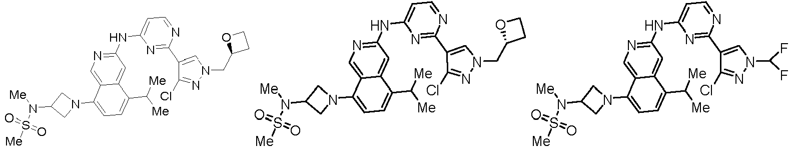

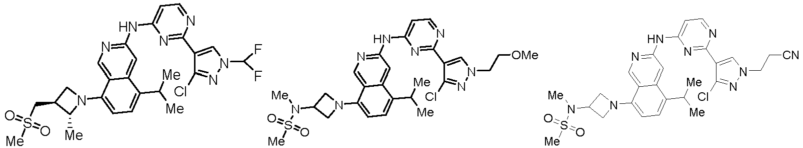

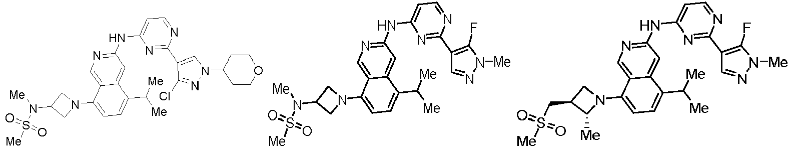

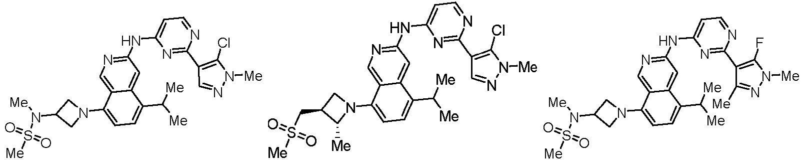

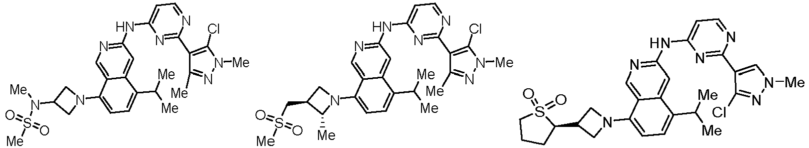

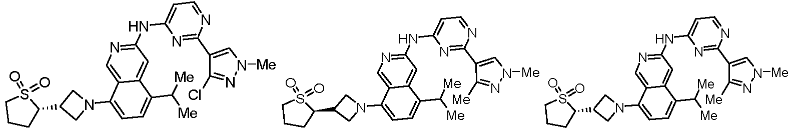

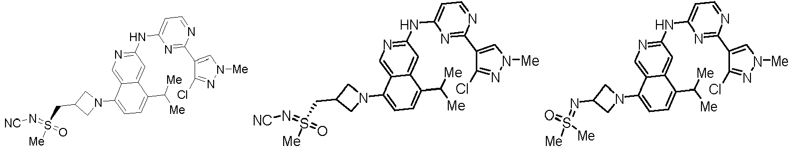

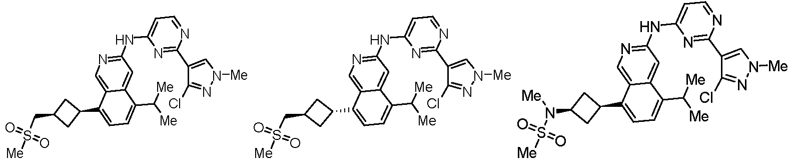

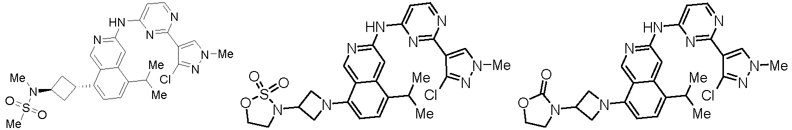

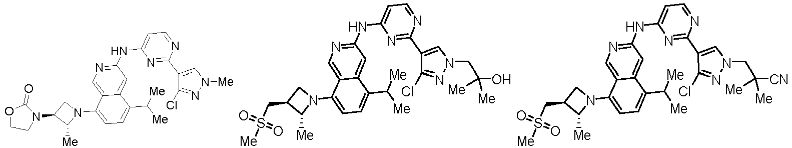

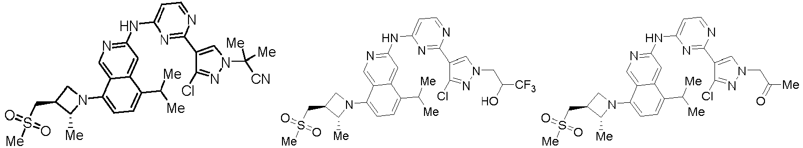

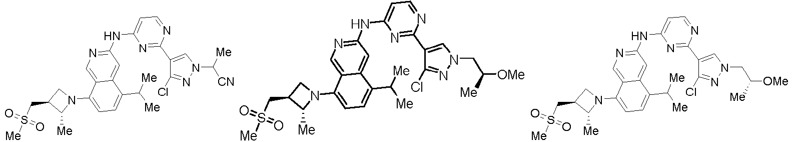

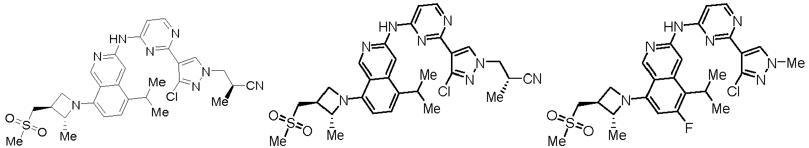

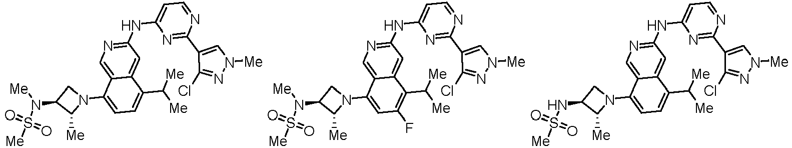

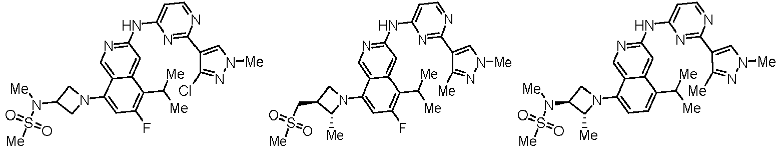

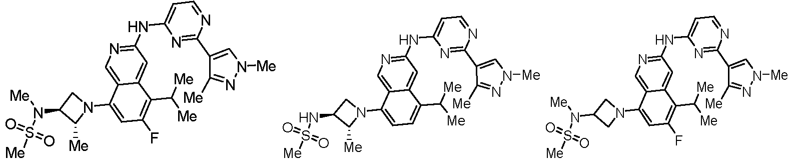

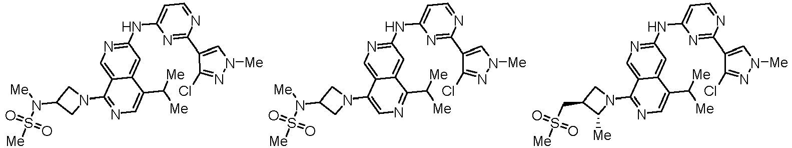

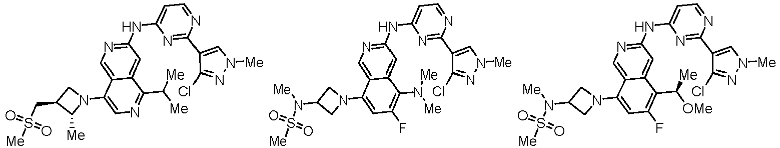

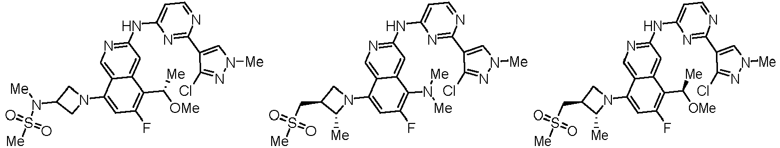

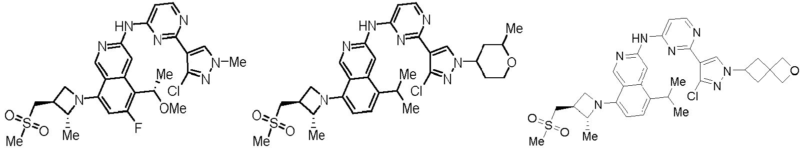

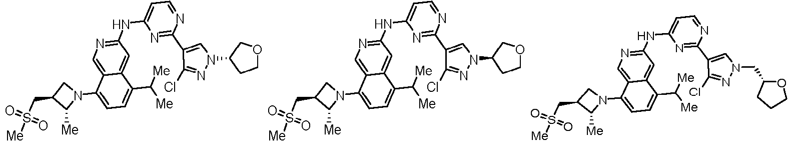

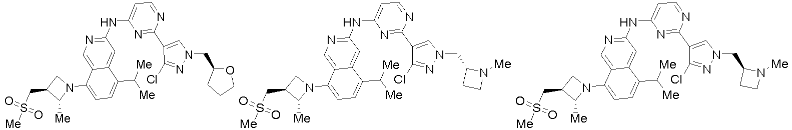

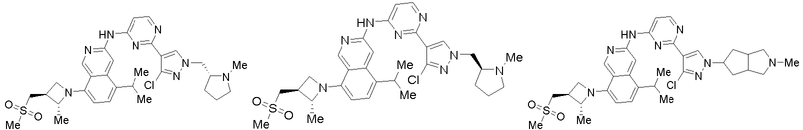

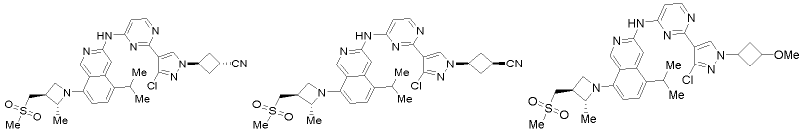

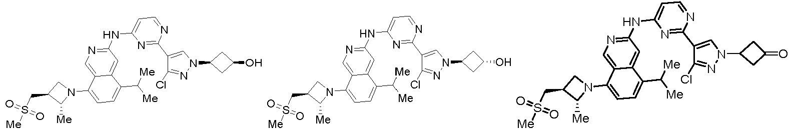

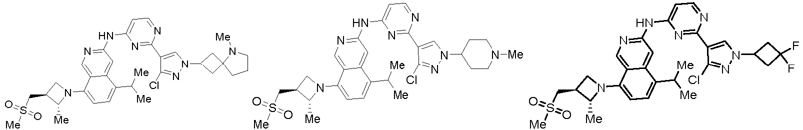

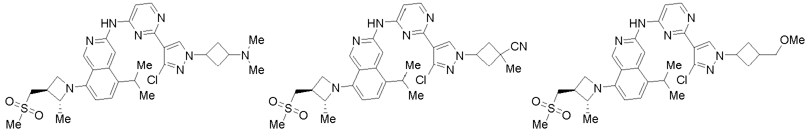

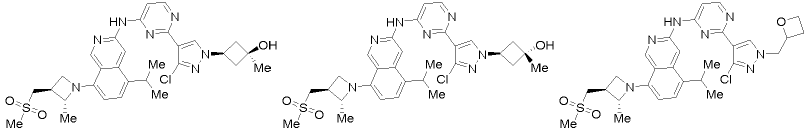

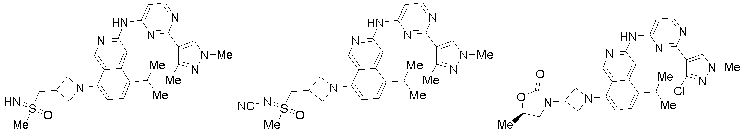

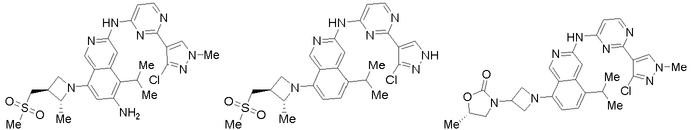

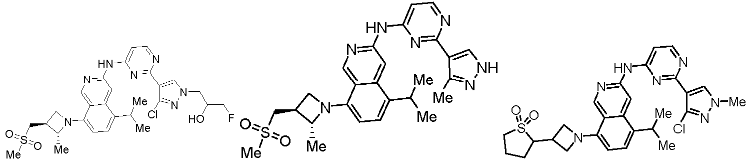

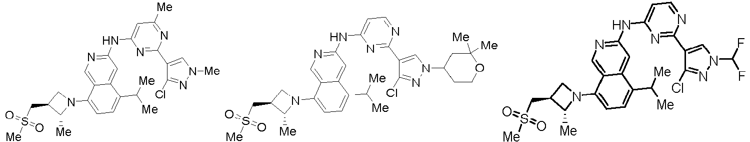

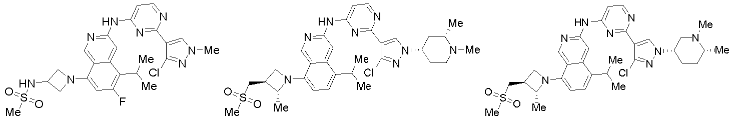

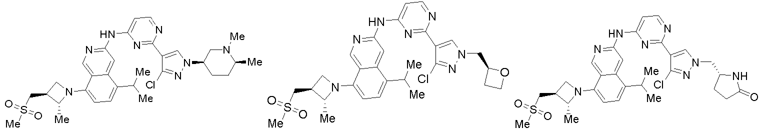

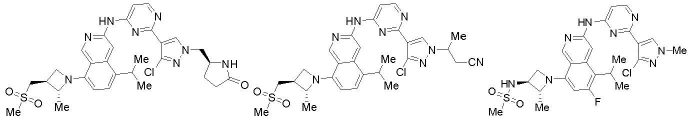

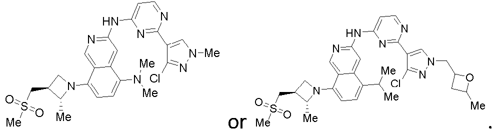

12. A method for preparing the compound represented by general formula (II-G), or the stereoisomer or pharmaceutically acceptable salt thereof according to claim 1, wherein the method comprises the following steps: reacting the compound represented by formula (XV) with the compound represented by formula (XVI) to obtain a compound represented by formula (II-G), 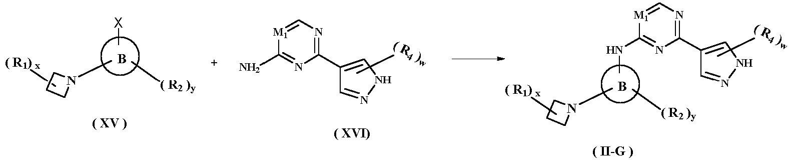 wherein X is halogen, preferably chlorine.

13. A pharmaceutical composition comprising a therapeutically effective dose of the compound of the general formula, or the stereoisomer or pharmaceutically acceptable salt thereof according to any one of claims 1-11, and one or more pharmaceutically acceptable carriers, diluents, or excipients.

14. Use of the compound, the stereoisomer or pharmaceutically acceptable salt thereof according to any one of claims 1-11 or the pharmaceutical composition of claim 13 in the preparation of an EGFR inhibitor.

15. The use according to claim 14, wherein the EGFR is a mutated EGFR, preferably with one or more mutations of Del19, L858R, T790M or C797S, and more preferably with L858R/T790M, Del19/T790M, Del19/C797S, L858R/C797S, Del19/T790M/C797S or L858R/T790M/C797S mutation.

16. Use of the compound, the stereoisomer or pharmaceutically acceptable salt thereof according to any one of claims 1-11 or the pharmaceutical composition of claim 13 in the preparation of a drug for the treatment of a cancer; preferably, the cancer is selected from ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular carcinoma, gastric cancer, gastrointestinal stromal tumor, thyroid cancer, bile duct cancer, endometrial cancer, renal cancer, anaplastic large cell lymphoma, multiple myeloma, melanoma or mesothelioma; more preferably, the cancer is non-small cell lung cancer; further preferably, the cancer is a non-small cell lung cancer with EGFR L858R/T790M, Del19/T790M, Del19/C797S, L858R/C797S, Del19/T790M/C797S or L858R/T790M/C797S mutation.
